(19) Europäisches Patentamt
European Patent Office
Office européen des brevets

(11) EP 1 095 025 B1

(12) **EUROPÄISCHE PATENTSCHRIFT**

(45) Veröffentlichungstag und Bekanntmachung des Hinweises auf die Patenterteilung:
**11.12.2002 Patentblatt 2002/50**

(21) Anmeldenummer: **99932765.3**

(22) Anmeldetag: **01.07.1999**

(51) Int Cl.7: **C07D 235/14**, A61K 31/415

(86) Internationale Anmeldenummer:
**PCT/EP99/04531**

(87) Internationale Veröffentlichungsnummer:
**WO 00/001704 (13.01.2000 Gazette 2000/02)**

(54) **BENZIMIDAZOLE, DEREN HERSTELLUNG UND DEREN VERWENDUNG ALS ARZNEIMITTEL**

BENZIMIDAZOLES, PRODUCTION THEREOF AND USE THEREOF AS MEDICAMENTS

BENZIMIDAZOLES, LEUR PREPARATION ET LEUR UTILISATION COMME MEDICAMENTS

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **04.07.1998 DE 19829964**
**11.12.1998 DE 19857202**
**20.03.1999 DE 19912690**

(43) Veröffentlichungstag der Anmeldung:
**02.05.2001 Patentblatt 2001/18**

(73) Patentinhaber: **Boehringer Ingelheim Pharma KG**
**55216 Ingelheim am Rhein (DE)**

(72) Erfinder:
- **RIES, Uwe**
  **D-88400 Biberach (DE)**
- **KAUFFMANN, Iris**
  **D-88400 Biberach (DE)**
- **HAUEL, Norbert**
  **D-88433 Schemmerhofen (DE)**
- **PRIEPKE, Henning**
  **D-88447 Warthausen (DE)**
- **NAR, Herbert**
  **88416 Ochsenhausen (DE)**
- **STASSEN, Jean Marie**
  **3012 Leuven (BE)**
- **WIENEN, Wolfgang**
  **D-88400 Biberach (DE)**

(74) Vertreter: **Laudien, Dieter, Dr.**
**Boehringer Ingelheim GmbH,**
**CD Patents**
**55216 Ingelheim am Rhein (DE)**

(56) Entgegenhaltungen:
**WO-A-94/08962** **WO-A-97/21437**
**WO-A-98/01428** **WO-A-98/37075**
**US-A- 4 634 783**

- **F. MARKWARDT ET AL.: "Na-arylsulfonyl-omega-(4-amidinophenyl)-a lpha-aminoalkylcarboxylic acid amides-novel selective inhibitors of thrombin" THROMBOSIS RESEARCH, Bd. 17, 1980, Seiten 425-431, XP002103556 united states**
- **TAKAYASU NAGAHARA ET AL.: "dibasic (amidinoaryl)propanoic Acid Derivatives as Novel blood Coagulation Factor Xa Inhibitors." J. MED. CHEM., Bd. 37, 1994, Seiten 1200-1207, XP000608128 columbus ohio**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Gegenstand der vorliegenden Erfindung sind Benzimidazole der allgemeinen Formel

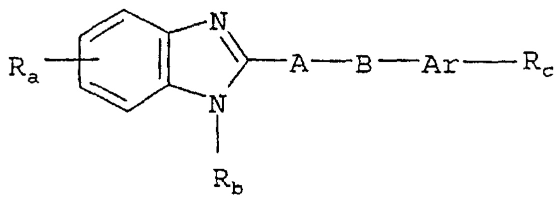

(I),

deren Tautomere, deren Stereoisomere, deren Gemische, deren Prodrugs, deren Derivate, die an Stelle einer Carboxygruppe eine unter physiologischen Bedingungen negativ geladene Gruppe enthalten, und deren Salze, insbesondere deren physiologisch verträglichen Salze mit anorganischen oder organischen Säuren oder Basen, welche wertvolle Eigenschaften aufweisen.

Die Verbindungen der obigen allgemeinen Formel I, in denen $R_c$ eine Cyanogruppe darstellt, stellen wertvolle Zwischenprodukte zur Herstellung der übrigen Verbindungen der allgemeinen Formel I dar, und die Verbindungen der obigen allgemeinen Formel I, in denen $R_c$ eine der nachfolgenden Amidinogruppen darstellt, sowie deren Tautomere, deren Stereoisomere, deren Gemische, deren Prodrugs, deren Derivate, die an Stelle einer Carboxygruppe eine unter physiologischen Bedingungen negativ geladene Gruppe enthalten, und deren Salze, insbesondere deren physiologisch verträgliche Salze mit anorganischen oder organischen Salze, und deren Stereoisomere weisen wertvolle pharmakologische Eigenschaften auf, insbesondere eine antithrombotische Wirkung.

Gegenstand der vorliegenden Anmeldung sind somit die neuen Verbindungen der obigen allgemeinen Formel I sowie deren Herstellung, die die pharmakologisch wirksamen Verbindungen enthaltende Arzneimittel, deren Herstellung und Verwendung.

In der obigen allgemeinen Formel bedeutet

Ar eine gegebenenfalls durch ein Fluor-, Chlor- oder Bromatom, durch eine Trifluormethyl-, $C_{1-3}$-Alkyl- oder $C_{1-3}$-Alkoxygruppe substituierte Phenylen- oder Naphthylengruppe,

eine gegebenenfalls im Kohlenstoffgerüst durch eine $C_{1-3}$-Alkylgruppe substituierte Thienylen-, Thiazolylen-, Pyridinylen-, Pyrimidinylen-, Pyrazinylen- oder Pyridazinylengruppe,

A eine $C_{1-3}$-Alkylengruppe,

B ein Sauerstoff- oder Schwefelatom, eine Methylen-, Carbonyl-, Sulfinyl- oder Sulfonylgruppe, eine gegebenenfalls durch eine $C_{1-3}$-Alkylgruppe substituierte Iminogruppe, in der der Alkylteil durch eine Carboxygruppe mono- oder disubstituiert sein kann,

$R_a$ eine $R_1$-CO-$C_{3-5}$-cycloalkylgruppe, in der

$R_1$ eine $C_{1-3}$-Alkoxy-, Amino-, $C_{1-4}$-Alkylamino- oder Di-($C_{1-4}$-Alkyl)-aminogruppe, in denen jeweils der Alkylteil durch eine Carboxygruppe substituiert sein kann,

eine 4- bis 7-gliedrige Cycloalkylenimino- oder Cycloalkenyleniminogruppe, die durch eine oder zwei $C_{1-3}$-Alkylgruppen substituiert sein können, wobei ein Alkylsubstituent gleichzeitig durch eine Hydroxy-, $C_{1-3}$-Alkoxy-, Carboxy-, Carboxy-$C_{1-3}$-alkoxy-, Carboxy-$C_{1-3}$-alkylamino-, N-($C_{1-3}$-Alkyl)-N-(carboxy-$C_{1-3}$-alkyl)-amino-, Carboxy-$C_{1-3}$-alkylaminocarbonyl-, N- ($C_{1-3}$-Alkyl)-N-(carboxy-$C_{1-3}$-alkyl)-aminocarbonyl-, Carboxy-$C_{1-3}$-alkylaminocarbonylamino-, 1- ($C_{1-3}$-Alkyl)-3- (carboxy-$C_{1-3}$-alkyl)-aminocarbonylamino-, 3-($C_{1-3}$-Alkyl)-3-(carboxy-$C_{1-3}$-alkyl)-aminocarbonylamino- oder 1,3-Di-($C_{1-3}$-alkyl)-3-(carboxy-$C_{1-3}$-alkyl)-aminocarbonylaminogruppe substituiert sein kann,

eine durch eine Hydroxygruppe substituierte 4- bis 7-gliedrige Cycloalkyleniminogruppe,

eine gegebenenfalls durch eine $C_{1-3}$-Alkylgruppe substituierte 5- bis 7-gliedrige Cycloalkyleniminogruppe, an die über zwei benachbarte Kohlenstoffatome ein Phenylring ankondensiert ist,

eine Morpholino-, Piperazino-, N-($C_{1-3}$-Alkyl)-piperazino-, Pyrrolino-, 3,4-Dehydro-piperidino- oder Pyrrol-1-yl-Gruppe darstellt,

eine $R_2$-CX-$C_{3-5}$-cycloalkylgruppe, in der

$R_2$ eine gegebenenfalls durch eine $C_{1-3}$-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine $C_{1-3}$-Alkylgruppe substituierte Iminogruppe, ein Sauerstoff- oder Schwefelatom oder eine gegebenenfalls durch eine $C_{1-3}$-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-$C_{1-3}$-alkoxy-, Carboxy-$C_{1-3}$-alkylamino- oder N-($C_{1-3}$-Alkyl)-carboxy-$C_{1-3}$-alkylaminogruppe substituiert sein kann, und

X ein Sauerstoffatom, eine $C_{1-3}$-Alkylimino-, $C_{1-3}$-Alkoxyimino-, $C_{1-3}$-Alkylhydrazino-, Di- ($C_{1-3}$-Alkyl) -hydrazino-, $C_{2-4}$-Alkanoylhydrazino-, N- ($C_{1-3}$-Alkyl)-$C_{2-4}$-alkanoylhydrazino- oder $C_{1-3}$-Alkylidengruppe, die jeweils im Alkyl- oder Alkanoylteil oder im Alkyl- und Alkanoylteil durch eine Carboxygruppe substituiert sein können, darstellen,

eine durch eine Imidazol- oder Imidazolongruppe substituierte $C_{1-3}$-Alkyl- oder $C_{3-5}$-Cycloalkylgruppe, in denen

der Imidazolring durch eine Phenyl- oder Carboxygruppe und durch eine oder zwei $C_{1-3}$-Alkylgruppen oder durch eine, zwei oder drei $C_{1-3}$-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl)-$C_{1-3}$-alkylamino- oder Di-($C_{1-3}$-Alkyl)-aminogruppe substituiert sein kann, und

der Imidazolonring durch eine $C_{1-3}$-Alkylgruppe substituiert sein kann, wobei der Alkylsubstituent durch eine Carboxygruppe oder in 2- oder 3-Stellung durch eine Amino-, $C_{2-4}$-Alkanoylamino-, $C_{1-3}$-Alkylamino-, N- ($C_{2-4}$-Alkanoyl) -$C_{1-3}$-alkylamino- oder Di-($C_{1-3}$-Alkyl)-aminogruppe substituiert sein kann, und

zusätzlich an die vorstehend erwähnten Imidazol- und Imidazolonringe über zwei benachbarte Kohlenstoffatome ein Phenyl- oder Pyridinring ankondensiert sein kann,

eine Imidazolidin-2,4-dion-5-yl-Gruppe, die durch eine oder zwei $C_{1-3}$-Alkylgruppen substituiert sein kann, wobei gleichzeitig ein Alkylsubstituent durch eine Carboxygruppe substituiert sein kann,

eine $C_{1-4}$-Alkylgruppe, die

durch eine $C_{1-3}$-Alkyl-$Y_1$-$C_{1-3}$-alkyl-, HOOC-$C_{1-3}$-alkyl-$Y_1$-$C_{1-3}$-alkyl-, Tetrazolyl-$C_{1-3}$-alkyl-$Y_2$-, $R_3NR_4$- oder $R_3NR_4$-$C_{1-3}$-alkyl-Gruppe und

durch eine gegebenenfalls durch eine $C_{1-3}$-Alkylgruppe substituierte Isoxazolidinylcarbonylgruppe, durch eine Pyrrolinocarbonyl-, 3,4-Dehydro-piperidinocarbonyl-, Pyrrol-1-ylcarbonyl-, Carboxy-, Aminocarbonyl-, $C_{1-3}$-Alkylaminocarbonyl-, Di-($C_{1-3}$-Alkyl)-aminocarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei $C_{1-3}$-Alkylgruppen substituiert und gleichzeitig jeweils ein Alkylteil oder Alkylsubstituent der vorstehend erwähnten $C_{1-3}$-Alkylaminocarbonyl-, Di-($C_{1-3}$-Alkyl)-aminocarbonyl- oder Cycloalkyleniminocarbonylgruppen durch eine Carboxygruppe substituiert sein kann, und die verbleibenden Wasserstoffatome der $C_{1-4}$-Alkylgruppe ganz oder teilweise durch Fluoratome ersetzt sein können, in denen

$R_3$ ein Wasserstoffatom oder eine gegebenenfalls durch eine Carboxygruppe substituierte $C_{1-3}$-Alkylgruppe und

$R_4$ ein Wasserstoffatom, eine $C_{1-3}$-Alkyl-$Y_1$-$C_{1-3}$-alkyl-$Y_2$-, Carboxy-$C_{1-3}$-alkyl-$Y_1$-$C_{1-3}$-alkyl-$Y_2$-, $C_{1-3}$-Alkyl-$Y_2$- oder Carboxy-$C_{1-3}$-alkyl-$Y_2$-Gruppe oder

$R_3$ und $R_4$ zusammen mit dem dazwischen liegenden Stickstoffatom eine gegebenenfalls durch eine Carboxy-, $C_{1-3}$-Alkyl- oder Carboxy-$C_{1-3}$-alkylgruppe substituierte 4- bis 7-gliedrige Cycloalkyleniminogruppe darstellen, in denen

$Y_1$ eine Kohlenstoff-Kohlenstoff-Bindung, ein Sauerstoffatom, eine Sulfenyl-, Sulfinyl-, Sulfonyl-, -NH-, -NH-CO- oder -NH-CO-NH-Gruppe und

Y2 eine Kohlenstoff-Stickstoffbindung oder eine Carbonyl-, Sulfonyl-, Imino- oder -NH-CO-Gruppe darstellen, wobei die Carbonylgruppe der -NH-CO-Gruppe mit dem Stickstoffatom der $R_3NR_4$-Gruppe verknüpft ist, und die bei der Definition der Reste $Y_1$ und $Y_2$ vorkommenden Iminogruppen jeweils zusätzlich durch eine $C_{1-3}$-Alkyl- oder Carboxy-$C_{1-3}$-alkylgruppe substituiert sein können,

eine durch eine $R_5NR_6$-Gruppe substituierte $C_{1-3}$-Alkyl- oder $C_{3-5}$-Cycloalkylgruppe, in denen

$R_5$ ein Wasserstoffatom, eine $C_{1-3}$-Alkyl-, $C_{5-7}$-Cycloalkyl-, Phenylcarbonyl-, Phenylsulfonyl- oder Pyridinylgruppe und

$R_6$ eine $C_{1-3}$-Alkyl-, Carboxy-$C_{1-3}$-alkyl- oder Carboxy-$C_{1-3}$-alkylcarbonylgruppe darstellen,

eine $C_{1-3}$-Alkylgruppe, die durch $C_{2-4}$-Alkanoyl- oder $C_{5-7}$-Cycloalkanoylgruppe und durch eine durch ein Chlor-, Brom- oder Jodatom substituierte $C_{1-3}$-Alkylgruppe substituiert ist,

$R_b$ ein Wasserstoffatom oder eine $C_{1-3}$-Alkylgruppe und

$R_c$ eine Cyanogruppe oder eine gegebenenfalls durch eine oder zwei $C_{1-3}$-Alkylgruppen substituierte Amidinogruppe.

Die bei der Definition der vorstehend erwähnten Resten erwähnten Carboxygruppen können außerdem durch eine in-vivo in eine Carboxygruppe überführbare Gruppe oder durch eine unter physiologischen Bedingungen negativ geladene Gruppe ersetzt sein oder

die bei der Definition der vorstehend erwähnten Resten erwähnten Amino- und Iminogruppen können außerdem durch einen in vivo abspaltbaren Rest substituiert sein. Derartige Gruppen werden beispielsweise in der WO 98/46576 und von N.M. Nielson et al. in International Journal of Pharmaceutics 39, 75-85 (1987) beschrieben.

Unter einer in-vivo in eine Carboxygruppe überführbare Gruppe ist beispielsweise eine Hydroxmethylgruppe, eine mit einem Alkohol veresterte Carboxygruppe, in der der alkoholische Teil vorzugsweise ein $C_{1-6}$-Alkanol, ein Phenyl-$C_{1-3}$-alkanol, ein $C_{3-9}$-Cycloalkanol, wobei ein $C_{5-8}$-Cycloalkanol zusätzlich durch ein oder zwei $C_{1-3}$-Alkylgruppen substituiert sein kann, ein $C_{5-8}$-Cycloalkanol, in dem eine Methylengruppe in 3- oder 4-Stellung durch ein Sauerstoffatom oder durch eine gegebenenfalls durch eine $C_{1-3}$-Alkyl-, Phenyl-$C_{1-3}$-alkyl-, Phenyl-$C_{1-3}$-alkoxycarbonyl- oder $C_{2-6}$-Alkanoylgruppe substituierte Iminogruppe ersetzt ist und der Cycloalkanolteil zusätzlich durch ein oder zwei $C_{1-3}$-Alkylgruppen substituiert sein kann, ein $C_{4-7}$-Cycloalkenol, ein $C_{3-5}$-Alkenol, ein Phenyl-$C_{3-5}$-alkenol, ein $C_{3-5}$-Alkinol oder Phenyl- $C_{3-5}$-alkinol mit der Maßgabe, daß keine Bindung an das Sauerstoffatom von einem Kohlenstoffatom ausgeht, welches eine Doppel- oder Dreifachbindung trägt, ein $C_{3-8}$-Cycloalkyl-$C_{1-3}$-alkanol, ein Bicycloalkanol mit insgesamt 8 bis 10 Kohlenstoffatomen, das im Bicycloalkylteil zusätzlich durch eine oder zwei $C_{1-3}$-Alkylgruppen substituiert sein kann, ein 1,3-Dihydro-3-oxo-1-isobenzfuranol oder ein Alkohol der Formel

$$R_d\text{-CO-O-}(R_eCR_f)\text{-OH},$$

in dem
$R_d$ eine $C_{1-8}$-Alkyl-, $C_{5-7}$-Cycloalkyl-, Phenyl- oder Phenyl-$C_{1-3}$-alkylgruppe,
$R_e$ ein Wasserstoffatom, eine $C_{1-3}$-Alkyl-, $C_{5-7}$-Cycloalkyl- oder Phenylgruppe und
$R_f$ ein Wasserstoffatom oder eine $C_{1-3}$-Alkylgruppe darstellen,
unter einer unter physiologischen Bedingungen negativ geladenen Gruppe wie eine Tetrazol-5-yl-, Phenylcarbonylaminocarbonyl-, Trifluormethylcarbonylaminocarbonyl-, $C_{1-6}$-Alkylsulfonylamino-, Phenylsulfonylamino-, Benzylsulfonylamino-, Trifluormethylsulfonylamino-, $C_{1-6}$-Alkylsulfonylaminocarbonyl-, Phenylsulfonylaminocarbonyl-, Benzylsulfonylaminocarbonyl- oder Perfluor-$C_{1-6}$-alkylsulfonylaminocarbonylgruppe
und unter einem von einer Imino- oder Aminogruppe in-vivo abspaltbaren Rest beispielsweise eine Hydroxygruppe, eine Acylgruppe wie eine gegebenenfalls durch Fluor-, Chlor-, Bromoder Jodatome, durch $C_{1-3}$-Alkyl- oder $C_{1-3}$-Alkoxygruppen mono- oder disubstituierte Benzoylgruppe, wobei die Substituenten gleich oder verschieden sein können, eine Pyridinoylgruppe oder eine $C_{1-16}$-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine $C_{1-16}$-Alkoxycarbonyl- oder $C_{1-16}$-Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl -, Isopropoxycarbonyl-, Butoxycarbonyl-, tert. Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy- oder Hexadecylcarbonyloxygruppe, eine Phenyl-$C_{1-6}$-alkoxycarbonylgruppe wie die Benzyloxycarbonyl-, Phenylethoxycarbonyl- oder Phenylpropoxycarbonylgruppe, eine 3-Amino-propionylgruppe, in der die Aminogruppe durch $C_{1-6}$-Alkyl- oder $C_{3-7}$-Cycloalkylgruppen mono- oder disubstituiert und die Substituenten gleich oder verschieden sein können, eine $C_{1-3}$-Alkylsulfonyl-$C_{2-4}$-alkoxycarbonyl-, $C_{1-3}$-Alkoxy-$C_{2-4}$-alkoxy-$C_{2-4}$-alkoxycarbonyl-, $R_d$-CO-O-($R_dCR_f$)-O-CO-, $C_{1-6}$-Alkyl-CO-NH-($R_gCR_h$)-O-CO- oder $C_{1-6}$-Alkyl-CO-O-($R_gCR_h$)-($R_gCR_h$)-O-CO-Gruppe, in denen $R_d$ bis $R_f$ wie vorstehend erwähnt definiert sind,
$R_g$ und $R_h$, die gleich oder verschieden sein können, Wasserstoffatome oder $C_{1-3}$-Alkylgruppen darstellen,
zu verstehen.

Desweiteren schließen die bei der Definition der vorstehend erwähnten gesättigten Alkyl- und Alkoxyteile, die mehr als 2 Kohlenstoffatome enthalten, auch deren verzweigte Isomere wie beispielsweise die Isopropyl-, tert.Butyl-, Isobutylgruppe etc. ein.

Bevorzugte Verbindungen sind diejenigen der allgemeinen Formel

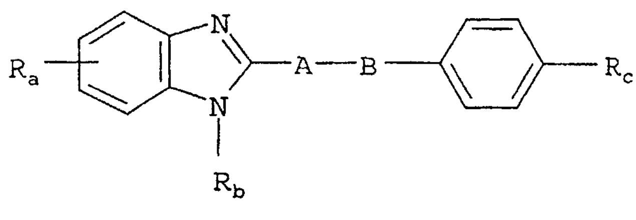

(Ia),

in der
A eine $C_{1-3}$-Alkylengruppe,
B ein Sauerstoff- oder Schwefelatom, eine Methylen-, Carbonyl-, Sulfinyl- oder Sulfonylgruppe, eine gegebenenfalls durch eine $C_{1-3}$-Alkylgruppe substituierte Iminogruppe, in der der Alkylteil durch eine Carboxygruppe mono- oder disubstituiert sein kann,
$R_a$ eine $R_1$-CO-$C_{3-5}$-cycloalkylgruppe, in der

$R_1$ eine $C_{1-3}$-Alkoxy-, Amino-, $C_{1-4}$-Alkylamino- oder Di-($C_{1-4}$-Alkyl)-aminogruppe, in denen jeweils der Alkylteil durch eine Carboxygruppe substituiert sein kann,

eine 4- bis 7-gliedrige Cycloalkylenimino- oder Cycloalkenylenyiminogruppe, die durch eine oder zwei $C_{1-3}$-Alkylgruppe substituiert sein können, wobei ein Alkylsubstituent gleichzeitig durch eine Hydroxy-, $C_{1-3}$-Alkoxy-, Carboxy-, Carboxy-$C_{1-3}$-alkoxy-, Carboxy-$C_{1-3}$-alkylamino-, N-($C_{1-3}$-Alkyl)-N-(carboxy-$C_{1-3}$-alkyl)-amino-, Carboxy-$C_{1-3}$-alkylaminocarbonyl-N- ($C_{1-3}$-Alkyl)-N- (carboxy-$C_{1-3}$-alkyl)-aminocarbonyl-, Carboxy-$C_{1-3}$-alkylaminocarbonylamino-, 1-($C_{1-3}$-Alkyl)-3-(carboxy-$C_{1-3}$-alkyl)-aminocarbonylamino-, 3- ($C_{1-3}$-Alkyl)-3- (carboxy-$C_{1-3}$-alkyl)-aminocarbonylamino- oder 1,3-Di-($C_{1-3}$-alkyl)-3-(carboxy-$C_{1-3}$-alkyl)-aminocarbonylaminogruppe substituiert sein kann,

eine durch eine Hydroxygruppe substituierte 4- bis 7-gliedrige Cycloalkyleniminogruppe, eine gegebenenfalls durch eine $C_{1-3}$-Alkylgruppe substituierte 5- bis 7-gliedrige Cycloalkyleniminogruppe, an die über zwei benachbarte Kohlenstoffatome ein Phenylring ankondensiert ist,

eine Morpholino-, Piperazino-, N-($C_{1-3}$-Alkyl)-piperazino-, Pyrrolino-, 3,4-Dehydro-piperidino- oder Pyrrol-1-yl-Gruppe darstellt,

eine $R_2$-CX-$C_{3-5}$-cycloalkylgruppe, in der

$R_2$ eine gegebenenfalls durch eine $C_{1-3}$-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine $C_{1-3}$-Alkylgruppe substituierte Iminogruppe, ein Sauerstoff- oder Schwefelatom oder eine gegebenenfalls durch eine $C_{1-3}$-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-$C_{1-3}$-alkoxy-, Carboxy-$C_{1-3}$-alkylamino- oder N-($C_{1-3}$-Alkyl)-carboxy-$C_{1-3}$-alkylaminogruppe substituiert sein kann, und

X ein Sauerstoffatom, eine $C_{1-3}$-Alkylimino-, $C_{1-3}$-Alkoxyimino-, $C_{1-3}$-Alkylhydrazino-, Di- ($C_{1-3}$-Alkyl)-hydrazino-, $C_{2-4}$-Alkanoylhydrazino-, N- ($C_{1-3}$-Alkyl)-$C_{2-4}$-alkanoylhydrazino- oder $C_{1-3}$-Alkylidengruppe, die jeweils im Alkyl- oder Alkanoylteil oder im Alkyl- und Alkanoylteil durch eine Carboxygruppe substituiert sein können, darstellen,

eine durch eine Imidazol- oder Imidazolongruppe substituierte $C_{1-3}$-Alkyl- oder $C_{3-5}$-Cycloalkylgruppe, in denen

der Imidazolring durch eine Phenyl- oder Carboxygruppe und durch eine oder zwei $C_{1-3}$-Alkylgruppen oder durch eine, zwei oder drei $C_{1-3}$-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl)-$C_{1-3}$-alkylamino- oder Di-($C_{1-3}$-Alkyl)-aminogruppe substituiert sein kann, und

der Imidazolonring durch eine $C_{1-3}$-Alkylgruppe substituiert sein kann, wobei der Alkylsubstituent durch eine Carboxygruppe oder in 2- oder 3-Stellung durch eine Amino-, $C_{2-4}$-Alkanoylamino-, $C_{1-3}$-Alkylamino-, N- ($C_{2-4}$-Alkanoyl)-$C_{1-3}$-alkylamino- oder Di-($C_{1-3}$-Alkyl)-aminogruppe substituiert sein kann, und

zusätzlich an die vorstehend erwähnten Imidazol- und Imidazolonringe über zwei benachbarte Kohlenstoffatome ein Phenyl- oder Pyridinring ankondensiert sein kann,

eine Imidazolidin-2,4-dion-5-yl-Gruppe, die durch eine oder zwei $C_{1-3}$-Alkylgruppen substituiert sein kann, wobei gleichzeitig ein Alkylsubstituent durch eine Carboxygruppe substituiert sein kann,

eine $C_{1-4}$-Alkylgruppe, die

durch eine $C_{1-3}$-Alkyl-$Y_1$-$C_{1-3}$-alkyl-, HOOC-$C_{1-3}$-alkyl-$Y_1$-$C_{1-3}$-alkyl-, Tetrazolyl-$C_{1-3}$-alkyl-$Y_2$-, $R_3NR_4$- oder $R_3NR_4$-$C_{1-3}$-alkyl-Gruppe und

durch eine gegebenenfalls durch eine $C_{1-3}$-Alkylgruppe substituierte Isoxazolidin-1-ylcarbonylgruppe, durch eine Pyrrolinocarbonyl-, 2,3-Dehydro-piperidinocarbonyl-, Pyrrol-1-yl-carbonyl-, Carboxy-, Aminocarbonyl-, $C_{1-3}$-Alkylaminocarbonyl-, Di-($C_{1-3}$-Alkyl)-aminocarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei $C_{1-3}$-Alkylgruppen substituiert und gleichzeitig jeweils ein Alkylteil oder Alkylsubstituent der vorstehend erwähnten $C_{1-3}$-Alkylaminocarbonyl-, Di-($C_{1-3}$-Alkyl)-aminocarbonyl- oder Cycloalkyleniminocarbonylgruppen durch eine Carboxygruppe substituiert sein kann, und die verbleibenden Wasserstoffatome der $C_{1-4}$-Alkylgruppe ganz oder teilweise durch Fluoratome ersetzt sein können, in denen

$R_3$ ein Wasserstoffatom oder eine gegebenenfalls durch eine Carboxygruppe substituierte $C_{1-3}$-Alkylgruppe und

$R_4$ ein Wasserstoffatom, eine $C_{1-3}$-Alkyl-$Y_1$-$C_{1-3}$-alkyl-$Y_2$-, Carboxy-$C_{1-3}$-alkyl-$Y_1$-$C_{1-3}$-alkyl-$Y_2$-, $C_{1-3}$-Alkyl-$Y_2$- oder Carboxy-$C_{1-3}$-alkyl-$Y_2$-Gruppe oder

$R_3$ und $R_4$ zusammen mit dem dazwischen liegenden Stickstoffatom eine gegebenenfalls durch eine Carboxy-, $C_{1-3}$-Alkyl- oder Carboxy-$C_{1-3}$-alkylgruppe substituierte 4- bis 7-gliedrige Cycloalkyleniminogruppe darstellen, in denen

$Y_1$ eine Kohlenstoff-Kohlenstoff-Bindung, ein Sauerstoffatom, eine Sulfenyl-, Sulfinyl-, Sulfonyl-, -NH-, -NH-CO- oder -NH-CO-NH-Gruppe und

$Y_2$ eine Kohlenstoff-Stickstoffbindung oder eine Carbonyl-, Sulfonyl-, Imino- oder -NH-CO-Gruppe darstellen,

wobei die Carbonylgruppe der -NH-CO-Gruppe mit dem Stickstoffatom der $R_3NR_4$-Gruppe verknüpft ist, und die bei der Definition der Reste $Y_1$ und $Y_2$ vorkommenden Iminogruppen jeweils zusätzlich durch eine $C_{1-3}$-Alkyl- oder Carboxy-$C_{1-3}$-alkylgruppe substituiert sein können,

eine durch eine $R_5NR_6$-Gruppe substituierte $C_{1-3}$-Alkyl- oder $C_{3-5}$-Cycloalkylgruppe, in denen

$R_5$ ein Wasserstoffatom, eine $C_{1-3}$-Alkyl-, $C_{5-7}$-Cycloalkyl-, Phenylcarbonyl-, Phenylsulfonyl- oder Pyridinylgruppe und

$R_6$ eine $C_{1-3}$-Alkyl-, Carboxy-$C_{1-3}$-alkyl- oder Carboxy-$C_{1-3}$-alkylcarbonylgruppe darstellen, eine $C_{1-3}$-Alkylgruppe, die durch $C_{2-4}$-Alkanoyl- oder $C_{5-7}$-Cycloalkanoylgruppe und durch eine durch ein Chlor-, Brom- oder Jodatom substituierte $C_{1-3}$-Alkylgruppe substituiert ist,

$R_b$ ein Wasserstoffatom oder eine $C_{1-3}$-Alkylgruppe und

$R_c$ eine Cyanogruppe oder eine Amidinogruppe, die durch eine Hydroxygruppe, durch eine oder zwei $C_{1-3}$-Alkylgruppen, durch eine oder zwei $C_{1-8}$-Alkoxycarbonylgruppen substituiert sein kann, bedeuten,

wobei die bei der Definition der vorstehend erwähnten Reste erwähnten Carboxy-, Amino- und Iminogruppen außerdem durch einen wie vorstehend erwähnten in vivo abspaltbaren Rest substituiert sein können,

deren Tautomere, deren Stereoisomere und deren Salze.

Besonders bevorzugte Verbindungen der obigen allgemeinen Formel Ia sind diejenigen, in denen

A eine $C_{1-3}$-Alkylengruppe,

B ein Sauerstoffatom, eine Methylen-, Imino- oder N-($C_{1-3}$-Alkyl)-iminogruppe, in der der Alkylteil durch eine Carboxygruppe substituiert sein kann,

$R_a$ eine in 1-Stellung durch den $R_1$-CO-Rest substituierte $C_{3-5}$-Cycloalkylgruppe, in der

$R_1$ eine $C_{1-3}$-Alkoxy-, Amino-, $C_{1-4}$-Alkylamino- oder Di-($C_{1-4}$-Alkyl)-aminogruppe, in denen jeweils der Alkylteil durch eine Carboxygruppe substituiert sein kann,

eine 4- bis 7-gliedrige Cycloalkyleniminogruppe, die durch eine Hydroxygruppe oder durch eine oder zwei $C_{1-3}$-Alkylgruppe substituiert sein kann, wobei ein Alkylsubstituent gleichzeitig durch eine Hydroxy-, $C_{1-3}$-Alkoxy-, Carboxy-, Carboxy-$C_{1-3}$-alkoxy-, Carboxy-$C_{1-3}$-alkylamino-, N- ($C_{1-3}$-Alkyl)-N- (carboxy-$C_{1-3}$-alkyl)-amino-, Carboxy-$C_{1-3}$-alkylaminocarbonyl-, N-($C_{1-3}$-Alkyl)-N-(carboxy-$C_{1-3}$-alkyl)-aminocarbonyl-, Carboxy-$C_{1-3}$-alkylaminocarbonylamino-, 1-($C_{1-3}$-Alkyl)-3- (carboxy-$C_{1-3}$-alkyl)-aminocarbonylamino-, 3- ($C_{1-3}$-Alkyl)-3- (carboxy-$C_{1-3}$-alkyl)-aminocarbonylamino- oder 1,3-Di-($C_{1-3}$-alkyl)-3-(carboxy-$C_{1-3}$-alkyl)-aminocarbonylaminogruppe substituiert sein kann,

eine gegebenenfalls durch eine $C_{1-3}$-Alkylgruppe substituierte 5- bis 7-gliedrige Cycloalkyleniminogruppe, an die über zwei benachbarte Kohlenstoffatome ein Phenylring ankondensiert ist,

eine Morpholino-, Piperazino-, N-($C_{1-3}$-Alkyl)-piperazino-, Pyrrolino-, 3,4-Dehydro-piperidino- oder Pyrrol-1-yl-Gruppe darstellt,

eine in 1-Stellung durch den $R_2$-CX-Rest substituierte $C_{3-5}$-Cycloalkylgruppe, in der

$R_2$ eine gegebenenfalls durch eine $C_{1-3}$-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine $C_{1-3}$-Alkylgruppe substituierte Iminogruppe, ein Sauerstoff- oder Schwefelatom oder eine gegebenenfalls durch eine $C_{1-3}$-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-$C_{1-3}$-alkoxy-, Carboxy-$C_{1-3}$-alkylamino- oder N-($C_{1-3}$-Alkyl)-carboxy-$C_{1-3}$-alkylaminogruppe substituiert sein kann, und

X ein Sauerstoffatom, eine $C_{1-3}$-Alkylimino-, $C_{1-3}$-Alkoxyimino- oder $C_{1-3}$-Alkylidengruppe, die jeweils im Alkyl- oder Alkoxyteil durch eine Carboxygruppe substituiert sein können, darstellen,

eine in 1-Stellung durch eine Imidazol- oder Imidazolongruppe substituierte $C_{1-3}$-Alkylgruppe, in denen

der Imidazolring durch eine Phenyl- oder Carboxygruppe und durch eine oder zwei $C_{1-3}$-Alkylgruppen oder durch eine, zwei oder drei $C_{1-3}$-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl) -$C_{1-3}$-alkylamino- oder Di-($C_{1-3}$-Alkyl)-aminogruppe substituiert sein kann, und

der Imidazolonring durch eine $C_{1-3}$-Alkylgruppe substituiert sein kann, wobei der Alkylsubstituent durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl) -$C_{1-3}$-alkylamino- oder Di-($C_{1-3}$-Alkyl)-aminogruppe substituiert sein kann, und

zusätzlich an die vorstehend erwähnten Imidazol- und Imidazolonringe über zwei benachbarte Kohlenstoffatome ein Phenyl- oder Pyridinring ankondensiert sein kann,

eine Imidazolidin-2,4-dion-5-yl-Gruppe, die durch eine oder zwei $C_{1-3}$-Alkylgruppen substituiert sein kann, wobei gleichzeitig ein Alkylsubstituent durch eine Carboxygruppe substituiert sein kann,

eine $C_{1-4}$-Alkylgruppe, die in 1-Stellung

durch eine $R_3NR_4$- oder $R_3NR_4$-$C_{1-3}$-alkyl-Gruppe und

durch eine Pyrrolinocarbonyl-, 2,3-Dehydro-piperidinocarbonyl-, Imidazol-1-yl-carbonyl-, Carboxy-, Aminocarbo-

nyl-, $C_{1-3}$-Alkylaminocarbonyl-, Di- ($C_{1-3}$-Alkyl)-aminocarbonyl-, Isoxazolidin-1-ylcarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei $C_{1-3}$-Alkylgruppen substituiert und gleichzeitig jeweils ein Alkylteil oder Alkylsubstituent der vorstehend erwähnten $C_{1-3}$-Alkylaminocarbonyl-, Di-($C_{1-3}$-Alkyl)-aminocarbonyl- oder Cycloalkyleniminocarbonylgruppen durch eine Carboxygruppe substituiert sein kann, und die verbleibenden Wasserstoffatome der $C_{1-4}$-Alkylgruppe ganz oder teilweise durch Fluoratome ersetzt sein können, in denen

$R_3$ ein Wasserstoffatom oder eine gegebenenfalls durch eine Carboxygruppe substituierte $C_{1-3}$-Alkylgruppe und

$R_4$ ein Wasserstoffatom, $C_{1-3}$-Alkyl-$Y_2$- oder Carboxy-$C_{1-3}$-alkyl-$Y_2$-Gruppe oder

$R_3$ und $R_4$ zusammen mit dem dazwischen liegenden Stickstoffatom eine gegebenenfalls in 1-Stellung durch eine Carboxy-, $C_{1-3}$-Alkyl- oder Carboxy-$C_{1-3}$-alkylgruppe substituierte 4-bis 7-gliedrige Cycloalkyleniminogruppe darstellen, in denen

$Y_2$ eine Kohlenstoff-Stickstoffbindung oder eine Carbonyl-, Imino- oder -NH-CO-Gruppe darstellt, wobei die Carbonylgruppe der -NH-CO-Gruppe mit dem Stickstoffatom der $R_3NR_4$-Gruppe verknüpft ist, und die bei der Definition des Restes $Y_2$ vorkommende Iminogruppe zusätzlich durch eine $C_{1-3}$-Alkyl- oder Carboxy-$C_{1-3}$-alkylgruppe substituiert sein können,

eine in 1-Stellung durch eine $R_5NR_6$-Gruppe substituierte $C_{1-3}$-Alkyl- oder $C_{3-5}$-Cycloalkylgruppe, in denen

$R_5$ ein Wasserstoffatom, eine $C_{1-3}$-Alkyl-, $C_{5-7}$-Cycloalkyl-, Phenylcarbonyl-, Phenylsulfonyl- oder Pyridinylgruppe und

$R_6$ eine $C_{1-3}$-Alkyl-, Carboxy-$C_{1-3}$-alkyl- oder Carboxy-$C_{1-3}$-alkylcarbonylgruppe darstellen,

eine $C_{1-3}$-Alkylgruppe, die durch $C_{2-4}$-Alkanoyl- oder $C_{5-7}$-Cycloalkanoylgruppe und durch eine durch ein Chlor-, Brom- oder Jodatom substituierte $C_{1-3}$-Alkylgruppe substituiert ist,

$R_b$ eine $C_{1-3}$-Alkylgruppe und

$R_c$ eine gegebenenfalls durch eine 2,2,2-Trichlorethoxycarbonyl-, $C_{1-8}$-Alkoxycarbonyl-, Acetoxymethyloxycarbonyl-, Benzyloxycarbonyl- oder Benzoylgruppe substituierte Amidinogruppe bedeuten, wobei der Benzoylteil durch Fluor-, Chlor-, Brom- oder Jodatome, durch $C_{1-3}$-Alkyl- oder $C_{1-3}$-Alkoxygruppen mono- oder disubstituiert und die Substituenten gleich oder verschieden sein können,

deren $C_{1-3}$-Alkanolester, deren Tautomere, deren Stereoisomere und deren Salze.

Ganz besonders bevorzugte Verbindungen der allgemeinen Formel I sind diejenigen, in denen

A eine Methylengruppe,

B ein Sauerstoffatom oder eine Iminogruppe,

$R_a$ eine in 1-Stellung durch den $R_1$-CO-Rest substituierte Cyclopropylgruppe, in der

$R_1$ eine gegebenenfalls durch eine Methyl- oder Ethylgruppe substituierte Pyrrolidino- oder Piperidinogruppe, in denen jeweils der Methyl- oder Ethylteil durch eine Carboxy-, Carboxy-$C_{1-3}$-alkoxy-, Carboxy-$C_{1-3}$-alkylamino- oder N-($C_{1-3}$-Alkyl)-carboxy-$C_{1-3}$-alkylaminogruppe substituiert sein kann, darstellt,

eine in 1-Stellung durch den $R_2$-CX-Rest substituierte Cyclopropylgruppe, in der

$R_2$ eine gegebenenfalls durch eine $C_{1-3}$-Alkylgruppe substituierte Phenyl-, Pyridyl- Pyrazolylgruppe und

X ein Sauerstoffatom, eine $C_{1-3}$-Alkoxyimino- oder $C_{1-3}$-Alkylidengruppe, die jeweils im Alkyl- oder Alkoxyteil durch eine Carboxygruppe substituiert sind, darstellen,

eine in 1-Stellung durch eine Imidazolgruppe substituierte $C_{1-2}$-Alkylgruppe, in der der Imidazolring durch eine Phenyl - oder Carboxygruppe und durch eine oder zwei $C_{1-3}$-Alkylgruppen oder durch eine, zwei oder drei $C_{1-3}$-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, $C_{2-4}$-Alkanoylamino-, $C_{1-3}$-Alkylamino-, N- ($C_{2-4}$-Alkanoyl)-$C_{1-3}$-alkylamino- oder Di- ($C_{1-3}$-Alkyl)-aminogruppe substituiert sein kann, wobei zusätzlich an die vorstehend erwähnten Imidazolringe über zwei benachbarte Kohlenstoffatome ein Phenyl- oder Pyridinring ankondensiert sein kann,

eine in 1-Stellung durch eine Benzimidazolon-1-yl-Gruppe substituierte $C_{1-2}$-Alkylgruppe, wobei der Imidazolonring durch eine gegebenenfalls durch eine Carboxygruppe substituierte Methyl- oder Ethylgruppe substituiert sein kann, darstellen,

eine Methyl- oder Ethylgruppe, die in 1-Stellung

durch eine $R_3NR_4$- oder $R_3NR_4$-$C_{1-3}$-alkyl-Gruppe und

durch eine Di-($C_{1-3}$-Alkyl)-aminocarbonylgruppe, durch eine Isoxazolidin-1-ylcarbonylgruppe, durch eine gegebenenfalls durch eine $C_{1-3}$-Alkylgruppe substituierte Pyrrolidinocarbonyl- oder Piperidinocarbonylgruppe, wobei bei den vorstehend erwähnten Gruppen jeweils ein Alkylteil oder Alkylsubstituent durch eine Carboxygruppe substituiert sein kann, in denen

$R_3$ ein Wasserstoffatom oder eine gegebenenfalls durch eine Carboxygruppe substituierte $C_{1-3}$-Alkylgruppe und

$R_4$ ein Wasserstoffatom, eine $C_{1-3}$-Alkyl-$Y_2$- oder Carboxy-$C_{i-3}$-alkyl-$Y_2$-Gruppe oder

$R_3$ und $R_4$ zusammen mit dem dazwischen liegenden Stickstoffatom eine gegebenfalls durch eine Carboxygruppe substituierte 4- bis 7-gliedrige Cycloalkyleniminogruppe darstellen, in denen

$Y_2$ eine Kohlenstoff-Stickstoffbindung, eine Carbonylgruppe oder eine gegebenenfalls durch eine $C_{1-3}$-Alkylgruppe substituierte Iminogruppe darstellt,

eine in 1-Stellung durch eine $R_5NR_6$-Gruppe substituierte $C_{1-2}$-Alkylgruppe, in der

$R_5$ eine Pyridinyl-, Phenylcarbonyl- oder Phenylsulfonylgruppe und

$R_6$ eine $C_{1-3}$-Alkyl- oder Carboxy-$C_{1-3}$-alkylgruppe darstellen,

eine durch eine in 3-Stellung durch ein Chloratom substituierte n-Propylgruppe, die in 1-Stellung durch eine Cyclopentylcarbonylgruppe substituiert ist,

eine in 1-Stellung durch eine Cyclopentylaminogruppe substituierte Cyclopropylgruppe, die am Stickstoffatom durch eine Carboxy-$C_{1-3}$-alkylcarbonylgruppe substituiert ist,

$R_b$ eine Methylgruppe und

$R_c$ eine gegebenenfalls durch eine $C_{1-8}$-Alkoxycarbonyl-, Acetoxymethyloxycarbonyl-, 2,2,2-Trichlorethoxycarbonyl-, Benzyloxycarbonyl- oder Benzoylgruppe substituierte Amidinogruppe bedeuten,

insbesondere diejenigen Verbindungen der allgemeinen Formel Ia, in denen

A eine Methylengruppe,

B eine Iminogruppe,

$R_a$ eine in 1-Stellung durch den $R_1$-CO-Rest substituierte Cyclopropylgruppe, in der

$R_1$ eine gegebenenfalls durch eine Methyl- oder Ethylgruppe substituierte Pyrrolidino- oder Piperidinogruppe, in denen jeweils der Methyl- oder Ethylteil durch eine Carboxy-, Carboxy-$C_{1-3}$-alkoxy-, Carboxy-$C_{1-3}$-alkylamino- oder N-($C_{1-3}$-Alkyl)-carboxy-$C_{1-3}$-alkylaminogruppe substituiert sein kann, darstellt,

eine in 1-Stellung durch den $R_2$-CX-Rest substituierte Cyclopropylgruppe, in der

$R_2$ eine gegebenenfalls durch eine $C_{1-3}$-Alkylgruppe substituierte Phenyl-, Pyridyl- Pyrazolylgruppe und

X ein Sauerstoffatom, eine $C_{1-3}$-Alkoxyimino- oder $C_{1-3}$-Alkylidengruppe, die jeweils im Alkyl- oder Alkoxyteil durch eine Carboxygruppe substituiert sind, darstellen,

eine in 1-Stellung durch eine Imidazolgruppe substituierte $C_{1-2}$-Alkylgruppe, in der der Imidazolring durch 1 bis 3 Methylgruppen substituiert sein kann oder durch zwei Methylgruppen und eine Ethylgruppe substituiert ist, wobei zusätzlich einer der vorstehend erwähnten Methyl- oder Ethylsubstituenten gleichzeitig durch eine Carboxygruppe substituiert sein kann,

eine Methyl- oder Ethylgruppe, die in 1-Stellung

durch eine $R_3NR_4$- oder $R_3NR_4$-$CH_2$-Gruppe und

durch eine Di-($C_{1-3}$-Alkyl)-aminocarbonylgruppe, eine gegebenenfalls durch eine $C_{1-3}$-Alkylgruppe substituierte Pyrrolidinocarbonyl- oder Piperidinocarbonylgruppe, wobei bei den vorstehend erwähnten Gruppen jeweils ein Alkylteil oder Alkylsubstituent durch eine Carboxygruppe substituiert sein kann, in denen

$R_3$ ein Wasserstoffatom oder eine gegebenenfalls durch eine Carboxygruppe substituierte $C_{1-3}$-Alkylgruppe und

$R_4$ eine $C_{1-3}$-Alkyl-$Y_2$- oder Carboxy-$C_{1-3}$-alkyl-$Y_2$-Gruppe darstellen, in denen

$Y_2$ eine Kohlenstoff-Stickstoffbindung, eine Carbonylgruppe oder eine gegebenenfalls durch eine $C_{1-3}$-Alkylgruppe substituierte Iminogruppe darstellt,

$R_b$ eine Methylgruppe und

$R_c$ eine gegebenenfalls durch eine $C_{1-8}$-Alkoxycarbonyl-, Acetoxymethyloxycarbonyl-, 2,2,2-Trichlorethoxycarbonyl-, Benzyloxycarbonyl- oder Benzoylgruppe substituierte Amidinogruppe bedeuten,

wobei insbesondere diejenigen vorstehend erwähnten Verbindungen der allgemeinen Formel I bevorzugt sind, in denen der Rest $R_a$ in 5-Stellung steht,

deren $C_{1-3}$-Alkanolester, deren Tautomere, deren Stereoisomere und deren Salze.

Als besonders bevorzugte Verbindungen seien beispielsweise folgende erwähnt:

(a) 2-(4-Amidinophenylaminomethyl)-1-methyl-5-[1-(pyrrolidin-1-yl-carbonyl)cyclopropyl]-benzimidazol,

(b) (E/Z)-2-(4-Amidinophenylaminomethyl)-1-methyl-5-[1-[(pyridin-2-yl)-(carboxymethyloxyimino)methylen]cyclopropyl]-benzimidazol,

(c) 2-(4-Amidinophenylaminomethyl)-1-methyl-5-[1-(2-carboxyethylamino)-1-(pyrrolidin-1-yl-carbonyl)-ethyl]-benzimidazol,

(d) 2-(4-Amidinophenylaminomethyl)-1-methyl-5-[1-[2-(2-carboxyethyl)-pyrrolidin-1-yl-carbonyl]cyclopropyl]-benzimidazol,

(e) 2-(4-Amidinophenylaminomethyl)-1-methyl-5-[2-(2-carboxyethyl)-4,5-dimethyl-imidazol-1-yl-methyl]-benzimidazol

(f) 2-(4-Amidinophenylaminomethyl)-1-methyl-5-[1-(carboxymethylamino)-1-(pyrrolidin-1-yl-carbonyl)-ethyl]-benzimidazol und

(g) 2-(4-Amidinophenylaminomethyl)-1-methyl-5-[1-(N-methylcarboxymethylcarbonylaminomethyl)-1-methyl-1-(pyrrolidin-1-yl-carbonyl)-ethyl]-benzimidazol

sowie deren $C_{1-3}$-Alkanolester, deren N-($C_{1-8}$-Alkoxycarbonyl)-, N-Benzyloxycarbonyl- und N-Benzoyl-amidine, deren Tautomere, deren Stereoisomere und deren Salze.

Erfindungsgemäß erhält man die Verbindungen der allgemeinen Formel I nach an sich bekannten Verfahren, beispielsweise nach folgenden Verfahren:

a) Zur Herstellung einer Verbindung der allgemeinen Formel I, in der $R_c$ eine Cyanogruppe darstellt:

Cyclisierung einer gegebenenfalls im Reaktionsgemisch gebildeten Verbindung der allgemeinen Formel

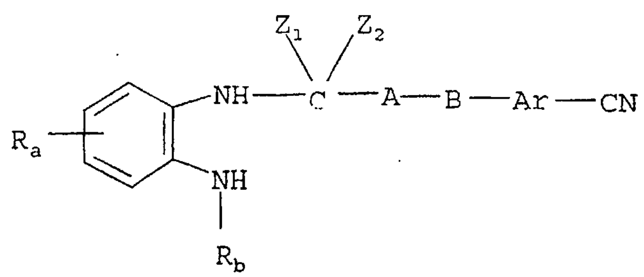

(II),

in der
$R_a$, $R_b$, Ar, A und B wie eingangs erwähnt definiert sind,
$Z_1$ und $Z_2$, die gleich oder verschieden sein können, gegebenenfalls durch Alkylgruppen mit 1 bis 6 Kohlenstoffatomen substituierte Amino-, Hydroxy- oder Mercaptogruppen oder
$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester oder Amid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäure, Essigsäureanhydrid, N,N-Dicyclohexyl-carbodiimid oder gegebenenfalls auch in Gegenwart einer Base wie Kalium-ethylat oder Kalium-tert.butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der allgemeinen Formel II im Reaktionsgemisch durch Reduktion einer entsprechenden o-Nitro-verbindung gegebenenfalls in Gegenwart einer Carbonsäure der allgemeinen Formel

$$HO\text{-}CO—A—B—Ar—CN \qquad (III),$$

in der
Ar, A und B wie eingangs erwähnt definiert sind, durch Acylierung einer gegebenenfalls im Reaktionsgemisch gebildeten entsprechenden Aminoverbindung hergestellt wird.

b) Zur Herstellung einer Verbindung der allgemeinen Formel I, in der $R_a$ eine $R_2$-CX'-$C_{3-5}$-cycloalkylengruppe bedeutet, in der $R_2$ wie eingangs erwähnt definiert ist und X' einen der für X eingangs erwähnten Iminoreste darstellt:

Umsetzung einer Verbindung der allgemeinen Formel

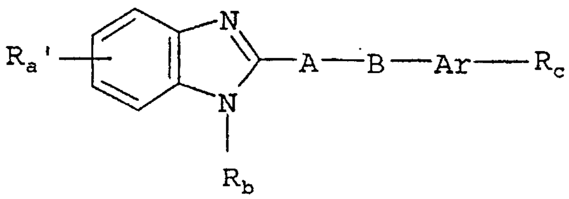

(IV),

in der
$R_b$, $R_c$, Ar, A und B wie eingangs erwähnt definiert sind und
$R_a$' eine $R_2$-CO-$C_{3-5}$-cycloalkylengruppe darstellt, wobei
$R_2$ wie eingangs erwähnt definiert ist,
mit einem Amin der allgemeinen Formel

$$H_2X' \qquad (V),$$

in der
X' eine der für X eingangs erwähnten Iminoreste darstellt.

Die Umsetzung wird vorzugsweise in einem Lösungsmittel wie Methanol/Toluol, Ethanol, Isopropanol oder Xylol und zweckmäßigerweise in Gegenwart eines wasserentziehenden Mittels wie Molekularsieb, Natriumsulfat oder Calciumchlorid gegebenenfalls in Gegenwart einer Base wie Triethylamin bei Temperaturen zwischen 50 und 100°C, vorzugsweise bei der Siedetemperatur des Reaktionsgemisches, durchgeführt.

c) Zur Herstellung einer Verbindung der allgemeinen Formel I, in der $R_a$ eine $R_2$-CX"-$C_{3-5}$-cycloalkylengruppe bedeutet, in der $R_2$ wie eingangs erwähnt definiert ist und X" einen der für X eingangs erwähnten Alkylidenreste darstellt:

Umsetzung einer Verbindung der allgemeinen Formel

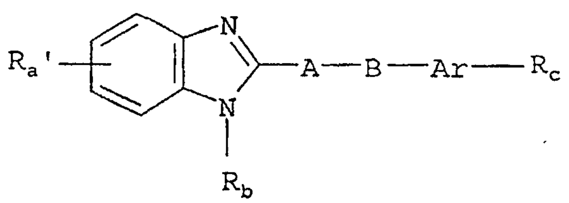

(IV),

in der
$R_b$, $R_c$; Ar, A und B wie eingangs erwähnt definiert sind und
$R_a$' eine $R_2$-CO-$C_{3-5}$-cycloalkylengruppe darstellt, wobei
$R_2$ wie eingangs erwähnt definiert ist,
mit einem Phosphon der allgemeinen Formel

$$Z_3\text{-}HX'' \qquad (VI),$$

in der
X" einen der für X eingangs erwähnten Alkylidenreste und
$Z_3$ eine Triphenylphosphono- oder Di-($C_{1-3}$-alkoxy)phosphonogruppe wie die Triethoxyphosphonogruppe darstellt.

Die Umsetzung wird vorzugsweise unter Schutzgas in einem Lösungsmittel wie Tetrahydrofuran, Dimethylformamid, Dioxan, Diethylether oder Dimethylsulfoxid in Gegenwart einer Base wie Kalium-tert.butylat, Natriumethylat oder Natriumhydrid bei Temperaturen zwischen -25 und 50°C, vorzugsweise bei Temperaturen zwischen -15° und der Raumtemperatur, durchgeführt.

d) Zur Herstellung einer Verbindung der allgemeinen Formel I, in der $R_c$ eine Amidinogruppe, die durch eine oder zwei $C_{1-3}$-Alkylgruppen substituiert sein kann:

Umsetzung einer gegebenenfalls im Reaktionsgemisch gebildeten Verbindung der allgemeinen Formel

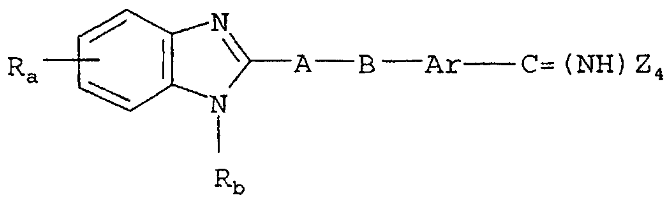

(VII),

in der

$R_a$, $R_b$, Ar, A und B wie eingangs erwähnt definiert sind und

$Z_4$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe darstellt, mit einem Amin der allgemeinen Formel

$$H - R_7NR_8 \quad ,(VIII)$$

in der

$R_7$ und $R_8$, die gleich oder verschieden sein können, jeweils ein Wasserstoffatom oder eine $C_{1-3}$-Alklylgruppe bedeuten, oder mit dessen Salzen.

Die Umsetzung wird zweckmäßigerweise in einem Lösungsmittel wie Methanol, Ethanol, n-Propanol, Tetrahydrofuran oder Dioxan bei Temperaturen zwischen 0 und 150°C, vorzugsweise bei Temperaturen zwischen 0 und 80°C, mit einem Amin der allgemeinen Formel VIII oder mit einem entsprechenden Säureadditionssalz wie beispielsweise Ammoniumcarbonat oder Ammoniumacetat durchgeführt.

Eine Verbindung der allgemeinen Formel VII erhält man beispielsweise durch Umsetzung einer entsprechenden Cyanoverbindung mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen 0 und 50°C, vorzugsweise jedoch bei 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

e) Zur Herstellung einer Verbindung der allgemeinen Formel I, in der $R_a$ eine Imidazolidin-2,4-dion-5-yl-Gruppe, die durch eine oder zwei $C_{1-3}$-Alkylgruppen substituiert sein kann, wobei gleichzeitig ein Alkylsubstituent durch eine Carboxy- oder $C_{1-3}$-Alkoxycarbonylgruppe substituiert sein kann:

Cyclisierung einer gegebenenfalls im Reationsgemisch gebildeten Verbindung der allgemeinen Formel

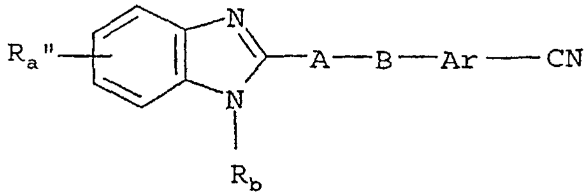

(IX),

in der
$R_b$, Ar, A und B wie eingangs erwähnt definiert sind und
$R_a$" eine Aminocarbonyaminogruppe, die in 3-Stellung durch eine $C_{1-3}$-Alkoxycarbonyl-$C_{1-3}$-alkylgruppe substituiert ist, bedeutet.

Die Umsetzung wird vorzugsweise in einem Lösungsmittel wie Methanol, Ethanol, n-Propanol, Isopropanol oder Benzylalkohol in Gegenwart einer Säure wie Salzsäure bei Temperaturen zwischen 0 und 50°C, vorzugsweise jedoch bei 20°C, durchgeführt.

f) Zur Herstellung einer Verbindung der allgemeinen Formel I, in der $R_c$ eine Hydroxyamidinogruppe darstellt:

Umsetzung eines Nitrils der allgemeinen Formel

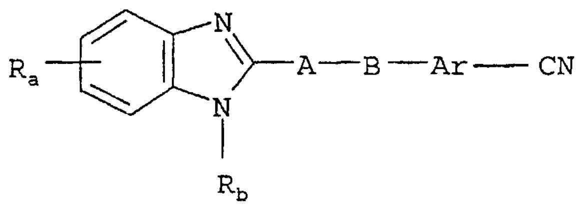

(X),

in der
$R_a$, $R_b$, Ar, A und B wie eingangs erwähnt definiert sind, mit Hydroxylamin oder dessen Salzen.

Die Umsetzung wird zweckmäßigerweise in einem Lösungsmittel wie Methanol, Ethanol, n-Propanol, Wasser, Methanol/Wasser, Tetrahydrofuran, Tetrahydrofuran/Wasser, Dioxan oder Dioxan/Wasser bei Temperaturen zwischen 0 und 150°C, vorzugsweise bei Temperaturen zwischen 0 und 80°C, durchgeführt.

g) Zur Herstellung einer Verbindung der allgemeinen Formel I, in der $R_a$ eine Carboxygruppe enthält und $R_c$ wie eingangs erwähnt definiert ist oder $R_a$ wie eingangs erwähnt definiert ist und $R_c$ eine gegebenenfalls durch eine Hydroxygruppe oder durch eine oder zwei $C_{1-3}$-Alkylgruppen substituierte Amidinogruppe darstellt:

Überführung einer Verbindung der allgemeinen Formel

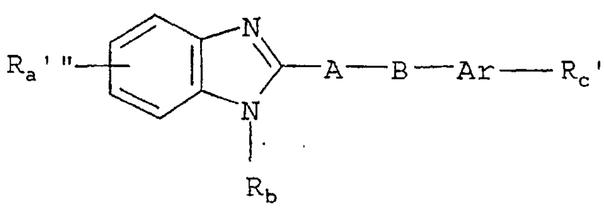

(XI),

in der
$R_b$, Ar, A und B wie eingangs erwähnt definiert sind und
$R_a$' " und $R_c$' die für $R_a$ und $R_c$ eingangs erwähnten Bedeutungen mit der Maßgabe besitzen, daß $R_a$ eine durch Hydrolyse, Behandeln mit einer Säure oder Base, Thermolyse oder Hydrogenolyse in eine Carboxygruppe überführbare Gruppe enthält und $R_c$ wie eingangs erwähnt definiert ist oder $R_c$ eine durch Hydrolyse, Behandeln mit einer Säure oder Base, Thermolyse oder Hydrogenolyse in eine gegebenenfalls durch eine Hydroxygruppe oder durch eine oder zwei $C_{1-3}$-Alkylgruppen substituierte Amidinogruppe überführbare Gruppe darstellt und $R_a$ wie eingangs erwähnt definiert ist,
mittels Hydrolyse, Behandeln mit einer Säure oder Base, Thermolyse oder Hydrogenolyse in eine Verbindung der allgemeinen Formel I übergeführt wird, in der $R_a$ eine Carboxygruppe enthält und $R_c$ wie eingangs erwähnt definiert ist oder $R_a$ wie eingangs erwähnt definiert ist und $R_c$ eine gegebenenfalls durch eine Hydroxygruppe oder durch eine oder zwei $C_{1-3}$-Alkylgruppen substituierte Amidinogruppe darstellt.

Als eine in eine Carboxygruppe überführbare Gruppe kommt beispielsweise eine durch einen Schutzrest geschützte Carboxylgruppe wie deren funktionelle Derivate, z. B. deren unsubstituierte oder substituierte Amide, Ester, Thioester, Trimethylsilylester, Orthoester oder Iminoester, welche zweckmäßigerweise mittels Hydrolyse in eine Carboxylgruppe übergeführt werden,
deren Ester mit tertiären Alkoholen, z.B. der tert.Butylester, welche zweckmäßigerweise mittels Behandlung mit einer Säure oder Thermolyse in eine Carboxylgruppe übergeführt werden, und
deren Ester mit Aralkanolen, z.B. der Benzylester, welche zweckmäßigerweise mittels Hydrogenolyse in eine Carboxylgruppe übergeführt werden, in Betracht.

Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemischen oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Enthält eine Verbindung der Formel XI beispielsweise die tert.Butyl- oder tert.Butyloxycarbonylygruppe, so können diese auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden.

Enthält eine Verbindung der Formel XI beispielsweise die Benzyloxy- oder Benzyloxycarbonylgruppe, so können diese auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden.

h) Zur Herstellung einer Verbindung der allgemeinen Formel I, in der $R_c$ eine Amidinogruppe, die durch eine oder zwei $C_{1-8}$-Alkoxycarbonylgruppen oder durch einen in vivo abspaltbaren Rest substituiert ist:

Umsetzung einer Verbindung der allgemeinen Formel I

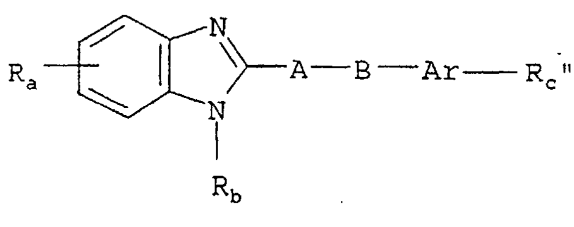

(XII),

in der
$R_a$, $R_b$, Ar, A und B wie eingangs erwähnt definiert sind und
$R_c$" eine Amidinogruppe bedeutet, mit einer Verbindung der allgemeinen Formel

$$Z_5 - R_9 \quad \text{(XIII),}$$

in der

$R_9$ eine $C_{1-8}$-Alkoxycarbonylgruppe oder den Acylrest einer der eingangs erwähnten in vivo abspaltbaren Reste und

$Z_5$ eine nukleofuge Austrittsgruppe wie ein Halogenatom, z.B. ein Chlor-, Brom- oder Jodatom, oder eine p-Nitrophenylgruppe bedeuten.

Die Umsetzung wird vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt.

Mit einer Verbindung der allgemeinen Formel XIII, in der $Z_5$ eine nukleofuge Austrittsgruppe darstellt, wird die Umsetzung vorzugsweise in einem Lösungsmittel wie Methylenchlorid, Acetonitril, Tetrahydrofuran, Toluol, Aceton/Wasser, Dimethylformamid oder Dimethylsulfoxid gegebenenfalls in Gegenwart einer Base wie Natrimhydrid, Kaliumcarbonat, Kalium-tert.butylat oder N-Ethyl-diisopropylamin bei Temperaturen zwischen 0 und 60°C, durchgeführt.

Erhält man erfindungsgemäß eine Verbindung der allgemeinen Formel I, die eine $(R_3NR_4)$-$C_{1-3}$-alkylgruppe enthält, in der mindestens einer der Reste $R_3$ oder $R_4$ ein Wasserstoffatom darstellt, so kann diese anschließend mit einem entsprechenden Isocyanat oder Carbamoylhalogenid in eine entsprechende Harnstoffverbindung der allgemeinen Formel I übergeführt werden und/oder

eine Verbindung der allgemeinen Formel I, die eine $NH_2$-$C_{1-3}$-alkylgruppe enthält, so kann diese anschließend mit einem entsprechenden Acrylsäureester in eine entsprechende 2-($C_{1-3}$-Alkoxycarbonyl)-ethyl-Verbindung der allgemeinen Formel I übergeführt werden und/oder

eine Verbindung der allgemeinen Formel I, die eine $(R_3NR_4)$-$C_{1-3}$-alkylgruppe enthält, in der $R_3$ und $R_4$ jeweils ein Wasserstoffatom darstellen, so kann diese anschließend mit einem entsprechenden Dihalogenalkan in eine entsprechende Verbindung der allgemeinen Formel I, in der $R_3$ und $R_4$ zusammen mit dem dazwischen liegenden Stickstoffatom eine entsprechende 4- bis 7-gliedrige Cycloalkyleniminogruppe darstellt, übergeführt werden und/oder

eine Verbindung der allgemeinen Formel I, in der $R_c$ eine Amidinogruppe darstellt, so kann diese anschließend durch Umsetzung mit einem Halogenessigsäurederivat sowie anschließender Hydrolyse und Decarboxylierung in eine durch eine oder zwei Methylgruppen substituierte entsprechende Amidinoverbindung übergeführt werden und/oder

eine Verbindung der allgemeinen Formel I, in der $R_c$ eine Hydroxyamidinogruppe darstellt, so kann diese anschließend mittels katalytischer Hydrierung in eine entsprechende Amidinoverbindung übergeführt werden und/oder

eine Verbindung der allgemeinen Formel I, in der $R_a$ eine Carboxygruppe enthält, so kann diese anschließend mittels Veresterung in einen entsprechenden Ester übergeführt werden.

Die anschließende Herstellung einer entsprechenden Harnstoffverbindung der allgemeinen Formel I wird zweckmäßigerweise mit einem entsprechenden Isocyanat oder Carbamoylchlorid vorzugsweise in einem Lösungsmittel wie Dimethylformamid und gegebenenfalls in Gegenwart einer tertiären organischen Base wie - Triethylamin bei Temperaturen zwischen 0 und 50°C, vorzugsweise bei der Raumtemperatur, durchgeführt.

Die anschließende Herstellung einer entsprechenden 2-($C_{1-3}$-Alkoxycarbonyl)-ethyl-Verbindung wird mit einem entsprechenden Acrylsäureester vorzugsweise in einem Lösungsmittel wie Methanol, Ethanol oder Isopropanol bei Temperaturen zwischen 50 und 100°C, vorzugsweise bei der Siedetemperatur des Reaktionsgemisches, durchgeführt.

Die anschließende Herstellung einer entsprechenden 4- bis 7-gliedrigen Cycloalkylenimino-Verbindung der allgemeinen Formel I wird zweckmäßigerweise mit einem entsprechenden Dihalogenalkan vorzugsweise in einem Lösungsmittel wie Methanol, Ethanol oder Isopropanol in Gegenwart einer Base wie Natriumcarbonat bei Temperaturen zwischen 50 und 100°C, vorzugsweise bei der Siedetemperatur des Reaktionsgemisches, durchgeführt.

Die anschließende Alkylierung wird zweckmäßigerweise in einem Lösungsmittel wie Methylenchlorid, Tetrahydrofuran, Dioxan, Dimethylsulfoxid, Dimethylformamid oder Aceton gegebenenfalls in Gegenwart eines Reaktionsbeschleunigers wie Natrium- oder Kaliumiodid und vorzugsweise in Gegenwart einer Base wie Natriumcarbonat oder Kaliumcarbonat oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

Die nachträgliche Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemischen oder in Gegenwart einer Base wie Lithiumhydroxid, Natriumhydroxid oder Kaliumhydroxid in einem geeigneten Lösungsmittel

wie Wasser, Wasser/Methanol, Wasser/Ethanol, Wasser/Isopropanol, Methanol, Ethanol, Wasser/Tetrahydrofuran oder Wasser/Dioxan und die anschließende Decarboxylierung in Gegenwart einer Säure wie vorstehend beschrieben bei Temperaturen zwischen -10 und 120°C, z.B. bei Temperaturen zwischen Raumtemperatur und der Siedetemperatur des Reaktionsgemisches, durchgeführt.

Die nachträgliche Veresterung wird mit einem entsprechenden Alkohol zweckmäßigerweise in einem Lösungsmittel oder Lösungsmittelgemisch wie Methylenchlorid, Benzol, Toluol, Chlorbenzol, Tetrahydrofuran, Benzol/Tetrahydrofuran oder Dioxan, vorzugsweise jedoch in einem Überschuß des eingesetzten Alkohols, gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol- oder N,N'-Thionyldiimidazol, Triphenylphosphin/Tetrachlorkohlenstoff oder Triphenylphosphin/Azodicarbonsäurediethylester gegebenenfalls in Gegenwart einer Base wie Kaliumcarbonat, N-Ethyl-diisopropylamin oder N,N-Dimethylamino-pyridin zweckmäßigerweise bei Temperaturen zwischen 0 und 150°C, vorzugsweise bei Temperaturen zwischen 0 und 80°C, oder mit einem entsprechenden Halogenid in einem Lösungsmittel wie Methylenchlorid, Tetrahydrofuran, Dioxan, Dimethylsulfoxid, Dimethylformamid oder Aceton. gegebenenfalls in Gegenwart eines Reaktionsbeschleunigers wie Natrium- oder Kaliumiodid und vorzugsweise in Gegenwart einer Base wie Natriumcarbonat oder Kaliumcarbonat oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)-chlorid in einem Lösungsmittel wie wässrigem Ethanol und gegebenenfalls in Gegenwart einer Base wie 1,4-Diazabicyclo-[2.2.2]octan bei Temperaturen zwischen 20 und 70°C.

Die als Ausgangsstoffe verwendeten Verbindungen der allgemeinen Formeln II bis XIII, welche teilweise literaturbekannt sind, erhält man nach literaturbekannten Verfahren, desweiteren wird ihre Herstellung in den Beispielen beschrieben.

Die Chemie der Verbindungen der allgemeinen Formel III wird beispielsweise von Jack Robinson in J. Chem. Soc. 1941, 744, die der Benzimidazole von Katritzky und Rees in Comprehensive Heterocyclic Chemistry, Oxford, Pergamon Press, 1984, von Schaumann in Hetarene III, Methoden der organischen Chemie (Houben-Weyl), 4. Auflage, Verlag Thieme, Stuttgart 1993, beschrieben.

So erhält man beispielsweise eine Verbindung der allgemeinen Formel II durch Acylierung einer entsprechenden o-Diaminoverbindung mit einem entsprechenden reaktionsfähigen Derivat einer Verbindung der allgemeinen Formel III,
eine Verbindung der allgemeinen Formeln IV, VII, IX, X, XI und XII durch Cyclisierung einer entsprechenden substituierten Verbindung gemäß Verfahren a) und erforderlichenfalls anschließende Reduktion einer im Phenylteil vorhandenen Nitrogruppe sowie anschließender Acylierung, Amidierung und/oder Halogenierung.

Ferner können die erhaltenen Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die neuen Verbindungen der allgemeinen Formel I und deren Salze wertvolle Eigenschaften auf. So stellen die Verbindungen der allgemeinen Formel I, in denen $R_c$ eine Cyanogruppe darstellt, wertvolle Zwischenprodukte zur Herstellung der übrigen Verbindungen der allgemeinen Formel I dar, und die Verbindungen der allgemeinen Formel I, in denen $R_c$ eine der eingangs erwähnten Amidinogruppen darstellt, sowie deren Tautomeren, deren Stereoisomeren und deren physiologisch verträglichen Salze weisen wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xa-hemmenden Wirkung, auf einer die aPTT-Zeit verlängernden Wirkung und auf einer Hemmwirkung auf verwandte Serinproteasen wie z. B. Trypsin, Urokinase Faktor VIIa, Faktor IX, Faktor XI und Faktor XII.

Beispielsweise wurden die Verbindungen

A = 2-(4-Amidinophenylaminomethyl)-1-methyl-5-[1-(pyrrolidin-1-yl-carbonyl)cyclopropyl]-benzimidazol-hydrochlorid,

B = (E/Z)-2-(4-Amidinophenylaminomethyl)-1-methyl-5-[1-[(pyridin-2-yl)-(carboxymethyloxyimino)methylen]cyclopropyl]-benzimidazol-hydrochlorid,

C = 2-(4-Amidinophenylaminomethyl)-1-methyl-5-[1-(2-carboxyethylamino)-1-(pyrrolidin-1-yl-carbonyl)-ethyl]-benzimidazol-hydrochlorid,

D = 2-(4-Amidinophenylaminomethyl)-1-methyl-5-[1-[2-(2-carboxyethyl)-pyrrolidin-1-yl-carbonyl]cyclopropyl]-benzimidazol-hydrochlorid,

E = 2-(4-Amidinophenylaminomethyl)-1-methyl-5-[2-(2-carboxyethyl)-4,5-dimethyl-imidazol-1-yl-methyl]-benzimidazol-hydrochlorid,

F = 2-(4-Amidinophenylaminomethyl)-1-methyl-5-[1-(carboxymethylamino)-1-(pyrrolidin-1-yl-carbonyl)-ethyl]-benzimidazol-hydrochlorid und

G = 2-(4-Amidinophenylaminomethyl)-1-methyl-5-[1-(N-methylcarboxymethylcarbonylaminomethyl)-1-methyl-1-(pyrrolidin-1-yl-carbonyl)-ethyl]-benzimidazol-dihydrochlorid

auf ihre Wirkung auf die aPTT-Zeit-Verlängerung wie folgt untersucht:

Material:-Plasma, aus humanem Citratblut,

- PTT-Reagenz, Boehringer Mannheim (524298),
- Calcium-Lösung (0.025 Mol/l), Behring Werke, Marburg (ORH 056/57),
- Diethylbarbituratacetat-Puffer, Behring Werke, Marburg (ORWH 60/61),
- Biomatic B10 Koagulometer, Desaga, Wiesloch.

Durchführung:

Die Bestimmung der aPTT-Zeit erfolgte mit einem Biomatic B10-Koagulometer der Firma Desaga.

Die Testsubstanz wurde in die vom Hersteller vorgeschriebenen Testgefäßen mit 0,1 ml humanem Citrat-Plasma und 0,1 ml PTT-Reagenz gegeben. Der Ansatz wurde für drei Minuten bei 37°C inkubiert. Durch Zugabe von 0.1 ml Calcium-Lösung wurde die Gerinnungsreaktion gestartet. Gerätebedingt erfolgt mit der Eingabe der Calcium-Lösung die Messung der Zeit bis zur Gerinnung des Ansatzes. Als Kontrolle dienten Ansätze bei denen 0,1 ml DBA-Puffer zugegeben wurden.

Gemäß der Definition wurde über eine Dosis-Wirkungskurve die effektive Substanzkonzentration ermittelt, bei der die aPTT-Zeit gegenüber der Kontrolle verdoppelt wurde.

Die nachfolgende Tabelle enthält die gefundenen Werte:

| Substanz | aPTT-Zeit ($ED_{200}$ in µM) |
|---|---|
| A | 0.12 |
| B | 0.42 |
| C | 0.31 |
| D | 0.29 |
| E | 0.29 |
| F | 0.20 |
| G | 0.17 |

Die erfindungsgemäß hergestellten Verbindungen sind gut verträglich, da bei therapeutischen Dosen keine toxischen Nebenwirkungen beobachtet werden konnten.

Aufgrund ihrer pharmakologischen Eigenschaften eignen sich die neuen Verbindungen und deren physiologisch verträglichen Salze zur Vorbeugung und Behandlung venöser und arterieller thrombotischer Erkrankungen, wie zum Beispiel der Behandlung von tiefen Beinvenen-Thrombosen, der Verhinderung von Reocclusionen nach Bypass-Operationen oder Angioplastie (PT(C)A), sowie der Occlusion bei peripheren arteriellen Erkrankungen wie Lungenembolie, der disseminierten intravaskulären Gerinnung, der Prophylaxe der Koronarthrombose, der Prophylaxe des Schlaganfalls und der Verhinderung der Occlusion von Shunts. Zusätzlich sind die erfindungsgemäßen Verbindungen zur antithrombotischen Unterstützung bei einer thrombolytischen Behandlung, wie zum Beispiel mit rt-PA oder Streptokinase, zur Verhinderung der Langzeitrestenose nach PT(C)A, zur Verhinderung der Metastasierung und des Wachstums von koagulationsabhängigen Tumoren und von fibrinabhängigen Entzündungsprozessen, z.B. bei der Behandlung der pulmonaren Fibrosis, geeignet.

Die zur Erzielung einer entsprechenden Wirkung erforderliche Dosierung beträgt zweckmäßigerweise bei intravenöser Gabe 0,1 bis 30 mg/kg, vorzugsweise 0,3 bis 10 mg/kg, und bei oraler Gabe 0,1 bis 50 mg/kg, vorzugsweise 0,3 bis 30 mg/kg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

Beispiel 1

2-(4-Amidinophenylaminomethyl)-1-methyl-5-[1-(pyrrolidin-1-ylcarbonyl)cyclopropyl]-benzimidazol-hydrochlorid

a. 1-(4-Chlor-3-nitro-phenyl)-1-cyclopropancarbonsäure

Zu 350 ml rauchender Salpetersäure werden bei -25°C portionsweise 50.0 g (0.21 Mol) 1-(4-Chlorphenyl)-1-cyclopropancarbonsäure gegeben. Die Lösung wird 15 Minuten bei -25°C gerührt und anschließend auf Eiswasser gegossen. Das ausgefallene Produkt wird abgesaugt, mit Wasser gewaschen und getrocknet.
Ausbeute: 58.5 g (95 % der Theorie),
$R_f$-Wert: 0.45 (Kieselgel; Methylenchlorid/Methanol = 9.5:0.5)

b. 1-(4-Methylamino-3-nitro-phenyl)-1-cyclopropancarbonsäure

20.0 g (0.083 Mol) 1-(4-Chlor-3-nitro-phenyl)-1-cyclopropancarbonsäure und 100 ml Methylaminlösung (40%ig in $H_2O$) werden in einem Druckgefäß fünf Stunden auf 80°C erhitzt. Der Inhalt wird zur Trockene eingedampft, in Wasser gelöst und mit Eisessig angesäuert. Das ausgefallene Produkt wird abgesaugt, mit Wasser gewaschen und getrocknet.
Ausbeute: 16.9 g (93 % der Theorie),
$R_f$-Wert: 0.58 (Kieselgel; Methylenchlorid/Methanol = 9:1)

c. 4-[1-(Pyrrolidin-1-yl-carbonyl)cyclopropyl]-2-nitro-N-methyl-anilin

2.4 g (0.01 Mol) 1-(4-Methylamino-3-nitro-phenyl)-1-cyclopropancarbonsäure werden in 50 ml Dimethylformamid gelöst und nach Zugabe von 3.2 g (0.01 Mol) O-(Benzotriazol-1-yl)-N,N,N',N'-tetramethyluroniumtetrafluoroborat, 0.7 g (0.01 Mol) Pyrrolidin und 1.1 g (0.01 Mol) N-Methyl-morpholin 20 Stunden bei Raumtemperatur gerührt. Das Lösungsmittel wird abdestilliert und der Rückstand an Kieselgel chromatographiert, wobei mit Methylenchlorid eluiert wird. Die gewünschten Fraktionen werden eingedampft, mit Ether verrieben, abgesaugt und getrocknet.
Ausbeute: 1.8 g (61 % der Theorie),
$R_f$-Wert: 0.51 (Kieselgel; Methylenchlorid/Methanol = 9:1)

d. 4-[1-(Pyrrolidin-1-yl-carbonyl)cyclopropyl]-2-amino-N-methyl-anilin

1.8 g (6.2 mMol) 4-[1-(Pyrrolidin-1-yl-carbonyl)cyclopropyl]-2-nitro-N-methyl-anilin werden in 40 ml Methanol und 40 ml Methylenchlorid gelöst und nach Zugabe von 0.4 g Palladium-auf Aktivkohle (10%) 4 Stunden bei Raumtemperatur hydriert. Anschließend wird vom Katalysator abfiltriert und eingedampft.
Ausbeute: 1.6 g (100 % der Theorie),
$R_f$-Wert: 0.26 (Kieselgel; Methylenchlorid/Methanol = 9:1)

e. 4-[1-(Pyrrolidin-1-yl-carbonyl)cyclopropyl]-2-(4-cyanophenyl)aminomethylcarbonylamino-N-methyl-anilin

Hergestellt analog Beispiel 1c aus 4-[1-(Pyrrolidin-1-yl-carbonyl)cyclopropyl]-2-amino-N-methyl-anilin, O-(Benzotriazol-1-yl)-N,N,N',N'-tetramethyluroniumtetrafluoroborat, 4-Cyanophenylglycin und Triethylamin in Dimethylformamid.
Ausbeute: 66 % der Theorie,
$R_f$-Wert: 0.51 (Kieselgel; Methylenchlorid/Methanol = 9:1)

f. 2-(4-Cyanophenylaminomethyl)-1-methyl-5-[1-(pyrrolidin-1-yl-carbonyl)cyclopropyl]-benzimidazol

1.7 g (0.004 Mol) 4-[1-(Pyrrolidin-1-yl-carbonyl)cyclopropyl]-2-(4-cyanophenyl)aminomethylcarbonylamino-N-methyl-anilin werden in 7 ml Eisessig 2 Stunden zum Rückfluß erhitzt. Das Solvens wird abdestilliert, der Rückstand in Wasser gelöst und mit Methylenchlorid extrahiert. Die organische Phase wird getrocknet, eingedampft und anschließend an Kieselgel chromatographiert, wobei mit Methylenchlorid + 2 bis 3 % Methanol eluiert wird.
Ausbeute: 1.0 g (62 % der Theorie),
$R_f$-Wert: 0.49 (Kieselgel; Methylenchlorid/Methanol = 9:1)

g. 2-(4-Amidinophenylaminomethyl)-1-methyl-5-[1-(pyrrolidin-1-yl-carbonyl)cyclopropyl]-benzimidazol-hydrochlorid

1.0 g (2.5 mMol) 2-(4-Cyanophenylaminomethyl)-1-methyl-5-[1-(pyrrolidin-1-yl-carbonyl)cyclopropyl]-benzimidazol werden in 50 ml gesättigter ethanolischer Salzsäure gelöst und 5 Stunden bei Raumtemperatur gerührt. Das Solvens wird abdestilliert, der Rückstand in 50 ml absolutem Ethanol gelöst und mit 2.3 g (25 mMol) Ammoniumcarbonat versetzt. Nach 60 Stunden bei Raumtemperatur wird zur Trockene eingedampft. Der Rückstand wird an Kieselgel chromatographiert, wobei mit Methylenchlorid/Methanol (7:1) eluiert wird.
Ausbeute: 700 mg (62 % der Theorie),
$R_f$-Wert: 0.61 (Kieselgel; Methylenchlorid/Methanol = 4:1) $C_{24}H_{28}N_6O$ x HCl (416.54/453.0)
Massenspektrum: $(M+H)^+$ = 417

Analog Beispiel 1 werden folgende Verbindungen erhalten:

(1) 2-(4-Amidinophenylaminomethyl)-1-methyl-5-[1-(3-methyl-piperidin-1-yl-carbonyl)cyclopropyl]-benzimidazol-hydrochlorid
Ausbeute: 45 % der Theorie,
$R_f$-Wert: 0.25 (Kieselgel; Methylenchlorid/Methanol = 4:1) $C_{26}H_{32}N_6O$ x HCl (444.59/481.05)
Massenspektrum: $(M+H)^+$ = 445

(2) 2-(4-Amidinophenylaminomethyl)-1-methyl-5-[1-(piperidin-1-yl-carbonyl)cyclopropyl]-benzimidazol-hydrochlorid
Ausbeute: 57 % der Theorie,
$R_f$-Wert: 0.23 (Kieselgel; Methylenchlorid/Methanol = 4:1) $C_{25}H_{30}N_6O$ x HCl (430.56/467.93)
Massenspektrum: $(M+H)^+$ = 431

(3) 2-(4-Amidinophenylaminomethyl)-1-methyl-5-[1-(4-methyl-piperazin-1-yl-carbonyl)cyclopropyl)-benzimidazol-hydrochlorid
Ausbeute: 32 % der Theorie,
$R_f$-Wert: 0.26 (Kieselgel; Methylenchlorid/Methanol/Ammoniak = 2:1:0.25)
$C_{25}H_{31}N_7O$ x HCl (445.58/482.04)
Massenspektrum: $(M+H)^+$ = 446

(4) 2-(4-Amidinophenylaminomethyl)-1-methyl-5-[1-(2,3-dihydroindolin-1-yl-carbonyl)cyclopropyl]-benzimidazol-hydrochlorid
Ausbeute: 60 % der Theorie,
$R_f$-Wert: 0.34 (Kieselgel; Methylenchlorid/Methanol = 4:1) $C_{28}H_{28}N_6O$ x HCl (464.58/501.04)
Massenspektrum: $(M+H)^+$ = 465

(5) 2-(4-Amidinophenylaminomethyl)-1-methyl-5-[1-((2-ethoxycarbonylethyl)-piperidin-1-yl-carbonyl)cyclopropyl]-benzimidazol-hydrochlorid
Ausbeute: 85 % der Theorie,
$R_f$-Wert: 0.57(Kieselgel; Methylenchlorid/Methanol = 4:1) $C_{30}H_{38}N_6O_3$ x HCl (530.67/567.13)
Massenspektrum: $(M+H)^+$ = 531

(6) 2-(4-Amidinophenylaminomethyl)-1-methyl-5-[1-((2-ethoxycarbonylethyl)-pyrrolidin-1-yl-carbonyl)cyclopropyl]-benzimidazol-hydrochlorid
Ausbeute: 60 % der Theorie,
$C_{29}H_{36}N_6O_3$ x HCl (516.64/553.10)
Massenspektrum: $(M+H)^+$ = 517
$(M+2H)^{++}$ = 259

(7) 2-(4-Amidinophenylaminomethyl)-1-methyl-5-[1-[(N-(2-ethoxycarbonylethyl)-N-methyl-aminomethyl)-pyrrolidin-1-yl-carbonyl]cyclopropyl]-benzimidazol-hydrochlorid
Ausbeute: 65 % der Theorie,
$C_{31}H_{41}N_7O_3$ x HCl (559.72/ 596.18)
Massenspektrum: $(M+H)^+ = 560$
$(M+2H)^{++} = 280.6$

(8) 2-(4-Amidinophenylaminomethyl)-1-methyl-5-[1-[(2-ethoxycarbonylmethyloxymethyl)-pyrrolidin-1-yl-carbonyl] cyclopropyl]-benzimidazol-hydrochlorid
Ausbeute:. 61 % der Theorie,
$R_f$-Wert: 0.20 (Kieselgel; Methylenchlorid/Ethanol = 8:2 + 1 % Eisessig)
$C_{29}H_{36}N_6O_4$ x HCl (532.66/569.11)
Massenspektrum: $(M+H)^+ = 533$
$(M+2H)^{++} = 267$

Beispiel 2

2-(4-Amidinophenylaminomethyl)-1-methyl-5-(1-cyclopentylcarbonyl-3-chlor-n-propyl)-benzimidazol-hydrochlorid

a. 4-[1-(Cyclopentylcarbonyl)cyclopropyl]-chlorbenzol

2.4 g (0.1 Mol) Magnesiumspäne werden in 10 ml Ether suspendiert. Nach Zugabe von einer Spatelspitze Jod werden 14.9 g (0.1 Mol) Bromcyclopentan in 40 ml Ether langsam zugetropft, wobei am Anfang durch leichtes Erwärmen die Reaktion gestartet wird. Nach beendeter Zugabe wird noch 30 Minuten unter Rückfluß erhitzt. Anschließend wird eine Lösung von 14.0 g (0.08 Mol) 1-(4-Chlorphenyl)-1-cyclopropancarbonitril in 75 ml Ether zugeben und weitere 3 Stunden unter Rückfluß erhitzt. Die Reaktionslösung wird auf Eiswasser gegossen, mit Salzsäure auf pH 3 gestellt und mit Ether extrahiert. Die organischen Extrakte werden getrocknet und eingedampft. Der Rückstand wird an Kieselgel chromatographiert und mit Petrolether/Essigester (19:1 und 15:1) eluiert.
Ausbeute: 3.0 g (12 % der Theorie),
$R_f$-Wert: 0.58 (Kieselgel; Petrolether/Essigester = 4:1)

b. 4-[1-(Cyclopentylcarbonyl)cyclopropyl]-2-nitro-chlorbenzol

Hergestellt analog Beispiel 1a aus 4-[1-(Cyclopentylcarbonyl)cyclopropyl]-chlorbenzol und rauchender Salpetersäure.
Ausbeute: 87 % der Theorie,
$R_f$-Wert: 0.60 (Kieselgel; Petrolether/Essigester = 9:1)

c. 4-[1-(Cyclopentylcarbonyl)cyclopropyl]-2-nitro-N-methylanilin

Hergestellt analog Beispiel 1b aus 4-[1-(Cyclopentylcarbonyl)cyclopropyl]-2-nitro-chlorbenzol und wäßriger Methylaminlösung.
Ausbeute: 18 % der Theorie,
$R_f$-Wert: 0.54 (Kieselgel; Methylenchlorid/Ethanol = 19:1)

d. 4- [1-(Cyclopentylcarbonyl)cyclopropyl]-2-amino-N-methylanilin

2.3 g (7.9 mMol) 4-[1-(Cyclopentylcarbonyl)cyclopropyl]-2-nitro-N-methyl-anilin werden in 125 ml Essigester und 25 ml Ethanol gelöst und nach Zugabe von 1.0 g Raney-Nickel 1.5 Stunden bei Raumtemperatur hydriert. Anschließend wird vom-Katalysator abfiltriert und eingedampft.
Ausbeute: 2.0 g (98 % der Theorie),
$R_f$-Wert: 0.15 (Kieselgel; Methylenchlorid/Ethanol = 19:1)

e. 4-[1-(Cyclopentylcarbonyl)cyclopropyl]-2-(4-cyanophenylaminomethylcarbonylamino)-N-methyl-anilin

Hergestellt analog Beispiel 1c aus 4-[1-(Cyclopentylcarbonyl)cyclopropyl]-2-amino-N-methyl-anilin, O-(Benzotriazol-1-yl)-N,N,N',N'-tetramethyluroniumtetrafluoroborat, 4-Cyano-phenylglycin und Triethylamin in Dimethylformamid.

Ausbeute: 96 % der Theorie,
$R_f$-Wert: 0.54 (Kieselgel; Methylenchlorid/Ethanol = 19:1)

f. 2-(4-Cyanophenylaminomethyl)-1-methyl-5-[1-(cyclopentylcarbonyl)-cyclopropyl]-benzimidazol

Hergestellt analog Beispiel 1f aus 4-[1-(Cyclopentylcarbonyl)cyclopropyl]-2-(4-cyanophenyl-aminomethylcarbonylamino)-N-methyl-anilin in Eisessig.
Ausbeute: 53 % der Theorie,
$R_f$-Wert: 0.46 (Kieselgel; Methylenchlorid/Ethanol = 19:1)

g. 2-(4-Amidinophenylaminomethyl)-1-methyl-5-(1-cyclopentylcarbonyl-3-chlor-n-propyl)-benzimidazol-hydrochlorid

Hergestellt analog Beispiel 1g aus 2-(4-Cyanophenylaminomethyl)-1-methyl-5-(1-cyclopentylcarbonyl-3-chlor-n-propyl)-benzimidazol und Salzsäure/Ammoniumcarbonat in Ethanol.
Ausbeute: 61 % der Theorie,
$C_{25}H_{30}ClN_5O$ x HCl (452.00/488.56)
Massenspektrum: $(M+H)^+$ = 452/4 (Cl))

Beispiel 3

(E/Z)-2-(4-Amidinophenylaminomethyl)-1-methyl-5-[1-[(pyridin-3-yl)-(ethoxycarbonylmethyloxyimino)methylen]cyclopropyl]-benzimidazol-hydrochlorid

a. 1-[(Pyridin-3-yl)-carbonyl]cyclopropyl-benzol

Zu 100 ml Butyllithium (1.6 M in Hexan) wird bei -40 bis -50°C eine Lösung von 21.4 g (0.135 Mol) 3-Brompyridin in 125 ml Ether zugetropft und danach noch 20 Minuten bei -40°C gerührt. Anschließend wird auf -60°C abgekühlt und eine Lösung von 20.1 g (0.14 Mol) 1-Phenyl-cyclopropan-carbonitril in 125 ml Ether zugetropft. Nach beendeter Zugabe wird das Reaktionsgemisch auf Raumtemperatur erwärmt und 5 Stunden gerührt. Die Suspension wird mit 20%iger Salzsäure versetzt und 30 Minuten auf 100°C erhitzt. Nach Abkühlung wird mit 20%iger Natronlauge auf pH 8 gestellt und mit Essigester extrahiert. Die vereinigten organischen Extrakte werden getrocknet und eingedampft. Der Rückstand wird an Aluminiumoxid chromatographiert, wobei mit Petrolether/Essigester (9:1) eluiert wird.
Ausbeute: 14.0 g (46 % der Theorie),
$R_f$-Wert: 0.27 (Aluminiumoxid; Petrolether/Essigester = 9:1)

b. 4-[1-[(Pyridin-3-yl)-carbonyl]cyclopropyl]-nitrobenzol

Hergestellt analog Beispiel 1a aus 1-[(Pyridin-3-yl)-carbonyl]cyclopropyl-benzol und rauchender Salpetersäure.
Ausbeute: 53.7 % der Theorie,
$R_f$-Wert: 0.29 (Aluminiumoxid; Petrolether/Essigester = 4:1)

c. 4-[1-[(Pyridin-3-yl)-carbonyl]cyclopropyl]-anilin

Hergestellt analog Beispiel 2d aus 4-[1-[(Pyridin-3-yl)-carbonyl]cyclopropyl]-nitrobenzol und Raney-Nickel in Essigester/Ethanol.
Ausbeute: 94 % der Theorie,
$R_f$-Wert: 0.51 (Kieselgel; Methylenchlorid/Ethanol = 19:1)

d. 4-[1-[(Pyridin-3-yl)-carbonyl]cyclopropyl]-trifluoracetylanilin

8.0 g (33.5 mMol) 4-[1-[(Pyridin-3-yl)-carbonyl]cyclopropyl]-anilin werden in 100 ml Chlorbenzol gelöst und nach Zugabe von 15 ml Trifluoressigsäureanhydrid zwei Stunden bei 110°C gerührt. Das Solvens wird abdestilliert, der Rückstand mit Petrolether/Ether (9:1) verrührt, abgesaugt und getrocknet.
Ausbeute: 10.0 g (88 % der Theorie),
$R_f$-Wert: 0.54 (Kieselgel; Methylenchlorid/Ethanol = 19:1)

e. 4-[1-[(Pyridin-3-yl)-carbonyl]cyclopropyl]-2-nitro-trifluoracetylanilin

13 ml konz. Schwefelsäure und 16 ml 65%ige Salpetersäure werden bei -5°C portionsweise mit 1.7 g (5 mMol) 4-[1-[(Pyridin-3-yl)-carbonyl]cyclopropyl]-trifluoracetylanilin versetzt. Anschließend wird noch 30 Minuten ohne Kühlung gerührt, auf Eiswasser gegossen und mit Essigester extrahiert. Die organischen Extrakte werden getrocknet und eingedampft. Der Rückstand wird an Kieselgel chromatographiert und mit Methylenchlorid/Ethanol (50:1 und 25:1) eluiert. Die gewünschten Fraktionen werden eingedampft, mit Ether/Petrolether verrieben, abgesaugt und getrocknet.
Ausbeute: 1.2 g (75 % der Theorie),
$R_f$-Wert: 0.70 (Kieselgel; Methylenchlorid/Ethanol = 19:1)

f. 4-[1-[(Pyridin-3-yl)-carbonyl]cyclopropyl]-2-nitro-N-trifluoracetyl-N-methyl-anilin

1.15 g (3.0 mMol) 4-[1-[(Pyridin-3-yl)-carbonyl]cyclopropyl]-2-nitro-trifluoracetylanilin werden in 50 ml Aceton gelöst und nach Zugabe von 2.0 g Kaliumcarbonat und 0.8 ml Methyliodid zwei Stunden unter Rückfluß erhitzt. Das unlösliche Material wird abfiltriert und die Lösung eingeengt. Der Rückstand wird an Kieselgel chromatographiert, wobei mit Petrolether/Essigester (1:1 und 1:4) eluiert wird.
Ausbeute: 0.88 g (75 % der Theorie),
$R_f$-Wert: 0.38 (Kieselgel; Petrolether/Essigester = 1:1)

g. 4-[1-[(Pyridin-3-yl)-carbonyl]cyclopropyl]-2-nitro-N-methyl-anilin

7.4 g (18.8 mMol) 4-[1-[(Pyridin-3-yl)-carbonyl]cyclopropyl]-2-nitro-N-trifluoracetyl-N-methyl-anilin werden in 200 ml 20%ige Kalilauge eine Stunde bei 30°C gerührt. Anschließend wird mit Isopropanol verdünnt, die organische Phase abgetrennt, mit 10.0 g Aluminiumoxid versetzt und zur Trockene eingedampft. Der Rückstand wird an Aluminiumoxid chromatographiert, wobei mit Petrolether/Essigester (4:1 und 1:1) eluiert wird.
Ausbeute: 2.6 g (47 % der Theorie),
$R_f$-Wert: 0.50 (Kieselgel; Petrolether/Essigester = 1:1)

h. 4-[1-[(Pyridin-3-yl)-carbonyl]cyclopropyl]-2-amino-N-methyl-anilin

Hergestellt analog Beispiel 2d aus 4-[1-[(Pyridin-3-yl)-carbonyl]cyclopropyl]-2-nitro-N-methyl-anilin und Raney-Nickel in Essigester/Ethanol.
Ausbeute: 98 % der Theorie,
$R_f$-Wert: 0.51 (Kieselgel; Methylenchlorid/Ethanol = 19:1)

i. 4-[1-[(Pyridin-3-yl)-carbonyl]cyclopropyl]-2-(4-cyanophenyl)-aminomethylcarbonylamino-N-methyl-anilin

Hergestellt analog Beispiel 1c aus 4-[1-[(Pyridin-3-yl)-carbonyl]cyclopropyl]-2-amino-N-methyl-anilin, O-(Benzotriazol-1-yl)-N,N,N', N'-tetramethyluroniumtetrafluoroborat, 4-Cyanophenylglycin und Triethylamin in Dimethylformamid.
Ausbeute: 97 % der Theorie,
$R_f$-Wert: 0.48 (Kieselgel; Methylenchlorid/Ethanol = 19:1)

k. 2-(4-Cyanophenylaminomethyl)-1-methyl-5-[1-[(pyridin-3-yl)-carbonyl]cyclopropyl]-benzimidazol

Hergestellt analog Beispiel 1f aus 4-(Pyridin-3-yl-carbonyl)-cyclopropyl-2-(4-cyanophenyl)-aminomethylcarbonylamino-N-methyl-anilin in Eisessig.
Ausbeute: 76 % der Theorie,
$R_f$-Wert: 0.52 (Kieselgel; Methylenchlorid/Ethanol = 19:1)

l. (E/Z)-2-(4-Cyanophenylaminomethyl)-1-methyl-5-[1-[(pyridin-3-yl)-(carboxymethyloxyimino)methylen]cyclopropyl] benzimidazol

1.6 g (4.0 mMol) 2-(4-Cyanophenylaminomethyl)-1-methyl-5-[1-[(pyridin-3-yl)-carbonyl]cyclopropyl]-benzimidazol, 3.0 g (12 mMol) Carboxy-methoxylamin-hemihydrat, 0.84 ml Triethylamin, 12 g Molekularsieb 3A und 12 g Molekularsieb 4A werden in 80 ml Methanol und 40 ml Toluol 12 Stunden unter Rückfluß erhitzt. Anschließend wird vom Molekularsieb abfiltriert und eingedampft. Der Rückstand wird mit Wasser verrührt, abgesaugt und getrocknet.

Das Rohprodukt wird an Kieselgel chromatographiert, wobei mit Methylenchlorid/Ethanol/Eisessig (25:1:0 und 8:2:0.2) eluiert wird.
Ausbeute: 0.9 g (48 % der Theorie),
$R_f$-Wert: 0.34 (Kieselgel; Methylenchlorid/Ethanol/Eisessig = 8:2:0.2)

m. (E/Z)-2-(4-Amidinophenylaminomethyl)-1-methyl-5-[1-[(pyridin-3-yl)-(ethoxycarbonylmethyloxyimino)methylen]cyclopropyl]-benzimidazol-hydrochlorid

Hergestellt analog Beispiel 1g aus (E/Z)-2-(4-Cyanophenylaminomethyl)-1-methyl-5-[1-[(pyridin-3-yl)-(ethoxycarbonylmethyloxyimino)methylen]cyclopropyl]-benzimidazol und Salzsäure/Ammoniumcarbonat in Ethanol.
Ausbeute: 60 % der Theorie,
$R_f$-Wert: 0.28 (Kieselgel; Methylenchlorid/Ethanol/Eisessig = 8:2:0.2)
$C_{29}H_{32}N_7O_3$ x HCl (525.62/562.09)
Massenspektrum: $(M+H)^+$ = 526

Analog Beispiel 3 werden folgende Verbindungen erhalten:

(1) (E/Z)-2-(4-Amidinophenylaminomethyl)-1-methyl-5-[1-[(pyridin-2-yl)-(ethoxycarbonylmethyloxyimino)methylen]cyclopropyl]-benzimidazol-hydrochlorid
Ausbeute: 52 % der Theorie,
$R_f$-Wert: 0.21 (Kieselgel; Methylenchlorid/Ethanol/Eisessig = 8:2:0.2)
$C_{29}H_{31}N_7O_3$ x HCl (525.62/562.09)
Massenspektrum: $(M+H)^+$ = 526

(2) (E/Z)-2-(4-Amidinophenylaminomethyl)-1-methyl-5-[1-[phenyl-(ethoxycarbonylmethyloxyimino)methylen]cyclopropyl]-benzimidazol-hydrochlorid
Ausbeute: 18 % der Theorie,
$C_{30}H_{32}N_6O_3$ x HCl (524.63/561.09)
Massenspektrum: $(M+H)^+$ = 525
$(M-H+HCl)^-$ = 559/61 (Cl)

(3) 2-(4-Amidinophenylaminomethyl)-1-methyl-5-[1-(1-methylpyrazol-5-yl-carbonyl)cyclopropyl]-benzimidazol-hydrochlorid
Ausbeute: 10 % der Theorie,
$C_{24}H_{25}N_7O$ x HCl (427.51/ 463.97)
Massenspektrum: $(M+H)^+$ = 428
$(M+H+HCl)^-$ = 464/6 (Cl)

(4) (E/Z)-2-(4-Amidinophenylaminomethyl)-1-methyl-5-[1-[(1-methyl-pyrazol-5-yl-(ethoxycarbonylmethyloxyimino)methylen]cyclopropyl]-benzimidazol-hydrochlorid
Ausbeute: 80 % der Theorie,
$R_f$-Wert: 0.20 (Kieselgel; Methylenchlorid/Ethanol = 8:2 + 1 % Eisessig)
$C_{28}H_{32}N_8O_3$ x HCl (528.63/565.08)
Massenspektrum: $(M+H)^+$ = 529

(5) (E/Z)-2-(4-Amidinophenylaminomethyl)-1-methyl-5-[1-[phenyl-(3-ethoxycarbonyl-n-propyloxyimino)methylen] cyclopropyl]-benzimidazol-dihydrochlorid
Ausbeute: 47 % der Theorie,
$R_f$-Wert: 0.06 (Kieselgel; Methylenchlorid/Methanol = 9:1)
$C_{32}H_{36}N_6O_3$ x 2 HCl (552.69/625.60)
Massenspektrum: $(M+H)^+$ = 553

Beispiel 4

(E/Z)-2-(4-Amidinophenylaminomethyl)-1-methyl-5-[1-[(pyridin-2-yl)-(carboxymethyloxyimino)methylen]cyclopropyl]-benzimidazol-hydrochlorid

150 mg (E/Z)-2-(4-Amidinophenylaminomethyl)-1-methyl-5-[1-[(pyridin-2-yl-(ethoxycarbonylmethyloxyimino) methylen]cyclopropyl]-benzimidazol-hydrochlorid und 2.5 ml 2N Natronlauge werden in 10 ml Ethanol 5 Stunden bei

Raumtemperatur gerührt. Der Alkohol wird abdestilliert und der Rückstand mit Salzsäure auf pH 5 eingestellt. Das kristalline Produkt wird abgesaugt, mit Wasser gewaschen und getrocknet.
Ausbeute: 46 % der Theorie,
$R_f$-Wert: 0.10 (Kieselgel; Methylenchlorid/Ethanol/Eisessig = 8:2:0.1)
$C_{27}H_{27}N_7O_3$ x HCl (497.58/534.05)
Massenspektrum: $(M+H)^+ = 498$
$(M+Na)^+ = 520$

Analog Beispiel 4 werden folgende Verbindungen erhalten:

(1) 2-(4-Amidinophenylaminomethyl)-1-methyl-5-[1-[2-(2-carboxyethyl)-piperidin-1-yl-carbonyl]cyclopropyl]-benzimidazolhydrochlorid
Ausbeute: 94 % der Theorie,
$R_f$-Wert: 0.57 (Reversed Phase RP 18; Methanol/5% Natriumchloridlösung = 3:2)
$C_{28}H_{34}N_6O_3$ x HCl (502.62/539.08)
Massenspektrum: $(M+H)^+ = 503$
$(M+Na)^+ = 525$

(2) 2-(4-Amidinophenylaminomethyl)-1-methyl-5-[1-(2-carboxyethylamino)-1-(pyrrolidin-1-yl-carbonyl)-ethyl]-benzimidazolhydrochlorid
Ausbeute: 98 % der Theorie,
$R_f$-Wert: 0.73 (Reversed Phase RP 8; Methanol/5% Natriumchloridlösung = 1:2)
$C_{26}H_{33}N_7O_3$ x HCl (491.60/564.54)
Massenspektrum: $(M+H)^+ = 492$
$(M+2H)^{++} = 247$

(3) 2-(4-Amidinophenylaminomethyl)-1-methyl-5-[1-(3-carboxypropionylamino)-1-(ethoxycarbonyl)-ethyl]-benzimidazol-hydrochlorid
Ausbeute: 98 % der Theorie,
$R_f$-Wert: 0.70 (RP 8; Methanol/5% Natriumchloridlösung = 1 :2)
$C_{25}H_{30}N_6O_5$ x HCl (494.55/531.05)
Massenspektrum: $(M+H)^+ = 495$
$(2M+H)^+ = 989$

(4) 2-(4-Amidinophenylaminomethyl)-1-methyl-5-[1-(carboxymethylamino)-1-(pyrrolidin-1-yl-carbonyl)-methyl]-benzimidazolhydrochlorid
Ausbeute: 87 % der Theorie,
$C_{24}H_{29}N_7O_3$ x HCl (463.54/500.04)
Massenspektrum: $(M+H)^+ = 464$
$(M+2H)^+ = 232.6$

(5) 2-(4-Amidinophenylaminomethyl)-1-methyl-5-[1-[2-(2-carboxyethyl)-pyrrolidin-1-yl-carbonyl]cyclopropyl]-benzimidazolhydrochlorid
Ausbeute: 94 % der Theorie,
$C_{27}H_{32}N_6O_3$ x HCl (488.59/525.05)
Massenspektrum: $(M+H)^+ = 489$
$(M+Na)^+ = 511$

(6) 2-(4-Amidinophenylaminomethyl)-1-methyl-5-[1-(carboxymethylcarbonylamino)-1-(pyrrolidin-1-yl-carbonyl)-methyl]-benzimidazol-hydrochlorid
Ausbeute: 49 % der Theorie,
$C_{25}H_{29}N_7O_4$ x HCl (491.55/528.01)
Massenspektrum: $(M+H)^+ = 492$
$(M+H+Na)^{++} = 257.7$

(7) 2-(4-Amidinophenylaminomethyl)-1-methyl-5-[1-(carboxymethylcarbonylamino)-1-(pyrrolidin-1-yl-carbonyl)-ethyl]-benzimidazol-hydrochlorid
Ausbeute: 92 % der Theorie,
$C_{26}H_{31}N_7O_4$ x HCl (505.58/542.04)

Massenspektrum: $(M+H)^+ = 506$
$(M+H+Na)^{++} = 264.7$

(8) 2-(4-Amidinophenylaminomethyl)-1-methyl-5-(5-methyl-3-carboxymethyl-imidazolin-2,4-dion-5-yl)-benzimidazol-hydrochlorid
Ausbeute: 88 % der Theorie,
$C_{22}H_{23}N_7O_4$ x HCl (449.47/485.94)
Massenspektrum: $(M+H)^+ = 450$
$(M+2Na)^{++} = 247.7$

(9) (E/Z)-2-(4-Amidinophenylaminomethyl)-1-methyl-5-[1-[(pyridin-3-yl)-(carboxymethyloxyimino)methylen]cyclopropyl]-benzimidazol-hydrochlorid
Ausbeute: 54 % der Theorie,
$C_{27}H_{27}N_7O_3$ x HCl (497.56/534.09)
Massenspektrum: $(M+H)^+ = 498$
$(M+Na)^+ = 520$

(10) 2-(4-Amidinophenylaminomethyl)-1-methyl-5-[1-[2-(N-(2-carboxyethyl)-N-methyl-aminomethyl)-pyrrolidin-1-yl-carbonyl]cyclopropyl]-benzimidazol-hydrochlorid
Ausbeute: 100 % der Theorie,
$C_{29}H_{37}N_7O_3$ x HCl (531.66/568.12)
Massenspektrum: $(M+H)^+ = 532$
$(M-H)^- = 530$

(11) 2-(4-Amidinophenylaminomethyl)-1-methyl-5-[N-(2-carboxyethyl)-N-(2-pyridyl)-aminomethyl]-benzimidazol-hydrochlorid
Ausbeute: 91 % der Theorie,
$C_{25}H_{27}N_7O_2$ x HCl (457.54/493.96)
Massenspektrum: $(M+H)^+ = 458$

(12) 2-(4-Amidinophenylaminomethyl)-1-methyl-5-(N-benzolsulfonyl-N-carboxymethyl-aminomethyl)-benzimidazol-hydrochlorid
Ausbeute: 84 % der Theorie,
$C_{25}H_{26}N_6O_4S$ x HCl (506.59/543.06)
Massenspektrum: $(M+H)^+ = 507$

(13) 2-(4-Amidinophenylaminomethyl)-1-methyl-5-[2-(2-carboxyethyl)-benzimidazol-1-yl-methyl]-benzimidazol-hydrochlorid
Ausbeute: 76% der Theorie,
$C_{27}H_{27}N_7O_2$ x HCl (481.56/518.05)
Massenspektrum: $(M+H)^+ = 482$
$(M+2H)^{2+} = 242$
$(M+Na)^+ = 504$
$(M+H+Na)^{2+} = 253$
$(M-H+2Na)^+ = 526$
$(M+2Na)^{2+} = 264$

(14) 2-(4-Amidinophenylaminomethyl)-1-methyl-5-(2-methyl-4-carboxy-imidazol-1-yl-methyl)-benzimidazol-hydrochlorid
Ausbeute: 61% der Theorie,
$C_{22}H_{23}N_7O_2$ x HCl (417.47/453.92)
Massenspektrum: $(M+H)^+ = 418$

(15) 2-(4-Amidinophenylaminomethyl)-1-methyl-5-[3-(3-carboxyn-propyl)-benzimidazol-2-on-1-yl-methyl]-benzimidazol-hydrochlorid
Ausbeute: 86% der Theorie,
$C_{28}H_{29}N_7O_3$ x HCl (511.59/548.04)
Massenspektrum: $(M+H)^+ = 512$

$(M+Na)^+$ = 534
$(M+H+Na)^{2+}$ = 267.7
$(M+2Na)^{2+}$ = 278.8

(16) 2-(4-Amidinophenylaminomethyl)-1-methyl-5-[3-(2-carboxyethyl)-imidazo[4,5-b]pyridin-2-on-1-yl-methyl]-benzimidazolhydrochlorid
Ausbeute: 83% der Theorie,
$C_{26}H_{26}N_8O_3$ x HCl (498.55/535)
Massenspektrum: $(M+H)^+$ = 499
$(M+Na)^+$ = 521
$(M-H)^-$ = 497
$(2M-H)^-$ = 995

(17) 2-(4-Amidinophenylaminomethyl)-1-methyl-5-[2-(2-carboxyethyl)-4,5-dimethyl-imidazol-1-yl-methyl]-benzimidazol-hydrochlorid
Ausbeute: 68% der Theorie,
$R_f$-Wert: 0.70 (Reversed Phase RP 8; Methanol/5% Natriumchloridlösung = 6:4)
$C_{25}H_{29}N_7O_2$ x HCl (459.56/496.01)
Massenspektrum: $(M+H)^+$ = 460
$(M+Na)^+$ = 482
$(M+H+Na)^{2+}$ = 241

(18) (E/Z)-2-(4-Amidinophenylaminomethyl)-1-methyl-5-[1-[phenyl-(carboxymethyloxyimino)methylen]cyclopropyl]-benzimidazoldihydrochlorid
Ausbeute: 70% der Theorie,
$C_{28}H_{28}N_6O_3$ x 2HCl (496.57/569.5)
Massenspektrum: $(M+H)^+$ = 497
$(M-H)^-$ = 495

(19) (E/Z)-2-(4-Amidinophenylaminomethyl)-1-methyl-5-[1-[(pyridin-3-yl)-(carboxymethyliden)-methylen]cyclopropyl]-benzimidazol-hydrochlorid
Ausbeute: 37% der Theorie
$R_f$-Wert: 0.45 (Reversed Phase RP 8; Methanol/5% Natriumchloridlösung = 6:4)
$C_{27}H_{26}N_6O_2$ x HCl (466.55/503.0)
Massenspektrum: $(M+H)^+$ = 467

(20) (E/Z)-2-(4-Amidinophenylaminomethyl)-1-methyl-5-[1-[(1-methyl-pyrazol-5-yl)-(carboxymethyloxyimino)methylen]-cyclopropyl]-benzimidazol-hydrochlorid
Ausbeute: 30% der Theorie,
$R_f$-Wert: 0.25 (Reversed Phase RP 8; (5%ige Kochsalzlösung/Methanol = 1:1)
$C_{26}H_{28}N_8O_3$ x HCl (500.58/537.03)
Massenspektrum: $(M+H)^+$ = 501
$(M-H)^-$ = 499
$(M+Cl)^+$ = 535/537 (Cl)

(21) (E/Z)-2-(4-Amidinophenylaminomethyl)-1-methyl-5-[1-[phenyl-(3-carboxy-n-propyloxyimino)methylen]cyclopropyl]-benzimidazol-dihydrochlorid
Ausbeute: 37 % der Theorie,
$R_f$-Wert: 0.35 (Reversed Phase RP 8; 5%ige Kochsalzlösung/Methanol = 3:2)
$C_{30}H_{32}N_6O_3$ x 2 HCl (524.64/597.55)
Massenspektrum: $(M+H)^+$ = 525

Beispiel 5

2-(4-Amidinophenylaminomethyl)-1-methyl-5-[1-(pyrrolidin-1-yl-carbonyl)-aminomethyl]-benzimidazol-hydrochlorid

a. 5-(4-Chlorphenyl)-imidazolidin-2,4-dion

15.0 g (0.11 Mol) 4-Chlorbenzaldehyd, 51.3 g (0.53 Mol) Ammoniumcarbonat und 7.6 g (0.12 Mol) Kaliumcyanat werden in 150 ml Wasser und 150 ml Methanol 18 Stunden bei 55°C gerührt. Das Solvens wird abdestilliert, der Rückstand in Wasser gelöst und mit Essigester extrahiert. Die vereinigten organischen Extrakte werden getrocknet und eingedampft.
Ausbeute: 8.6 g (38 % der Theorie),
Schmelzpunkt: 215°C

b. 5-(4-Chlor-3-nitro-phenyl)-imidazolidin-2,4-dion

Hergestellt analog Beispiel 1a aus 5-(4-Chlorphenyl)-imidazolidin-2,4-dion und rauchender Salpetersäure.
Ausbeute: 52 % der Theorie,
$R_f$-Wert: 0.63 (Kieselgel; Methylenchlorid/Methanol = 9:1)

c. 4-Chlor-3-nitro-phenylalanin-hydrochlorid

560 mg (2.2 mMol) 5-(4-Chlor-3-nitro-phenyl)-imidazolidin-2,4-dion werden in 20 ml halbkonz. Salzsäure 24 Stunden zum Rückfluß erhitzt. Das Solvens wird abdestilliert, der Rückstand in Wasser gelöst, vom Unlöslichen abfiltriert und eingedampft. Der Rückstand wird dreimal in Ethanol gelöst, zur Trockene eingedampft, mit Ether verrieben, abgesaugt und getrocknet.
Ausbeute: 380 mg (65 % der Theorie),
Schmelzpunkt: 186°C

d. 4-Chlor-3-nitro-N-tert.butyloxycarbonyl-phenylalanin

5.7 g (17.8 mMol) 4-Chlor-3-nitro-phenylalanin-hydrochlorid werden in 50 ml Dioxan und 25 ml Wasser gelöst und nach Zugabe von 5.5 ml (39.1 mMol) Triethylamin und 4.8 g (21.3 mMol) Ditert.butyldicarbonat 18 Stunden bei Raumtemperatur gerührt. Anschließend wird mit 0.5 M Kaliumhydrogensulfatlösung verdünnt und mit Essigester extrahiert. Die vereinigten organischen Extrakte werden getrocknet und eingedampft.
Ausbeute: 6.3 g (100 % der Theorie),
$R_f$-Wert: 0.20 (Kieselgel; Methylenchlorid/Methanol = 9:1)

e. 2-(4-Chlor-3-nitro-phenyl)-2-tert.butyloxycarbonylamino-1-(pyrrolidin-1-yl)-ethanon

Hergestellt analog Beispiel 1c aus 4-Chlor-3-nitro-N-tert.butyloxycarbonyl-phenylalanin, O-(Benzotriazol-1-yl)-N,N,N',N'-tetramethyluroniumtetrafluoroborat, Pyrrolidin und N-Ethyl-diisopropylamin in Tetrahydrofuran.
Ausbeute: 68 % der Theorie,
Schmelzpunkt: 203°C

f. 2-(4-Methylamino-3-nitro-phenyl)-2-tert.butyloxycarbonylamino-1-(pyrrolidin-1-yl)-ethanon

Hergestellt analog Beispiel 1b aus 2-(4-Chlor-3-nitro-phenyl)-2-tert.butyloxycarbonylamino-1-(pyrrolidin-1-yl)-ethanon und Methylaminlösung.
Ausbeute: 76 % der Theorie,
$R_f$-Wert: 0.33 (Kieselgel; Cyclohexan/Essigester = 1:1)

g. 2-(4-Methylamino-3-amino-phenyl)-2-tert.butyloxycarbonylamino-1-pyrrolidin-1-yl-ethanon

Hergestellt analog Beispiel 1d aus 2-(4-Methylamino-3-nitrophenyl)-2-tert.butyloxycarbonylamino-1-(pyrrolidin-1-yl)-ethanon und Palladium auf Aktivkohle in Methylenchlorid/Ethanol.
Ausbeute: 100 % der Theorie,
$R_f$-Wert: 0.12 (Kieselgel; Cyclohexan/Essigester = 1:1)

h. 2-[4-Methylamino-3-(4-cyanophenylaminomethylcarbonylamino)-phenyl]-2-tert.butyloxycarbonylamino-1-(pyrrolidin-1-yl)-ethanon

Hergestellt analog Beispiel 1c aus 2-(4-Methylamino-3-aminophenyl)-2-tert.butyloxycarbonylamino-1-(pyrrolidin-1-yl)-ethanon, O-(Benzotriazol-1-yl)-N,N,N',N'-tetramethyluroniumtetrafluoroborat, 4-Cyano-phenylglycin und Triethylamin in Tetrahydrofuran.
Ausbeute: 100 % der Theorie,
$R_f$-Wert: 0.50 (Kieselgel; Methylenchlorid/Methanol = 9:1)

i. 2-(4-Cyanophenylaminomethyl)-1-methyl-5-[1-(pyrrolidin-1-yl-carbonyl)-aminomethyl]-benzimidazol

Hergestellt analog Beispiel 1f aus 2-[4-Methylamino-3-(4-cyanophenylaminomethylcarbonylamino)-phenyl]-2-tert.butyloxycarbonylamino-1-(pyrrolidin-1-yl)-ethanon in Eisessig. Ausbeute: 30 % der Theorie,
$R_f$-Wert: 0.19 (Kieselgel; Methylenchlorid/Methanol = 9.5:0.5)

k. 2-(4-Amidinophenylaminomethyl)-1-methyl-5-[1-(pyrrolidin-1-yl-carbonyl)-aminomethyl]-benzimidazol-hydrochlorid

Hergestellt analog Beispiel 1g aus 2-(4-Cyanophenylaminomethyl)-1-methyl-5-[1-(pyrrolidin-1-yl-carbonyl)-aminomethyl]-benzimidazol und Salzsäure/Ammoniumcarbonat in Ethanol.
Ausbeute: 27 % der Theorie,
$C_{22}H_{27}N_7O$ x HCl (405.50/441.96)
Massenspektrum: $(M+H)^+$ = 406

Analog Beispiel 5 werden folgende Verbindungen erhalten:

(1) 2-(4-Amidinophenylaminomethyl)-1-methyl-5-[1-(pyrrolidin-1-yl-carbonyl)-N-acetyl-aminomethyl]-benzimidazol-hydrochlorid
Ausbeute: 29 % der Theorie,
$C_{24}H_{29}N_7O_2$ x HCl (447.54/484.54)
Massenspektrum: $(M+H)^+$ = 448

(2) 2-(4-Amidinophenylaminomethyl)-1-methyl-5-[1-(pyrrolidin-1-yl-carbonyl)-N-(2-ethoxycarbonylethyl)-N-methyl-aminomethyl]-benzimidazol-hydrochlorid
Ausbeute: 74 % der Theorie,
$C_{28}H_{37}N_7O_3$ x HCl (519.65/556.11)
Massenspektrum: $(M+H)^+$ = 520

(3) 2-(4-Amidinophenylaminomethyl)-1-methyl-5-[1-(pyrrolidin-1-yl-carbonyl)-N-(ethoxycarbonylmethyl)-aminomethyl]-benzimidazol-hydrochlorid
Ausbeute: 76 % der Theorie,
$C_{26}H_{33}N_7O_3$ x HCl (491.59/528.05)
Massenspektrum: $(M+H)^+$ = 492
$(M+2H)^{++}$ = 246.7

(4) 2-(4-Amidinophenylaminomethyl)-1-methyl-5-[1-(pyrrolidin-1-yl-carbonyl)-N,N-di-(ethoxycarbonylmethyl)-aminomethyl]-benzimidazol-hydrochlorid
Ausbeute: 51 % der Theorie,
$C_{30}H_{39}N_7O_5$ x HCl (577.68/614.14)
Massenspektrum: $(M+H)^+$ = 578
$(M+Na)^+$ = 600

(5) 2-(4-Amidinophenylaminomethyl)-1-methyl-5- [1-(pyrrolidin-1-yl-carbonyl)-N-(ethoxycarbonylmethylcarbonyl)-aminomethyl]-benzimidazol-hydrochlorid
Ausbeute: 29 % der Theorie,
$C_{27}H_{33}N_7O_4$ x HCl (519.60/556.06)
Massenspektrum: $(M+H)^+$ = 520
$(M+2 H)^{++}$ = 260.7

(6) 2-(4-Amidinophenylaminomethyl)-1-methyl-5-[1-(pyrrolidin-1-yl-carbonyl)-N-(2-ethoxycarbonylethyl)-aminomethyl]-benzimidazol-hydrochlorid
Ausbeute: 84 % der Theorie,
$C_{27}H_{35}N_7O_3$ x HCl (505.62/542.62)
Massenspektrum: $(M+H)^+$ = 506
$(M+2H)^{++}$ = 253.7

Beispiel 6

2-(4-Amidinophenylaminomethyl)-1-methyl-5-[1-[N-(2-ethoxycarbonylethyl)-amino]-1-(pyrrolidin-1-yl-carbonyl)-ethyl]-benzimidazol-hydrochlorid

a. 5-(4-Chlor-3-nitro-phenyl)-5-methyl-imidazolidin-2,4-dion

Zu 50 ml rauchender Salpetersäure werden bei -25°C bis -35°C portionsweise 10.0 g (4.45 mMol) 5-(4-Chlorphenyl)-5-methylimidazolidin-2,4-dion gegeben. Nach 45 Minuten bei -25 bis -20°C wird das Reaktionsgemisch auf Eiswasser gegossen. Das kristalline Produkt wird abgesaugt, mit Wasser gewaschen und getrocknet.
Ausbeute: 10.5 g (100 % der Theorie),
Schmelzpunkt: 173-178°C
$R_f$-Wert: 0.30 (Kieselgel; Cyclohexan/Essigester = 1:1)

b. 2-Amino-2-(4-chlor-3-nitro-phenyl)-propionsäure

10.5 g (0.044 Mol) 5-(4-Chlor-3-nitro-phenyl)-5-methyl-imidazolidin-2,4-dion werden in 200 ml Dioxan und 700 ml 6N Salzsäure 5 Tage unter Rückfluß erhitzt. Die Lösung wird eingedampft, der Rückstand in Wasser aufgenommen und mit Essigester extrahiert. Die wässrige Phase wird eingedampft, mit Toluol versetzt und zur Trockene eingedampft. Der Rückstand wird mit Ether verrieben, abgesaugt und getrocknet.
Ausbeute: 6.8 g (63 % d.Theorie),
$R_f$-Wert: 0.24 (Reversed Phase RP8, 5%ige Kochsalzlösung/Methanol = 1:1)

c. 2-tert.Butyloxycarbonylamino-2-(4-chlor-3-nitro-phenyl)-propionsäure

Hergestellt analog Beispiel 5d aus 2-Amino-2-(4-chlor-3-nitrophenyl)-propionsäure, Pyrokohlensäure-di-tert. butyldicarbonat und Triethylamin in Dioxan.
Ausbeute: 9.6 g (100 % d.Theorie),
$R_f$-Wert: 0.31 (Reversed Phase RP8, 5%ige Kochsalzlösung/Methanol = 1:2)

d. 2-(4-Chlor-3-nitro-phenyl)-2-tert.butyloxycarbonylamino-1-(pyrrolidin-1-yl)-propanon

Hergestellt analog Beispiel 1c aus 2-tert.Butyloxycarbonylamino-2-(4-chlor-3-nitro-phenyl)-propionsäure, O-(Benzotriazol-1-yl)-N,N,N',N'-tetramethyluroniumtetrafluoroborat, Pyrrolidin und N-Methylmorpholin in Dimethylformamid.
Ausbeute: 94 % d.Theorie,
$R_f$-Wert: 0.11 (Kieselgel; Cyclohexan/Essigester = 1:1)

e. 2-(4-Methylamino-3-nitro-phenyl)-2-tert.butyloxycarbonylamino-1-(pyrrolidin-1-yl)-propanon

Hergestellt analog Beispiel 1b aus 2-(4-Chlor-3-nitro-phenyl)-2-tert.butyloxycarbonylamino-1-(pyrrolidin-1-yl)-propanon und Methylaminlösung in Dimethylformamid bei. 160°C.
$R_f$-Wert: 0.79 (Kieselgel; Essigester/Ethanol = 9:1)

f. 2-(4-Methylamino-3-amino-phenyl)-2-tert.butyloxycarbonylamino-1-pyrrolidin-1-yl-propanon

Hergestellt analog Beispiel 1d aus 2-(4-Methylamino-3-nitrophenyl)-2-tert.butyloxycarbonylamino-1-(pyrrolidin-1-yl)-propanon und Palladium auf Aktivkohle/Wasserstoff in Methanol.
Ausbeute: 100 % d.Theorie,
$R_f$-Wert: 0.63 (Kieselgel; Essigester/Ethanol = 9:1)

g. 2-[4-Methylamino-3-(4-cyanophenylaminomethylcarbonylamino)-phenyl]-2-tert.butyloxycarbonylamino-1-(pyrrolidin-1-yl)-propanon

Hergestellt analog Beispiel 1c aus 2-(4-Methylamino-3-aminophenyl)-2-tert.butyloxycarbonylamino-1-(pyrrolidin-1-yl)-propanon, O-(Benzotriazol-1-yl)-N,N,N',N'-tetramethyluroniumtetrafluoroborat, 4-Cyano-phenylglycin und N-Methylmorpholin in Dimethylformamid.
Ausbeute: 37 % der Theorie,
$R_f$-Wert: 0.47 (Kieselgel; Essigester)

h. 2-(4-Cyanophenylaminomethyl)-1-methyl-5-[1-(N-tert.butyloxycarbonylamino)-1-(pyrrolidin-1-yl-carbonyl)-ethyl]-benzimidazol

Hergestellt analog Beispiel 1f aus 2-[4-Methylamino-3-(4-cyanophenylaminomethylcarbonylamino)-phenyl]-2-tert.butyloxycarbonylamino-1-(pyrrolidin-1-yl)-propanon und Eisessig.
Ausbeute: 60 % der Theorie,
$R_f$-Wert: 0.37 (Kieselgel; Essigester)

i. 2-(4-Cyanophenylaminomethyl)-1-methyl-5-[1-amino-1-(pyrrolidin-1-yl-carbonyl)-ethyl]-benzimidazol

1.3 g (2.3 mMol)2-(4-Cyanophenylaminomethyl)-1-methyl-5-[1-(N-tert.butyloxycarbonylamino)-1-(pyrrolidin-1-yl-carbonyl)-ethyl]-benzimidazol werden in 20 ml Dioxan gelöst und nach Zugabe von 40 ml halbkonz. Salzsäure zwei Stunden bei Raumtemperatur gerührt. Die Lösung wird mit Eis versetzt, mit Ammoniak alkalisch gestellt und mit Essigester extrahiert. Die vereinigten organischen Extrakte werden getrocknet und eingedampft.
Ausbeute: 0.9 g (98 % der Theorie),
$R_f$-Wert: 0.14 (Kieselgel; Essigester/Ethanol = 9:1)

k. 2-(4-Cyanophenylaminomethyl)-1-methyl-5-[1-[N-(2-ethoxycarbonylethyl)-amino]-1-(pyrrolidin-1-yl-carbonyl)-ethyl]-benzimidazol

0.4 g (1.04 mMol) 2-(4-Cyanophenylaminomethyl)-1-methyl-5-[1-amino-1-(pyrrolidin-1-yl-carbonyl)-ethyl]-benzimidazol werden in 10 ml Ethanol gelöst und nach Zugabe von 0.3 ml (2.7 mMol) Acrylsäureethylester 24 Stunden bei 95°C. gerührt. Das Solvens wird abdestilliert, der Rückstand an Kieselgel chromatographiert, wobei mit Methylenchlorid/Ethanol (20:1 und 4:1) eluiert wird.
Ausbeute: 0.16 g (31 % der Theorie),
$R_f$-Wert: 0.26 (Kieselgel; Essigester/Ethanol = 9:1)

l. 2-(4-Amidinophenylaminomethyl)-1-methyl-5-[1-[N-(2-ethoxycarbonylethyl)-amino]-1-(pyrrolidin-1-yl-carbonyl)-ethyl]-benzimidazol-hydrochlorid

Hergestellt analog Beispiel 1g aus 2-(4-Cyanophenylaminomethyl)-1-methyl-5-[1-[N-(2-ethoxycarbonylethyl)-amino]-1-(pyrrolidin-1-yl-carbonyl)-ethyl]-benzimidazol und Salzsäure/Ammoniumcarbonat in Ethanol.
Ausbeute: 96 % der Theorie,
$C_{28}H_{37}N_7O_3$ x HCl (519.65/556.11)
Massenspektrum: $(M+H)^+ = 520$
$(M+Na)^+ = 542$

Analog Beispiel 6 werden folgende Verbindungen erhalten:

(1) 2-(4-Amidinophenylaminomethyl)-1-methyl-5-[1-(3-ethoxycarbonylpropionylamino)-1-ethoxycarbonyl-ethyl]-benzimidazol-hydrochlorid
Ausbeute: 69 % der Theorie,
$C_{27}H_{34}N_6O_5$ x HCl (522.62/555.08)
Massenspektrum: $(M+H)^+ = 523$
$(M+H+Na)^{++} = 273$

(2) 2-(4-Amidinophenylaminomethyl)-1-methyl-5-[1-(ethoxycarbonylmethylcarbonylamino)-1-(pyrrolidin-1-yl-carbonyl)-ethyl]-benzimidazol-hydrochlorid
Ausbeute: 95 % der Theorie,
$C_{28}H_{35}N_7O_4$ x HCl (533.64/570.10)

Massenspektrum: $(M+H)^+$ = 534
$(M+Na)^+$ = 556

(3) 2-(4-Amidinophenylaminomethyl)-1-methyl-5-[1-(3-ethoxycarbonylpropionylamino)-1-(pyrrolidin-1-yl-carbonyl)-ethyl]-benzimidazol-hydrochlorid
Ausbeute: 20 % der Theorie,
$C_{29}H_{37}N_7O_4$ x HCl (547.66/584.12)
Massenspektrum: $(M+H)^+$ = 548
$(M+H+Na)^{++}$ = 285.7

(4) 2-[4-Amidinophenyl-N-(2-ethoxycarbonylethyl)-aminomethyl]-1-methyl-5-[1-dimethylamino-1-(pyrrolidin-1-yl-carbonyl)-ethyl]-benzimidazol-hydrochlorid
Ausbeute: 91 % der Theorie,
$C_{30}H_{41}N_7O_3$ x HCl (547.71/584.17)
Massenspektrum: $(M+H)^+$ = 548
$(M-H)^-$ = 546

(5) 2-(4-Amidinophenylaminomethyl)-1-methyl-5-[1-(2-ethoxycarbonylethylamino)-1-(dimethylaminocarbonyl)-ethyl]-benzimidazol-hydrochlorid
Ausbeute: 40 % der Theorie,
$R_f$-Wert: 0.60 (Reversed Phase RP 8; 5%ige Kochsalzlösung/Methanol = 1/1)
$C_{26}H_{35}N_7O_3$ x HCl (493.63/530.08)
Massenspektrum: $(M+H)^+$ = 494
(M-H+2HCl) = 564/566/568 ($Cl_2$)

(6) 2-(4-Amidinophenylaminomethyl)-1-methyl-5-[1-(ethoxycarbonylmethylamino)-1-(pyrrolidin-1-yl-carbonyl)-ethyl]-benzimidazol-hydrochlorid
Ausbeute: 77 % der Theorie,
$R_f$-Wert: 0.40 (Kieselgel; Methylenchlorid/Methanol = 4:1 + 1 % Eisessig)
$C_{27}H_{35}N_7O_3$ x HCl (505.63/542.08)
Massenspektrum: $(M+H)^+$ = 506

(7) 2-(4-Amidinophenylaminomethyl)-1-methyl-5-[1-(2-ethoxycarbonylethyl-amino)-1-(N-ethyl-N-methylaminocarbonyl)-ethyl]-benzimidazol-hydrochlorid
Ausbeute: 85 % der Theorie,
$R_F$-Wert: 0.44 (Kieselgel; Methylenchlorid/Ethanol = 9:1) $C_{27}H_{37}N_7O_3$ x HCl (507.64/544.14)
Massenspektrum: $(M+H)^+$ = 508
$(M+Cl)^-$ = 542/4 (Cl)

(8) 2-(4-Amidinophenylaminomethyl)-1-methyl-5-[1-(methoxycarbonylmethylamino)-1-(pyrrolidinocarbonyl)-ethyl]-benzimidazol-hydrochlorid
Ausbeute: 99 % der Theorie,
$R_f$-Wert: 0.21 (Kieselgel; Methylenchlorid/Methanol = 4:1 + 1 % Eisessig)
$C_{26}H_{33}N_7O_3$ x HCl (491.60/528.05)
Massenspektrum: $(M+H)^+$ = 492
$(M-H+HCl)^-$ = 526/8 (Cl)
$(M-H+2HCl)^-$ = 562/4/8 ($Cl_2$)

(9) 2-(4-Amidinophenylaminomethyl)-1-methyl-5-[1-(N,N-bis(ethoxycarbonylmethyl)amino)-1-(pyrrolidinocarbonyl)-ethyl]-benzimidazol-hydrochlorid
Ausbeute: 65 % der Theorie,
$R_f$-Wert: 0.35 (Kieselgel; Methylenchlorid/Methanol = 4:1 + 1 % Eisessig)
$C_{31}H_{41}N_7O_5$ x HCl (591.72/628.17)
Massenspektrum: $(M+H)^+$ = 592
$(M-H+HCl)^-$ = 626/8 (Cl)
$(M-H+2HCl)^-$ = 662/4/6 ($Cl_2$)

(10) 2-(4-Amidinophenylaminomethyl)-1-methyl-5-[1-(ethoxycarbonylmethylamino)-1-(isoxazolidin-1-yl-carbonyl)-ethyl]-benzimidazol-hydrochlorid
Ausbeute: 9% der Theorie,
$R_f$-Wert: 0.50 (Reversed Phase RP 8; Methanol/5%ige Kochsalzlösung = 3:2)
$C_{26}H_{33}N_7O_4$ x HCl (507.60/544.05)
Massenspektrum: $(M+H)^+$ = 508

(11) 2-(4-Amidinophenylaminomethyl)-1-methyl-5-[1-(2-ethoxycarbonyl-ethylamino)-1-(isoxazolidin-1-yl-carbonyl)-ethyl]-benzimidazol-hydrochlorid

(12) 2-(4-Amidinophenylaminomethyl)-1-methyl-5-[1-(ethoxycarbonylmethylamino)-1-(N-methyl-N-ethylaminocarbonyl)-ethyl]-benzimidazol-dihydrochlorid
Ausbeute: 58% der Theorie,
$R_f$-Wert: 0.70 (Reversed Phase RP 8; Methanol/5%ige Kochsalzlösung = 3:2)
$C_{26}H_{35}N_7O_3$ x 2 HCl (493.62/566.52)
Massenspektrum: $(M+H)^+$ = 494
$(M+HCl-H)^-$ = 528/30 (Cl)

(13) 2-(4-Amidinophenylaminomethyl)-1-methyl-5-[1-((N,N-di-(ethoxycarbonylmethyl)-amino)-1-(N-methyl-N-ethylaminocarbonyl)-ethyl]-benzimidazol-dihydrochlorid
Ausbeute: 30% der Theorie,
$R_F$-Wert: 0.40 (Reversed Phase RP 8; Methanol/5%ige Kochsalzlösung = 3:2)
$C_{30}H_{41}N_7O_5$ x 2 HCl (579,71/652.62)
Massenspektrum: $(M+H)^+$ = 580
$(M-H)^-$ = 578

(14) 2-(4-Amidinophenylaminomethyl)-1-methyl-5-[1-(ethoxycarbonylmethylamino)-1-(piperidinocarbonyl)-ethyl] -benzimidazoldihydrochlorid
Ausbeute: 82% der Theorie,
$R_f$-Wert: 0.60 (Reversed Phase RP 8; Methanol/5%ige Kochsalzlösung = 3:2)
$C_{28}H_{37}N_7O_3$ x 2 HCl (519.65/592.75)
Massenspektrum: $(M+H)^+$ = 520
$(M-H+HCl)^-$ = 534\6 (Cl)
$(M-H+2HCl)^-$ = 590\2\4 ($Cl_2$)

(15) 2-(4-Amidinophenylaminomethyl)-1-methyl-5-[1-(ethoxycarbonylmethylamino)-1-(diethylaminocarbonyl)-ethyl]-benzimidazol-dihydrochlorid
Ausbeute: 88% der Theorie,
$R_f$-Wert: 0.60 (Reversed Phase RP 8; Methanol/5%ige Kochsalzlösung = 3:2)
$C_{27}H_{37}N_7O_3$ x 2 HCl (507.64/580.56)
Massenspektrum: $(M+H)^+$ = 508
$(M-H+2HCl)^-$ = 578/580/582 ($Cl_2$)

(16) 2-(4-Amidinophenylaminomethyl)-1-methyl-5-[1-(ethoxycarbonylmethyl-methylamino)-1-(pyrrolidin-1-yl-carbonyl)-ethyl]-benzimidazol-dihydrochlorid
Ausbeute: 46 % der Theorie,
$R_f$-Wert: 0.43 (Kieselgel; Methylenchlorid/Methanol = 4:1 + 1% Eisessig)
$C_{28}H_{37}N_7O_3$ x 2 HCl (519.65/592.56)
Massenspektrum: $(M+H)^+$ = 520
$(M+Cl)^-$ = 554/6 (Cl)

(17) 2-(4-Amidinophenylaminomethyl)-2-methyl-5-[1-(tetrazol-5-yl-methylamino)-1-(pyrrolidin-1-yl-carbonyl)-ethyl]-benzimidazol-dihydrochlorid

(18) 2-(4-Amidinophenylaminomethyl)-1-methyl-5-[1-(3-ethoxycarbonyl-propylamino)-1-(pyrrolidin-1-yl-carbonyl)-ethyl]-benzimidazol-dihydrochlorid
Ausbeute: 95% der Theorie,
$R_f$-Wert: 0.50 (Reversed Phase RP 8; Methanol/5%ige Kochsalzlösung = 1:1)
$C_{29}H_{39}N_7O_3$ x 2 HCl (533.68/606.58)

Massenspektrum: $(M+H)^+$ = 534

Beispiel 7

2-(4-Amidinophenylaminomethyl)-1-methyl-5-[1-[N-cyclopentyl-N-(3-ethoxycarbonylpropionyl)-amino]cyclopropyl]-benzimidazolhydrochlorid

a. 4-((1-tert.Butyloxycarbonylamino)cyclopropyl)-2-nitro-N-methyl-anilin

15.0 g (63.5 mMol) 4-((1-Carboxy)cyclopropyl)-2-nitro-N-methyl-anilin und 17.6 ml (127 mMol) Triethylamin werden in 250 ml Dichlormethan gelöst und bei 0°C mit 8.3 g (76 mMol) Chlorameisensäureethylester versetzt. Nach einer Stunde bei Raumtemperatur werden 0.75 g Tetrabutylammoniumbromid zugegeben. Anschließend wird eine Lösung von 6.3 g (96 mMol) Natriumazid in 20 ml Wasser zugetropft. Nach einer Stunde bei 0°C wird die Lösung mit Wasser verdünnt und mit Essigester extrahiert. Die organischen Extrakte werden getrocknet und eingedampft. Der Rückstand wird in 200 ml tert.Butanol gelöst und zwei Stunden zum Rückfluß erhitzt. Das Solvens wird eingedampft, der Rückstand an Kieselgel chromatographiert und mit Methylenchlorid eluiert.
Ausbeute: 15.5 g (77 % der Theorie),
$R_f$-Wert: 0.83 (Kieselgel; Methylenchlorid/Methanol = 9.5:0.5)

b. 4-((1-Amino)cyclopropyl)-2-nitro-N-methyl-anilin-hydrochlorid

15.5 g (0.05 Mol) 4-[(1-tert.Butyloxycarbonylamino)cyclopropyl]-2-nitro-N-methyl-anilin werden in 50 ml Ethanol und 50 ml ethanolischer Salzsäure gelöst und 7 Stunden bei Raumtemperatur gerührt. Das Solvens wird abdestilliert, der Rückstand mit Ether verrieben, abgesaugt und getrocknet.
Ausbeute: 98 % der Theorie,
$R_f$-Wert: 0.44 (Kieselgel; Methylenchlorid/Methanol = 9.5:0.5)

c. 4-[N-(1-Cyclopentylamino)cyclopropyl]-2-nitro-N-methyl-anilin

12.0 g (0.05 Mol) 4-[(1-Amino)cyclopropyl] -2-nitro-N-methyl-anilin-hydrochlorid werden in 500 ml Tetrahydrofuran gelöst und nach Zugabe von 4.1 g (0.05 Mol) Cyclopentanon und 3.2 ml Eisessig unter Stickstoffatmosphäre portionsweise mit 13.6 g (0.064 Mol) Natriumtriacetoxyborhydrid versetzt. Nach 16 Stunden bei Raumtemperatur wird mit Natriumhydrogencarbonatlösung verdünnt und mit Essigester extrahiert. Die organischen Extrakte werden getrocknet und eingedampft. Der Rückstand wird an Kieselgel chromatographiert und mit Essigester/Cyclohexan (1:1) eluiert.
Ausbeute: 10.8 g (80 % der Theorie
$R_f$-Wert: 0.56 (Kieselgel; Methylenchlorid/Methanol = 9.5:0.5)

d. 4-[1-(N-(3-Ethoxycarbonylpropionyl)-N-cyclopentyl-amino)-cyclopropyl]-2-nitro-N-methyl-anilin

1.0 g (3.6 mMol) 4-[(1-Cyclopentylamino)cyclopropyl]-2-nitro-N-methyl-anilin werden in 30 ml Tetrahydrofuran gelöst und nach Zugabe von 0.45 g (4.4 mMol) Triethylamin mit 0.65 g (4.4 mMol) Bernsteinsäureethylesterchlorid versetzt und vier Stunden bei Raumtemperatur gerührt. Danach wird mit Essigester und Natriumhydrogencarbonatlösung verdünnt, die organischen Extrakte werden getrocknet und eingedampft. Der Rückstand wird an Kieselgel chromatographiert, wobei mit Essigester/Cyclohexan (1:1) eluiert wird.
Ausbeute: 1.3 g (90 % der Theorie),
$R_f$-Wert: 0.46 (Kieselgel; Essigester/Cyclohexan = 1:1)

e. 4-[1-(N-(3-Ethoxycarbonylpropionyl)-N-cyclopentyl-amino)-cyclopropyl]-2-amino-N-methyl-anilin

Hergestellt analog Beispiel 1d 4-[1-(N-(3-Ethoxycarbonylpropionyl)-N-cyclopentyl-amino)cyclopropyl]-2-nitro-N-methyl-anilin und Palladium auf Aktivkohle/Wasserstoff in Methylenchlorid/Ethanol.
Ausbeute: 100 % der Theorie,
$R_f$-Wert: 0.18 (Kieselgel; Essigester/Cyclohexan = 1:1)

f. 4-[1-(N-(3-Ethoxycarbonylpropionyl)-N-cyclopentyl-amino)-cyclopropyl]-2-(4-cyanophenyl)-aminomethylcarbonylamino-N-methyl-anilin

Hergestellt analog Beispiel 1c 4-[1-(N-(3-Ethoxycarbonylpropionyl)-N-cyclopentyl-amino)cyclopropyl]-2-amino-N-methyl-anilin, O-(Benzotriazol-1-yl)-N,N,N',N'-tetramethyluroniumtetrafluoroborat, 4-Cyano-phenylglycin und Triethylamin in Dimethylformamid.
Ausbeute: 96 % der Theorie,
$R_f$-Wert: 0.54 (Kieselgel; Methylenchlorid/Methanol = 9.5:0.5)

g. 2-(4-Cyanophenylaminomethyl)-1-methyl-5-[1-[N-cyclopentyl-N-(3-ethoxycarbonylpropionyl)-amino]cyclopropyl]-benzimidazol

Hergestellt analog Beispiel 1f aus 4-[1-(N-(3-Ethoxycarbonylpropionyl)-N-cyclopentyl-amino)cyclopropyl]-2-(4-cyanophenyl)-aminomethylcarbonylamino-N-methyl-anilin in Eisessig.
Ausbeute: 52 % der Theorie,
$R_f$-Wert: 0.81 (Kieselgel; Methylenchlorid/Methanol = 9:1)

h. 2-(4-Amidinophenylaminomethyl)-1-methyl-5-[1-[N-cyclopentyl-N-(3-ethoxycarbonylpropionyl)-amino]cyclopropyl]-benzimidazol-hydrochlorid

Hergestellt analog Beispiel 1g aus 2-(4-Cyanophenylaminomethyl)-1-methyl-5-[1-[N-cyclopentyl-N-(3-ethoxycarbonylpropionyl)-amino]cyclopropyl]-benzimidazol und Salzsäure/Ammoniumcarbonat in Ethanol.
Ausbeute: 36 % der Theorie,
$C_{30}H_{38}N_6O_3$ x HCl (530.68/567.14)
Massenspektrum: $(M+H)^+$ = 531

Beispiel 8

2-(4-Amidinophenylaminomethyl)-1-methyl-5-(5-methyl-3-ethoxycarbonylmethyl-imidazolin-2,4-dion-5-yl) benzimidazol-hydrochlorid

a. 2-(4-Cyanophenylaminomethyl)-1-methyl-5-[1-(ethoxycarbonylmethylaminocarbonylamino)-1-(pyrrolidin-1-yl-carbonyl)-ethyl]-benzimidazol

1.0 g (2.5 mMol) 2-(4-Cyanophenylaminomethyl)-1-methyl-5-[1-amino-1-(pyrrolidin-1-yl-carbonyl)-ethyl]-benzimidazol werden in 10 ml Dimethylformamid gelöst und nach Zugabe von 0.9 ml (7.9 mMol) Isocyanatoessigsäureethylester 45 Minuten bei Raumtemperatur gerührt. Die Lösung wird auf Eiswasser gegossen, das kristalline Produkt abgesaugt und getrocknet. Der Rückstand wird an Kieselgel chromatographiert, wobei mit Methylenchlorid/Ethanol/Ammoniak (20:1:0.01 und 10:1:0.01) eluiert wird.
$R_f$-Wert: 0.77 (Kieselgel; Methylenchlorid/Ethanol/Ammoniak = 9:1:0.01)

b. 2-(4-Amidinophenylaminomethyl)-1-methyl-5-(5-methyl-3-ethoxycarbonylmethyl-imidazolin-2,4-dion-5-yl) benzimidazol-hydrochlorid

Hergestellt analog Beispiel 1g aus 2-(4-Cyanophenylaminomethyl)-1-methyl-5-[1-(ethoxycarbonylmethylaminocarbonylamino)-1-(pyrrolidin-1-yl-carbonyl)-ethyl]-benzimidazol und Salzsäure/Ammoniumcarbonat in Ethanol.
Ausbeute: 68 % der Theorie,
$C_{24}H_{27}N_7O_4$ x HCl (477.52/513.99)
Massenspektrum: $(M+H)^+$ = 478

Beispiel 9

2- (4-Amidinophenylaminomethyl)-1-methyl-5-[N- (2-pyridyl)-N-(2-ethoxycarbonylethyl)-aminomethyl]-benzimidazol-hydrochlorid

a. 4-(2-tert.Butyloxycarbonylethyl)-2-(pyridylaminomethyl)-2-nitro-chlorbenzol

13.4 g (0.053 Mol) 4-Chlor-3-nitro-benzylbromid und 11.8 g (0.053 Mol) 2-tert.Butyloxycarbonylethylamino-pyridin werden in 80 ml N-Ethyl-diisopropylamin 3 Stunden bei 90°C gerührt. Die Lösung wird eingedampft, der Rückstand an Kieselgel chromatographiert, wobei mit Petrolether/Essigester (8:2 und 7:3) eluiert wird.
Ausbeute: 8.2 g (40 % der Theorie),
$R_f$-Wert: 0.64 (Kieselgel; Petrolether/Essigester = 8:2)

b. 4-(2-tert.Butyloxycarbonylethyl)-2-(pyridylaminomethyl)-2-nitro-N-methyl-anilin

Hergestellt analog Beispiel 1b aus 4 -(2-tert.Butyloxycarbonylethyl)-2-(pyridylaminomethyl)-2-nitro-chlorbenzol und Methylaminlösung.
Ausbeute: 20 % der Theorie,
$R_f$-Wert: 0.65 (Kieselgel; Methylenchlorid/Ethanol = 9:1)

c. 4-(2-tert.Butyloxycarbonylethyl)-2-(pyridylaminomethyl)-2-amino-N-methyl-anilin

1.6 g (4 mMol) 4-(2-tert.Butyloxycarbonylethyl)-2-(pyridylaminomethyl)-2-nitro-N-methyl-anilin werden in 200 ml Methanol gelöst und nach Zugabe von 2 g Raney-Nickel mit 1 ml Hydrazinhydrat versetzt. Die Lösung wird 30 Minuten bei Raumtemperatur gerührt und eingedampft. Der Rückstand wird an Kieselgel chromatographiert und mit Methylenchlorid/Ethanol (95:5) eluiert.
Ausbeute: 1.2 g (82 % der Theorie),
$R_f$-Wert: 0.33 (Kieselgel; Methylenchlorid/Ethanol = 9:1)

d. 2-(4-Cyanophenylaminomethyl)-1-methyl-5-[N-(2-pyridyl)-N-(2-ethoxycarbonylethyl)-aminomethyl]-benzimidazol

Hergestellt analog Beispiel 1c aus 4-(2-tert.Butyloxycarbonylethyl)-2-(pyridylaminomethyl)-2-amino-N-methyl-anilin, O-(Benzotriazol-1-yl)-N,N,N',N'-tetramethyluroniumtetrafluoroborat, 4-Cyano-phenylglycin in Tetrahydrofuran und Eisessig.
Ausbeute: 72 % der Theorie,
$R_f$-Wert: 0.36 (Kieselgel; Methylenchlorid/Ethanol = 9:1)

e. 2-(4-Amidinophenylaminomethyl)-1-methyl-5-[N-(2-pyridyl)-N-(2-ethoxycarbonylethyl)-aminomethyl]-benzimidazol-hydrochlorid

Hergestellt analog Beispiel 1g aus 4-[(5-(2-tert.Butyloxycarbonylethyl)-2-pyridylaminomethyl-1-methyl-benzimidazol-2-yl)-methylamino]-benzonitril und Salzsäure/Ammoniumcarbonat in Ethanol.
Ausbeute: 59 % der Theorie,
$C_{27}H_{31}N_7O_2$ x HCl (485.59/522.1)
Massenspektrum: $(M+H)^+$ = 486

Analog Beispiel 9 werden folgende Verbindungen erhalten:

(1) 2-(4-Amidinophenylaminomethyl)-1-methyl-5-[N-(ethoxycarbonylmethyl)-benzolsulfonylaminomethyl]-benzimidazol-hydrochlorid
Ausbeute: 53 % der Theorie,
$C_{27}H_{30}N_6O_4S$ x HCl (534.64/571.1)
Massenspektrum: $(M+H)^+$ = 535

(2) 2-(4-Amidinophenylaminomethyl)-1-methyl-5-(N-methyl-phenylcarbonylaminomethyl)]-benzimidazol-hydrochlorid
Ausbeute: 42 % der Theorie,
$C_{25}H_{26}N_6O$ x HCl (426.53/462.96)
Massenspektrum: $(M+H)^+$ = 427

Beispiel 10

2-(4-Amidinophenylaminomethyl)-1-methyl-5-[(2-methyl-benzimidazol-1-yl)methyl]-benzimidazol-hydrochlorid

a. 1-(4-Chlor-3-nitrobenzyl)-2-methyl-benzimidazol

Hergestellt analog Beispiel 9a aus 2-Methyl-benzimidazol und 4-Chlor-3-nitrobenzylchlorid in Dimethylsulfoxid.
Ausbeute: 78% der Theorie,
$C_{15}H_{12}ClN_3O_2$ (301.7)
Massenspektrum: $M^+$ = 301/303

b. 1-(4-Methylamino-3-nitrobenzyl)-2-methyl-benzimidazol

Hergestellt analog Beispiel 1b aus 1-(4-Chlor-3-nitrobenzyl)-2-methyl-benzimidazol und Methylamin.
Ausbeute: 96% der Theorie,
$R_f$-Wert: 0.56 (Kieselgel; Methylenchlorid/Ethanol = 19:1)

c. 1-(4-Methylamino-3-aminobenzyl)-2-methyl-benzimidazol

Hergestellt analog Beispiel 1c aus 1-(4-Methylamino-3-nitrobenzyl)-2-methyl-benzimidazol und Wasserstoff/Raney-Nickel.
Ausbeute: 100% der Theorie,
$R_f$-Wert: 0.34 (Kieselgel; Methylenchlorid/Ethanol = 19:1)

d. 2-(4-Cyanophenylaminomethyl)-1-methyl-5-[(2-methyl-benzimidazol-1-yl)methyl]-benzimidazol

Eine Mischung aus 1.94 g (11.0 mMol) N-(4-Cyanophenyl)-glycin und 1.78 g (11.0 mMol) Carbonyldiimidazol wird in 80 ml absolutem Tetrahydrofuran 15 Minuten unter Rückfluß erhitzt. Nach Zugabe von 2.7 g (10.46 mMol) 1-(4-Methylamino-3-aminobenzyl)-2-methyl-benzimidazol wird die Mischung weitere 16 Stunden unter Rückfluß erhitzt. Anschließend wird die Lösung zur Trockene eingedampft, der Rückstand mit 80 ml Eisessig versetzt und 1 Stunde unter Rückfluß erhitzt. Danach wird erneut zur Trockene eingedampft, der so erhaltene Rückstand mit 50 ml Wasser versetzt und mit konz. Ammoniak alkalisch gestellt (ca. pH 10). Das dabei auskristallisierte Produkt wird abgesaugt, mit wenig Wasser gewaschen und getrocknet.
Ausbeute: 4.1 g (96% der Theorie),
$R_f$-Wert: 0.30 (Kieselgel; Methylenchlorid/Ethanol = 19:1)
$C_{25}H_{22}N_6$ (406.5)
Massenspektrum: M+ = 406

e. 2-(4-Amidinophenylaminomethyl)-1-methyl-5-[(2-methyl-benzimidazol-1-yl)methyl]-benzimidazol-hydrochlorid

Hergestellt analog Beispiel 1g aus 2-(4-Cyanophenylaminomethyl)-1-methyl-5-[(2-methyl-benzimidazol-1-yl)methyl]-benzimidazol Salzsäure/Ammoniumcarbonat.
Ausbeute: 59% der Theorie,
$C_{25}H_{25}N_7$ x HCl (423.5/459.9)
Massenspektrum: $(M+H)^+$ = 424
$(M+2H)^{2+}$ = 217.7

Analog Beispiel 10 werden folgende Verbindungen erhalten:

(1) 2-(4-Amidinophenyloxymethyl)-1-methyl-5-[(imidazol-1-yl)-methyl]-benzimidazol-hydrochlorid
Ausbeute: 30% der Theorie,
$C_{20}H_{20}N_6O$ x HCl (360.4/396.9)
Massenspektrum: $(M+H)^+$ = 361
$(M+2H)^{2+}$ = 181

(2) 2-(4-Amidinophenylaminomethyl)-1-methyl-5-[1-(imidazol-1-yl)-ethyl]-benzimidazol-hydrochlorid
Ausbeute: 70% der Theorie,
$C_{21}H_{23}N_7$ x HCl (373.46/410)
Massenspektrum: $(M+H)^+$ = 374
$(M+2H)^{2+}$ = 187.6

(3) 2-(4-Amidinophenylaminomethyl)-1-methyl-5-[1-(2-ethyl-4-methyl-imidazol-1-yl)-ethyl]-benzimidazol-dihydrochlorid
Ausbeute: 18% der Theorie,
$C_{24}H_{29}N_7$ x 2HCl (415.55/488.46)
Massenspektrum: $(M+H)^+$ = 416
$(M+2H)^{2+}$ = 208.7

(4) 2-(4-Amidinophenylaminomethyl)-1-methyl-5-[(2-ethyl-4-methyl-imidazol-1-yl)-methyl]-benzimidazol-dihydrochlorid Ausbeute: 36% der Theorie,

$C_{23}H_{27}N_7$ x 2HCl (401.52/437.97)
Massenspektrum: $(M+H)^+$ = 402
$(M+2H)^{2+}$ = 201.7

(5) 2-(4-Amidinophenylaminomethyl)-1-methyl-5-[N-(pyridin-2-yl)-N-methyl-aminomethyl]-benzimidazol-dihydrochlorid
Ausbeute: 78% der Theorie,
$C_{23}H_{25}N_7$ x 2HCl (399.5/435.95)
Massenspektrum: $(M+H)^+$ = 400
$(M+2H)^{2+}$ = 200.6

(6) 2-(4-Amidinophenylaminomethyl)-1-methyl-5-[(2-(2-ethoxycarbonyl-ethyl)-benzimidazol-1-yl)-methyl]-benzimidazol-dihydrochlorid
Ausbeute: 61% der Theorie,
$C_{29}H_{31}N_7O_2$ x HCl (509.62/546.07)
Massenspektrum: $(M+H)^+$ = 510
$(M+2H)^{2+}$ = 255.7
$(M+H+Na)^{2+}$ = 266.7

(7) 2-(4-Amidinophenylaminomethyl)-1-methyl-5-[(imidazol-1-yl)-methyl]-benzimidazol-hydrochlorid
Ausbeute: 35% der Theorie,
$C_{20}H_{21}N_7$ x HCl (359.44/395.89)
Massenspektrum: $(M+H)^+$ = 360
$(M+2H)^{2+}$ = 180.6

(8) 2-(4-Amidinophenylaminomethyl)-1-methyl-5-[(2-(2-acetylamino-ethyl)-4,5-dimethyl-imidazol-1-yl)-methyl]-benzimidazoldihydrochlorid
Ausbeute: 42% der Theorie,
$C_{26}H_{32}N_8O$ x 2HCl (472.6/545.51)
Massenspektrum: $(M+H)^+$ = 473
$(M+2H)^{2+}$ = 237
$(M+H+Na)^{2+}$ = 248

(9) 2-(4-Amidinophenylaminomethyl)-1-methyl-5-[(2-(2-aminocarbonyl-ethyl)-4,5-dimethyl-imidazol-1-yl)-methyl]-benzimidazoldihydrochlorid
Ausbeute: 68% der Theorie,
$C_{25}H_{30}N_8O$ x 2HCl (458.6/531.51)
Massenspektrum: $(M+H)^+$ = 459
$(M+2H)^{2+}$ = 230

(10) 2-(4-Amidinophenylaminomethyl)-1-methyl-5-[(2-methyl-4-ethoxycarbonyl-imidazol-1-yl)-methyl]-benzimidazol-hydrochlorid
Ausbeute: 34% der Theorie,
$C_{24}H_{27}N_7O_2$ x HCl (445.53/481.98)
Massenspektrum: $(M+H)^+$ = 446
$(M+2H)^{2+}$ = 223.5
$(M+H+Na)^{2+}$ = 234.5

(11) 2-(4-Amidinophenylaminomethyl)-1-methyl-5-[(3-(3-ethoxycarbonyl-n-propyl)-benzimidazol-2-on-1-yl)-methyl]-benzimidazol-hydrochlorid
Ausbeute: 58% der Theorie,
$C_{30}H_{33}N_7O_3$ x HCl (539.64/576.09)
Massenspektrum: $(M+H)^+$ = 540
$(M+H+Na)^{2+}$ = 281.7

(12) 2-(4-Amidinophenylaminomethyl)-1-methyl-5-[(3-(2-ethoxycarbonyl-ethyl)-imidazo[4,5-b]pyridin-2-on-1-yl)-methyl]-benzimidazol-hydrochlorid
Ausbeute: 29% der Theorie,

$C_{28}H_{30}N_8O_3$ x HCl (526.6/563.05)
Massenspektrum: $(M+H)^+$ = 527
$(M+2H)^{2+}$ = 264
$(M+H+Na)^{2+}$ = 275

(13) 2-(4-Amidinophenylaminomethyl)-1-methyl-5-[(2-phenyl-imidazol-1-yl)-methyl]-benzimidazol-hydrochlorid
Ausbeute: 58% der Theorie,
$C_{26}H_{25}N_7$ x HCl (435.54/472)
Massenspektrum: $(M+H)^+$ = 436
$(M+Na)^+$ = 218.6

(14) 2-(4-Amidinophenylaminomethyl)-1-methyl-5-[(4,5-dimethyl-2-(2-ethoxycarbonylethyl)-imidazol-1-yl)-methyl]-benzimidazoldihydrochlorid
Ausbeute: 52% der Theorie,
$C_{27}H_{33}N_7O_2$ x 2HCl (487.61/560.52)
Massenspektrum: $(M+H)^+$ = 488
$(M+2H)^{2+}$ = 244.6

Beispiel 11

2-(4-Amidinophenylaminomethyl)-1-methyl-5-[1-(2-ethoxycarbonyl-azetidin-1-yl)-1-(pyrrolidin-1-yl-carbonyl)-ethyl]-benzimidazol-hydrochlorid

a. 2-(4-Cyanophenylaminomethyl)-1-methyl-5-[1-(2-tert.butyloxycarbonyl-azetidin-1-yl)-1-(pyrrolidin-1-yl-carbonyl)-ethyl]-benzimidazol

0.8 g (1.86 mMol) 2-(4-Cyanophenylaminomethyl)-1-methyl-5-[1-amino-1-(pyrrolidin-1-yl-carbonyl)-ethyl]-benzimidazol und 1.65 g ((5.5 mMol) 2,4-Dibrombuttersäure-tert.butylester werden in 5 ml Ethanol gelöst, mit 0.2 g (1.86 mMol) Natriumcarbonat versetzt und unter Stickstoff 30 Stunden bei 55°C gerührt. Nach Abkühlung wird vom weißen Niederschlag abfiltriert und mit Ethanol gewaschen. Das Filtrat wird eingedampft, der Rückstand wird an Kieselgel chromatographiert, wobei als Elutionsmittel Essigester und Essigester/Ethanol/Ammoniak (20:1:0.01) verwendet werden. Die gewünschten Fraktionen werden vereinigt und eingedampft.
Ausbeute: 0.44 g (44% der Theorie) als Diastereomerengemisch, $C_{31}H_{38}N_6O_3$ (542.69)
Massenspektrum: $(M+H)^+$ = 543

b. 2-(4-Amidinophenylaminomethyl)-1-methyl-5-[1-(2-ethoxycarbonyl-azetidin-1-yl)-1-(pyrrolidin-1-yl-carbonyl)-ethyl]-benzimidazol-hydrochlorid (Diastereomerengemisch)

Hergestellt analog Beispiel 1g aus 2-(4-Cyanophenylaminomethyl)-1-methyl-5-[1-(2-tert.butyloxycarbonyl-azetidin-1-yl)-1-(pyrrolidin-1-yl-carbonyl)-ethyl]-benzimidazol und Salzsäure/Ammoniumcarbonat in Ethanol.
Ausbeute: 12 % der Theorie,
$C_{29}H_{37}N_7O_3$ x HCl (531.66/568.12)
Massenspektrum: $(M+H)^+$ = 532
$(M+H+HCl)^{2+}$ = 568/70 (Cl)

Beispiel 12

(E/Z)-2-(4-Amidinophenylaminomethyl)-1-methyl-5-[1-[(pyridin-3-yl)-ethoxycarbonylmethyliden)-methylen]cyclopropyl]-benzimidazol-hydrochlorid

a. (E/Z)-2-(4-Cyanophenylaminomethyl)-1-methyl-5-[1-[(pyridin-3-yl)-ethoxycarbonylmethyliden)-methylen]cyclopropyl]-benzimidazol

897 mg (4.0 mMol) Phosphonoessigsäure-triethylester werden unter Argon in 30 ml Tetrahydrofuran gelöst. Bei -15°C werden 449 mg (4.0 mMol) Kalium-tert.butylat zugesetzt. Nach 30 Minuten werden 815 mg (2.0 mMol) 2-(4-Cyanophenylaminomethyl)-1-methyl-5-[1-(pyridin-3-yl-carbonyl)cyclopropyl]-benzimidazol portionsweise zugegeben und über Nacht bei Raumtemperatur gerührt.Danach wird die Lösung noch 6 Stunden zum Rückfluß erhitzt und eingeengt. Der Rückstand wird mit Natriumchlorid-Lösung versetzt und 3x mit Essigester extrahiert. Die vereinigten

organischen Phasen werden über Natriumsulfat getrocknet und eingeengt. Der Rückstand wird in Dichlormethan gelöst und an Kieselgel chromatographiert, wobei als Elutionsmittel Dichlormethan mit 5% Ethanol verwendet wird. Die gewünschten Fraktionen werden eingeengt, der Rückstand wird mit Ether verrieben, abgesaugt und getrocknet.
Ausbeute: 365 mg (38% der Theorie).

b. ((E/Z)-2-(4-Amidinophenylaminomethyl)-1-methyl-5-[1-[(pyridin-3-yl)-ethoxycarbonylmethyliden)-methylen]cyclopropyl]-benzimidazol-hydrochlorid

Hergestellt analog Beispiel 1g aus (E/Z)-2-(4-Cyanophenylaminomethyl)-1-methyl-5-[1-[(pyridin-3-yl)-ethoxycarbonylmethyliden)-methylen]cyclopropyl]-benzimidazol und Salzsäure/Ammoniumcarbonat in Ethanol.
Ausbeute: 58 % der Theorie,
$C_{29}H_{30}N_6O_2$ x HCl (494.60/531.05)
Massenspektrum: $(M+H)^+$ = 495

Analog Beispiel 12 wird folgende Verbindung erhalten:

(1) (E/Z)-2-(4-Amidinophenylaminomethyl)-1-methyl-5-[1-[(pyridin-2-yl)-ethoxycarbonylmethyliden)-methylen]-cyclopropyl]-benzimidazol-hydrochlorid
Ausbeute: 72 % der Theorie,
$C_{29}H_{30}N_6O_2$ x HCl (494.60/531.05)
Massenspektrum: $(M+H)^+$ =495

Beispiel 13

2-(4-Amidinophenylaminomethyl)-1-methyl-5-[1-(2-carboxy-azetidin-1-yl)-1-(pyrrolidin-1-yl-carbonyl)-ethyl]-benzimidazolhydrochlorid (Diastereomerengemisch)

200 mg (0.35 mMol) 2-(4-Amidinophenylaminomethyl)-1-methyl-5-[1-(2-ethoxycarbonyl-azetidin-1-yl)-1-(pyrrolidin-1-ylcarbonyl)-ethyl]-benzimidazol-hydrochlorid werden in 30 ml 6N Salzsäure gelöst und 13 Stunden bei Raumtemperatur gerührt. Das Reaktionsgemisch wird unter Zusatz von Toluol eingedampft, der Rückstand wird mit Aceton/Ether verrieben, abgesaugt, mit Ether gewaschen und getrocknet.
Ausbeute: 200 mg (>100 % der Theorie, enthält Ammoniumchlorid),
$C_{27}H_{33}N_7O_3$ x HCl (503.62/540.07)
Massenspektrum: $(M+H)^+$ = 504

Analog Beispiel 13 werden folgende Verbindungen erhalten:

(1) 2-(4-Amidinophenylaminomethyl)-1-methyl-5-[1-(2-carboxyethylamino)-1-(dimethylaminocarbonyl)-ethyl]-benzimidazolhydrochlorid
Ausbeute: 91% der Theorie,
$R_F$-Wert: 0.75 (Reversed Phase; 5%ige Kochsalzlösung/Methanol = 1:1)
$C_{24}H_{31}N_7O_3$ x HCl (465.56/502.01)
Massenspektrum: $(M+H)^+$ = 466
$(M-H+2HCl)^-$ = 537/539 ($Cl_2$)

(2) 2-(4-Amidinophenylaminomethyl)-1-methyl-5-[1-(carboxymethylamino)-1-(pyrrolidin-1-yl-carbonyl)-ethyl]-benzimidazoldihydrochlorid
Ausbeute: 70 % der Theorie,
$R_F$-Wert: 0.51 (Reversed Phase; 5%ige Kochsalzlösung/Methanol = 3:2)
$C_{25}H_{31}N_7O_3$ x 2 HCl (477.57/550.48)
Massenspektrum: $(M+H)^+$ = 478

(3) (E/Z)-2-(4-Amidinophenylaminomethyl)-1-methyl-5-[1-[(pyridin-2-yl)-carboxymethyliden)-methylen]-cyclopropyl]-benzimidazol-hydrochlorid
Ausbeute: 65 % der Theorie,
$C_{27}H_{26}N_6O_2$ x HCl (466.55/503.0)
Massenspektrum: $(M+H)^+$ = 467
$(M+Cl)^+$ = 501/503 (Cl)

(4) 2-(4-Amidinophenylaminomethyl)-1-methyl-5-[1-(N-methylcarboxymethylcarbonylamino)-2-(pyrrolidinocarbonyl)-prop-2-yl]-benzimidazol-dihydrochlorid
Ausbeute: 99 % der Theorie,
$R_f$-Wert: 0.55 (Reversed Phase RP 8; Methanol/5%ige Kochsalzlösung = 1:1)
$C_{28}H_{35}N_7O_4$ x 2 HCl (533.64/606.64)
Massenspektrum: $(M+H)^+$ = 534
$(M-H)^-$ = 532
$(M-H+HCl)^-$ = 568/70 (Cl)

(5) 2-(4-Amidinophenylaminomethyl)-1-methyl-5-[1-(2-carboxyethyl-amino)-1-(N-ethyl-N-methylaminocarbonyl)-ethyl]-benzimidazol-dihydrochlorid
Ausbeute: 75 % der Theorie,
$R_f$-Wert: 0.54 (Reversed Phase RP 8; Methanol/5%ige Kochsalzlösung = 1:1)
$C_{25}H_{33}N_7O_3$ x 2 HCl (479.59/552.59)
Massenspektrum: $(M+H)^+$ = 480

Beispiel 14

2-[4-(N-Hexyloxycarbonylamidino)-phenylaminomethyl]-1-methyl-5-[1-(2-ethoxycarbonylethylamino)-1-(dimethylaminocarbonyl)-ethyl]-benzimidazol

1.5 g (2.8 mMol) 2-(4-Amidinophenylaminomethyl)-1-methyl-5-[1-(2-ethoxycarbonylethylamino)-1-(dimethylaminocarbonyl)-ethyl]-benzimidazol-hydrochlorid werden in 14 ml Wasser und 55 ml Tetrahydrofuran gelöst, mit 2.0 g Kaliumcarbonat und 1.0 ml (6 mMol) Chlorameisensäurehexylester versetzt und 4 Stunden bei Raumtemperatur gerührt. Nach Abzug des Solvens im Vakuum wird der Rückstand mit Kochsalzlösung versetzt und 3 x mit Methylenchlorid extrahiert. Die vereinigten organischen Phasen werden mit wenig Wasser gewaschen, über Magnesiumsulfat getrocknet und eingedampft. Das Rohprodukt wird an Kieselgel gereinigt, wobei mit Methylenchlorid plus 2 bis 7.5% Ethanol eluiert wird. Die einheitlichen Fraktionen werden vereinigt, eingedampft, in wenig Essigester gelöst und mit Petrolether versetzt. Der gebildete Feststoff wird abgesaugt, mit Petrolether gewaschen und getrocknet.
Ausbeute: 0.8 g (43 % der Theorie),
$C_{33}H_{47}N_7O_5$ (621.79)
$R_f$-Wert: 0.50 (Kieselgel; Methylenchlorid/Ethanol = 19:1) Massenspektrum: $(M+H)^+$ = 622
$(M+Na)^+$ = 644

Beispiel 15

2-(4-Amidinophenylaminomethyl)-1-methyl-5-[1-(N-ethoxycarbonylmethylcarbonyl-methylamino)-2-(pyrrolidinocarbonyl)-prop-2-yl]-benzimidazol-hydrochlorid

a. 2-(4-Chlor-phenyl)-3-hydroxy-2-methyl-propionsäuremethylester

Zu einer Lösung von 8.1 ml Diisopropylamin (85 mMol) in 20 ml Tetrahydrofuran werden bei -78°C 35 ml einer 1.6 molaren Lösung von n-Butyllithium in Hexan (61 mMol) zugetropft. Anschließend wird eine Lösung von 10.0 g (50 mMol) 2-(4-Chlorphenyl)-propionsäuremethylester in 30 ml Tetrahydrofuran bei -78°C zugetropft. In das Reaktionsgemisch wird anschließend bei -20°C für 30 Minuten gasförmiges Formaldehyd eingeleitet. Nach Zugabe von 5%iger Citronensäure und Eisessig wird mit Essigester extrahiert. Die organischen Phasen werden mit 1N Schwefelsäure, Wasser, gesättigter Natriumbicarbonatlösung und Kochsalzlösung gewaschen und über Magnesiumsulfat getrocknet. Das Rohprodukt wird an Kieselgel gereinigt, wobei mit Cyclohexan/Essigester (19:1; 9:1; 4:1; 1:1 und 0:1) eluiert wird. Die einheitlichen Fraktionen werden vereinigt und eingedampft.
Ausbeute: 9.7 g (84% der Theorie) gelbes Öl,
$R_F$-Wert: 0.25 (Kieselgel; Petrolether/Essigester 4:1)

b. 2-(4-Chlor-phenyl)-3-hydroxy-2-methyl-propionsäure

Hergestellt analog Beispiel 4 aus 2-(4-Chlor-phenyl)-3-hydroxy-2-methyl-propionsäuremethylester und Natronlauge in Ethanol.
Ausbeute: 83% der Theorie,
$R_F$-Wert: 0.55 (Kieselgel; Essigester/Cyclohexan 2:1 + Eisessig)

c. 2-(4-Chlor-3-nitro-phenyl)-2-methyl-3-nitrooxy-propionsäure

Hergestellt analog Beispiel 1a aus 2-(4-Chlor-phenyl)-3-hydroxy-2-methyl-propionsäure und Salpetersäure.
Ausbeute: 90% der Theorie,
Schmelzpunkt: 129-132°C
$C_{10}H_9ClN_2O_7$ (304.64)

d. 2-(4-Chlor-3-nitro-phenyl)-2-methyl-3-hydroxy-propionsäure

Hergestellt analog Beispiel 6 aus 2-(4-Chlor-3-nitro-phenyl)-3-nitrooxy-2-methyl-propionsäure und 6N Salzsäure in Dioxan.
Ausbeute: 98% der Theorie,
$C_{10}H_{10}ClNO_5$ (259.65)
Massenspektrum: $(M-H)^-$ = 258/60 (Cl)
$(2M-H)^-$ = 517/9 ($Cl_2$)

e. 2-[4-(N-Benzyl-methylamino)-3-nitro-phenyl]-2-methyl-3-hydroxy-propionsäure

Hergestellt analog Beispiel 1b aus 2-(4-Chlor-3-nitro-phenyl)-3-hydroxy-2-methyl-propionsäure und N-Methyl-benzylamin. Ausbeute: 81% der Theorie,
$C_{18}H_{20}ClN_2O_5$ (344.37)
Massenspektrum: $M^+$ = 344

f. 2-[4-(N-Benzyl-methylamino)-3-nitro-phenyl]-2-methyl-3-hydroxy-1-pyrrolidin-1-yl-propan-1-on

Hergestellt analog Beispiel 1c aus 2-[4-(N-Benzyl-methylamino)-3-nitro-phenyl]-3-hydroxy-2-methyl-propionsäure und N-Methyl-benzylamin.
Ausbeute: 96% der Theorie,
$C_{22}H_{27}N_3O_4$ (397.48)
Massenspektrum: $M^+$ = 398
$(M+Na)^+$ = 420

g. 2-[4-(N-Benzyl-methylamino)-3-nitro-phenyl]-2-methyl-3-methansulfonyloxy-1-pyrrolidin-1-yl-propan-1-on

Eine Lösung von 1.2 g (3.0 mMol) 2-[4-(N-Benzyl-methylamino)-3-nitro-phenyl]-2-methyl-3-hydroxy-1-pyrrolidin-1-yl-propan-1-on in 20 ml Tetrahydrofuran wird bei Raumtemperatur mit 1.3 ml (9.3 mMol) Triethylamin versetzt. Anschließend werden bei 2-5°C 0.27 ml (3.5 mMol) Methansulfonylchlorid zugetropft. Nach 2 Stunden bei Raumtemperatur wird der gebildete Niederschlag abgesaugt und das Filtrat eingedampft. Das Rohprodukt wird ohne Reinigung weiter umgesetzt.
Ausbeute: 1.4 g (98% der Theorie)

h. 2-[4-(N-Benzyl-methylamino)-3-nitro-phenyl]-2-methyl-3-methylamino-1-pyrrolidin-1-yl-propan-1-on

Eine Lösung von 1.4 g (2.9 mMol) 2-[4-(N-Benzyl-methylamino)-3-nitro-phenyl]-2-methyl-3-methansulfonyloxy-1-pyrrolidin-1-yl-propan-1-on in 10 ml Dimethylformamid wird mit 20 ml einer 40%igen wäßrigen Methylaminlösung versetzt und 70 Minuten auf 100°C erhitzt. Nach Abkühlung wird das Reaktionsgemisch mit Eiswasser versetzt und mit Essigester extrahiert. Die organischen Phasen werden mit Wasser und mit Kochsalzlösung gewaschen, über Magnesiumsulfat getrocknet und eingedampft. Das Rohprodukt wird an Kieselgel gereinigt, wobei als Elutionsmittel Essigester/Ethanol (10:1, 9:1, 4:1 + 1% konz. Ammoniak) verwendet wird. Die einheitlichen Fraktionen werden vereinigt und eingedampft.
Ausbeute: 740 mg (61% der Theorie),
$R_F$-Wert: 0.45 (Kieselgel; Methylenchlorid/Ethanol 9:1 + 1% konz. Ammoniak)

i. 2-[4-(Benzyl-methylamino)-3-nitro-phenyl]-2-methyl-3-(N-methoxycarbonylmethylcarbonyl-methylamino)-1-pyrrolidin-1-yl-propan-1-on

Hergestellt analog Beispiel 7d aus 2-[4-(N-Benzyl-methylamino)-3-nitro-phenyl]-2-methyl-3- methylamino-1-pyrrolidin-1-yl-propan-1-on und Malonsäure-methylester-chlorid.

Ausbeute: 84% der Theorie,
$R_F$-Wert: 0.65 (Kieselgel; Essigester/Ethanol 9:1 + Ammoniak) $C_{27}H_{34}N_4O_6$ (510.60)
Massenspektrum: $(M-H)^-$ = 509
$(M+Na)^+$ = 533

j. 2-(4-Methylamino-3-amino-phenyl)-2-methyl-3-(N-methoxycarbonylmethylcarbonyl-methylamino)-1-pyrrolidin-1-yl-propan-1-on

Hergestellt analog Beispiel 1d aus 2-[4-(N-Benzyl-methylamino)-3-nitro-phenyl]-2-methyl-3-(N-methoxycarbonylmethylcarbonyl-methylamino)-1-pyrrolidin-1-yl-propan-1-on und Wasserstoff/Palladium auf Aktivkohle.
Ausbeute: 100% der Theorie,
$R_F$-Wert: 0.40 (Kieselgel; Essigester/Ethanol 9:1 +1% konz. Ammoniak)
$C_{20}H_{30}N_4O_4$ (390.49)
Massenspektrum: $M^+$ = 390

k. 4-[2-(3-(N-Methoxycarbonylmethylcarbonyl-methylamino))-2-methyl-1-pyrrolidin-1-yl-propan-1-on-2-yl]-2-(4-cyanophenyl)-aminomethylcarbonylamino-N-methyl-anilin

Hergestellt analog Beispiel 1e aus 2-(4-Methylamino-3-aminophenyl)-2-methyl-3-(N-methoxycarbonylmethylcarbonyl-methylamino)-1-pyrrolidin-1-yl-propan-1-on und O-(Benzotriazol-1-yl)-N,N,N'N'-tetramethyluroniumtetrafluoroborat, 4-Cyanophenylglycin und Triethylamin in Dimethylformamid.
Ausbeute: 95% der Theorie,
$R_F$-Wert: 0.35 (Kieselgel; Essigester/Ethanol 9:1 + 1% konz. Ammoniak)

l. 2-(4-Cyanophenylaminomethyl)-1-methyl-5-[1-(N-ethoxycarbonylmethylcarbonyl-methylamino)-2-(pyrrolidinocarbonyl)-prop-2-yl]-benzimidazol

Hergestellt analog Beispiel 1f aus 4-[2-(3-(N-Methoxycarbonylmethylcarbonyl-methylamino))-2-methyl-1-pyrrolidin-1-ylpropan-1-on-2-yl]-2-(4-cyanophenyl)-aminomethylcarbonylamino-N-methyl-anilin in Eisessig.
Ausbeute: 47 % der Theorie,
$R_f$-Wert: 0.20 (Kieselgel; Essigester/Ethanol = 9:1)
$C_{29}H_{34}N_6O_4$ (530.63)
Massenspektrum: $(M+H)^+$ = 531
$(M+Na)^+$ = 553

m.2-(4-Amidinophenylaminomethyl)-1-methyl-5-[1-(N-ethoxycarbonylmethylcarbonyl-methylamino)-2-(pyrrolidinocarbonyl)-prop-2-yl]-benzimidazol-hydrochlorid

Hergestellt analog Beispiel 1g aus 2-(4-Cyanophenylaminomethyl)-1-methyl-5-[1-(N-ethoxycarbonylmethylcarbonyl-methylamino)-2-(pyrrolidinocarbonyl)-prop-2-yl]-benzimidazol und Salzsäure/Ammoniumcarbonat in Ethanol.
Ausbeute: 65 % der Theorie,
$R_f$-Wert: 0.30 (Kieselgel; Methylenchlorid/Methanol = 4:1 + Eisessig)
$C_{30}H_{39}N_7O_4$ x HCl (561.69/598.19)
Massenspektrum: $(M+H)^+$ = 562
$(M+Cl)^-$ = 596/8 (Cl)

Analog Beispiel 15 werden folgende Verbindungen erhalten:

(1) 2-(4-Amidinophenylaminomethyl)-1-methyl-5-[1-(N-methoxycarbonylmethyl-methylamino)-2-(pyrrolidinocarbonyl)-prop-2-yl]-benzimidazol-hydrochlorid
Ausbeute: 89 % der Theorie,
$R_f$-Wert: 0.35 (Reversed Phase RP 8; Methanol/5%ige Kochsalzlösung = 3:2)
$C_{28}H_{37}N_7O_3$ x HCl (519.66/556.11)
Massenspektrum: $(M+H)^+$ = 520
$(M-H+HCl)^-$ = 554/6 (Cl)

(2) 2-(4-Amidinophenylaminomethyl)-1-methyl-5-[1-(N-ethoxycarbonylmethylcarbonylamino)-2-(pyrrolidinocarbonyl)-prop-2-yl]-benzimidazol-acetat

Ausbeute: 45% der Theorie,
$R_f$-Wert: 0.20 (Kieselgel; Methylenchlorid/Ethanol = 8:2 + 1 % Essigester)
$C_{29}H_{37}N_7O_4$ x $CH_3COOH$ (547.66/607.71)
Massenspektrum: $(M+H)^+ = 548$
$(M-H)^- = 546$
$(M-H+CH_3COOH)^- = 606$

(3) 2-(4-Amidinophenylaminomethyl)-1-methyl-5-[1-(N-ethoxycarbonylmethylcarbonyl-methylamino)-2-(N-methyl-ethylaminocarbonyl)-prop-2-yl]-benzimidazol-hydrochlorid

(4) 2-(4-Amidinophenylaminomethyl)-1-methyl-5-[1-(N-ethoxycarbonylmethyl-methylamino)-2-(N-methyl-N-ethylaminocarbonyl)-prop-2-yl]-benzimidazol-hydrochlorid

(5) 2-(4-Amidinophenylaminomethyl)-1-methyl-5-[1-(N-ethoxycarbonylmethylcarbonyl-methylamino)-2-(piperidinocarbonyl)-prop-2-yl]-benzimidazol-hydrochlorid

(6) 2-(4-Amidinophenylaminomethyl)-1-methyl-5-[1-(N-ethoxycarbonylmethylsulfonyl-methylamino)-2-(piperidinocarbonyl)-prop-2-yl]-benzimidazol-hydrochlorid

(7) 2-(4-Amidinophenylaminomethyl)-1-methyl-5-[1-(N-methoxycarbonylmethylcarbonyl-methylamino)-2-(pyrrolidinocarbonyl)-prop-2-yl]-benzimidazol-acetat
Ausbeute: 72% der Theorie,
$R_f$-Wert: 0.20 (Kieselgel; Methylenchlorid/Ethanol = 8:2 + 1 % Eisessig)
$C_{29}H_{37}N_7O_4$ x $CH_3COOH$ (547.66/607.71)
Massenspektrum: $(M+H)^+ = 548$
$(M-H)^- = 546$

(8) 2-(4-Amidinophenylaminomethyl)-1-methyl-5-[1-(2-ethoxycarbonyl-ethylcarbonylamino)-2-(pyrrolidinocarbonyl)-prop-2-yl] -benzimidazol-hydrochlorid

(9) 2-(4-Amidinophenylaminomethyl)-1-methyl-5-[1-(ethoxycarbonylmethylsulfonylamino)-2-(pyrrolidinocarbonyl)-prop-2-yl]-benzimidazol-hydrochlorid
Ausbeute: 14 % der Theorie,
$R_f$-Wert: 0.20 (Kieselgel; Methylenchlorid/Ethanol = 8:2) $C_{28}H_{37}N_7O_5S$ x HCl (583.65/620.17)
Massenspektrum: $(M+H)^+ = 584$
$(M+Na)^+ = 606$

(10) 2-(4-Amidinophenylaminomethyl)-1-methyl-5-[1-(1H-tetrazol-5-yl)-methylcarbonylamino)-2-(pyrrolidinocarbonyl)-prop-2-yl]-benzimidazol-hydrochlorid

(11) 2-(4-Amidinophenylaminomethyl)-1-methyl-5-[1-(ethoxycarbonylmethylamino)-2-(pyrrolidinocarbonyl)-prop-2-yl)-benzimidazol-dihydrochlorid
Ausbeute: 40 % der Theorie,
$C_{28}H_{37}N_7O_3$ x 2 HCl (519.65/592.56)
Massenspektrum: $(M+H)^+ = 520$
$(M+Na)^+ = 542$
$(M+HCOO)^- = 564$
$(M+Cl)^- = 554$

<u>Beispiel 16</u>

<u>2-(4-Amidinophenylaminomethyl)-1-methyl-5-[1-(carboxymethylamino)-1-(isoxazolidin-1-yl-carbonyl)-ethyl]-benzimidazol-hydrochlorid</u>

Hergestellt durch Hydrolyse von 2-(4-Amidinophenylaminomethyl)-1-methyl-5-[1-(ethoxycarbonylmethylamino)-1-(isoxazolidin-1-yl-carbonyl)-ethyl]-benzimidazol-hydrochlorid mit Natronlauge in Ethanol.
Ausbeute: 90 % der Theorie,
$R_f$-Wert: 0.65 (Reversed Phase RP 8; Methanol/5%ige Kochsalzlösung = 3:2)

$C_{24}H_{29}N_7O_4$ x HCl (479.54/515.99)
Massenspektrum: $(M+H)^+$ = 480

Analog Beispiel 16 werden folgende Verbindungen hergestellt:

(1) 2-(4-Amidinophenylaminomethyl)-1-methyl-5-[1-(2-carboxyethylamino)-1-(isoxazolidin-1-yl-carbonyl)-ethyl]-benzimidazol-hydrochlorid

(2) 2-(4-Amidinophenylaminomethyl)-1-methyl-5-[1-(carboxymethylamino)-1-(N-methyl-N-ethylaminocarbonyl)-ethyl] -benzimidazol-hydrochlorid
Ausbeute: 93% der Theorie,
$R_f$-Wert: 0.40 (Reversed Phase RP 8; Methanol/5%ige Kochsalzlösung = 1:1)
$C_{24}H_{31}N_7O_3$ x HCl (465.57/502.02)
Massenspektrum: $(M+H)^+$ = 466
$(M+Cl-H)^-$ = 500/2 (Cl)

(3) 2-(4-Amidinophenylaminomethyl)-1-methyl-5-[1-(N-carboxymethyl-methylamino)-2-(pyrrolidinocarbonyl)-prop-2-yl]-benzimidazol-dihydrochlorid
Ausbeute: 89% der Theorie,
$R_f$-Wert: 0.57 (Reversed Phase RP 8; Methanol/5%ige Kochsalzlösung = 4:3)
$C_{27}H_{35}N_7O_3$ x 2 HCl (505.63/578.54)
Massenspektrum: $(M+H)^+$ = 506
$(M+2H)^{++}$ = 253
$(M+H+Na)^{++}$ = 264.5

(4) 2-(4-Amidinophenylaminomethyl)-1-methyl-5-[1-(carboxymethylcarbonylamino)-2-(pyrrolidinocarbonyl)-prop-2-yl]-benzimidazol-hydrochlorid
Ausbeute: 90% der Theorie,
$R_f$-Wert: 0.55 (Reversed Phase RP 8; Methanol/5%ige Kochsalzlösung = 4:6)
$C_{27}H_{33}N_7O_4$ x HCl (519.61/556.06)
Massenspektrum: $(M+H)^+$ = 520
$(M-H)^-$ = 518

(5) 2-(4-Amidinophenylaminomethyl)-1-methyl-5-[1-(N-carboxymethyl-methylamino)-2-(N-ethyl-methylaminocarbonyl)-prop-2-yl]-benzimidazol-hydrochlorid

(6) 2-(4-Amidinophenylaminomethyl)-1-methyl-5-[1-(N-carboxymethylcarbonyl-methylamino)-2-(N-ethyl-methylaminocarbonyl)-prop-2-yl]-benzimidazol-hydrochlorid

(7) 2-(4-Amidinophenylaminomethyl)-1-methyl-5-[1-(N-carboxymethylcarbonyl-methylamino)-2-(piperidinocarbonyl)-prop-2-yl]-benzimidazol-hydrochlorid

(8) 2-(4-Amidinophenylaminomethyl)-1-methyl-5-[1-(N-carboxymethylsulfonyl-methylamino)-2-(piperidinocarbonyl)-prop-2-yl]-benzimidazol-hydrochlorid

(9) 2-(4-Amidinophenylaminomethyl)-1-methyl-5-[1-(carboxymethylamino)-1-(piperidinocarbonyl)-ethyl]-benzimidazol-hydrochlorid
Ausbeute: 81% der Theorie,
$R_f$-Wert: 0.40 (Reversed Phase RP 8; Methanol/5%ige Kochsalzlösung = 1:1)
$C_{26}H_{33}N_7O_3$ (491.60/528.05)
Massenspektrum: $(M+H)^+$ = 492
$(M-H)^-$ = 490

(10) 2-(4-Amidinophenylaminomethyl)-1-methyl-5-[1-(2-carboxyethylcarbonylamino)-2-(pyrrolidinocarbonyl)-prop-2-yl]-benzimidazol-hydrochlorid

(11) 2-(4-Amidinophenylaminomethyl)-1-methyl-5-[1-(carboxymethylsulfonylamino)-2-(pyrrolidinocarbonyl)-prop-2-yl]-benzimidazol-hydrochlorid

(12) 2-(4-Amidinophenylaminomethyl)-1-methyl-5-[1-(carboxymethylamino)-1-(diethylaminocarbonyl)-ethyl]-benzimidazolhydrochlorid
Ausbeute: 70% der Theorie,
$R_f$-Wert: 0.50 (Reversed Phase RP 8; Methanol/5%ige Kochsalzlösung = 1:1)
$C_{25}H_{33}N_7O_3$ x HCl (479.59/516.05)
Massenspektrum: $(M+H)^+$ = 480
$(M-H)^-$ = 478
$(M-H+HCl)^-$ = 514/516 (Cl)

(13) 2-(4-Amidinophenylaminomethyl)-1-methyl-5-[1-(carboxymethylamino)-2-(pyrrolidinocarbonyl)-prop-2-yl]-benzimidazol-dihydrochlorid
Ausbeute: 22 % der Theorie,
$R_f$-Wert: 0.50 (Reversed Phase RP8; 5%ige Kochsalzlösung/Methanol = 1:1)
$C_{26}H_{33}N_7O_3$ x 2 HCl (491.60/564.51)
Massenspektrum: $(M+H)^+$ = 492
$(M-H)^-$ = 490

(14) 2-(4-Amidinophenylaminomethyl)-1-methyl-5-[1-(N-carboxymethyl-methylamino)-1-(pyrrolidinocarbonyl)-ethyl]-benzimidazol-dihydrochlorid
$R_f$-Wert: 0.48 (Reversed Phase RP8; 5%ige Kochsalzlösung/Methanol = 3:2)
$C_{26}H_{33}N_7O_3$ x 2 HCl (491.60/564.51)
Massenspektrum: $(M+H)^+$ = 492
$(M-H)^-$ = 490

(15) 2-(4-Amidinophenylaminomethyl)-1-methyl-5-[1-(3-carboxypropylamino)-1-(pyrrolidinocarbonyl)-ethyl]-benzimidazol-dihydrochlorid
Ausbeute: 82 % der Theorie,
$R_f$-Wert: 0.73; (Reversed Phase; 5%ige Natriumchloridlösung/Methanol = 1:1)
$C_{27}H_{35}N_7O_3$ x 2 HCl (505.63/578.54)
Massenspektrum: $(M+H)^+$ = 506

Beispiel 17

2-[4-(N-Phenylcarbonylamidino)-phenylaminomethyl]-1-methyl-5-[1-(ethoxycarbonylmethylamino)-1-(pyrrolidinocarbonyl)-ethyl]-benzimidazol

Eine Suspension von 1.4 g (2.4 mMol) 2-(4-Amidinophenylaminomethyl)-1-methyl-5-[1-(ethoxycarbonylmethylamino)-1-(pyrrolidinocarbonyl)-ethyl]-benzimidazol-dihydrochlorid in 5 ml N-Ethyl-diisopropylamin und 2 ml Dimethylformamid wird mit 1.5 g (6 mMol) Benzoesäure-4-nitrophenylester versetzt, wobei unter Erwärmung in eine klare Lösung entsteht. Nach 2 Stunden bei 120°C wird die Lösung im Vakuum eingedampft, der Rückstand wird nach Abkühlung in Dichlormethan gelöst und an Kieselgel gereinigt, wobei mit zunächst mit Dichlormethan, später mit Dichlormethan/Ethanol (50:1, 25:1, 18:1) eluiert wird. Die einheitlichen Fraktionen werden vereinigt, eingedampft, mit Wasser verrieben, abgesaugt und getrocknet.
Ausbeute: 0.7 g (49% der Theorie),
$R_f$-Wert: 0.40 (Kieselgel; Methylenchlorid/Ethanol = 19:1) $C_{34}H_{39}N_7O_4$ (609.73)
Massenspektrum: $(M+H)^+$ = 610
$(M+Na)^+$ = 632
$(M-H)^-$ = 608

Analog den Beispielen 14 und 17 werden folgende Verbindungen erhalten:

(1) 2-[4-(N-n-Hexyloxycarbonylamidino)-phenylaminomethyl]-1-methyl-5-[1-(ethoxycarbonylmethylamino)-1-(pyrrolidinocarbonyl)-ethyl]-benzimidazol
Ausbeute: 53% der Theorie,
$R_f$-Wert: 0.35 (Kieselgel; Methylenchlorid/Ethanol = 9:1) $C_{34}H_{47}N_7O_5$ (633.79)
Massenspektrum: $(M+Na)^+$ = 656
$(M-H)^-$ = 632

(2) 2- [4-(N-n-Octyloxycarbonylamidino)-phenylaminomethyl]-1-methyl-5-[1-(ethoxycarbonylmethylamino)-1-(pyrrolidinocarbonyl)-ethyl]-benzimidazol
Ausbeute: 46% der Theorie,
$R_f$-Wert: 0.43 (Kieselgel; Methylenchlorid/Ethanol = 9:1) $C_{36}H_{51}N_7O_5$ (661.84)
Massenspektrum: $(M+Na)^+$ = 684
$(M-H)^-$ = 660

(3) 2-[4-(N-n-Hexyloxycarbonyl-amidino)-phenylaminomethyl)-1-methyl-5-[1-(methoxycarbonylmethylamino)-1-(pyrrolidinocarbonyl)-ethyl]-benzimidazol

(4) 2-[4-(N-n-Octyloxycarbonylamidino)-phenylaminomethyl]-1-methyl-5-[1-(methoxycarbonylmethylamino)-1-(pyrrolidinocarbonyl)ethyl]-benzimidazol
Ausbeute: 32% der Theorie,
$R_f$-Wert: 0.27 (Kieselgel; Methylenchlorid/Ethanol = 9:1)
$C_{35}H_{49}N_7O_5$ (647.82)
Massenspektrum: $(M+Na)^+$ = 670
$(M-H)^-$ = 646

(5) 2-[4-(N-n-Hexyloxycarbonylamidino)-phenylaminomethyl]-1-methyl-5-[1-(N-ethoxycarbonylmethylcarbonyl-methylamino)-2-(pyrrolidinocarbonyl)-prop-2-yl]-benzimidazol
Ausbeute: 52% der Theorie,
$R_f$-Wert: 0.60 (Kieselgel; Methylenchlorid/Ethanol = 9:1)
$C_{37}H_{51}N_7O_6$ (689.85)
Massenspektrum: $(M+H)^+$ = 690
$(M-H)^-$ = 688)
$(M+Na)^+$ = 712
$(M+HCl-H)^-$ = 724/26 (Cl)

(6) 2-[4-(N-n-Octyloxycarbonyl-amidino)-phenylaminomethyl)-1-methyl-5-[1-(N-ethoxycarbonylmethylcarbonyl-methylamino)-2-(pyrrolidinocarbonyl)-prop-2-yl]-benzimidazol

(7) 2-[4-(N-n-Hexyloxycarbonylamidino)-phenylaminomethyl]-1-methyl-5-[1-(N-methoxycarbonylmethylcarbonyl-methylamino)-2- (pyrrolidinocarbonyl) -prop-2-yl]-benzimidazol
Ausbeute: 21% der Theorie,
$R_f$-Wert: 0.55 (Kieselgel; Methylenchlorid/Ethanol = 9:1)
$C_{36}H_{49}N_7O_6$ (675.83)
Massenspektrum: $(M+H)^+$ = 676
$(M+Na)^+$ = 698
$(M+HCl-H)^-$ = 724/26 (Cl)

(8) 2-[4-(N-n-Octyloxycarbonyl-amidino)-phenylaminomethyl)-1-methyl-5-[1-(N-methoxycarbonylmethylcarbonyl-methylamino)-2-(pyrrolidinocarbonyl)-prop-2-yl] -benzimidazol

(9)2-[4-(N-Phenylcarbonylamidino)-phenylaminomethyl]-1-methyl-5-[1-(methoxycarbonylmethylamino)-1-(pyrrolidinocarbonyl)-ethyl]-benzimidazol
Ausbeute: 34% der Theorie.
$R_f$-Wert: 0.55 (Kieselgel; Methylenchlorid/Ethanol = 9:1 + 1 % Ammoniak)
$C_{33}H_{37}N_7O_4$ (595.70)
Massenspektrum: $(M-H)^-$ = 594
$(M+Na)^+$ = 618

(10) 2-[4-(N-Isopropyloxycarbonylamidino)-phenylaminomethyl]-1-methyl-5-[1-(N-ethoxycarbonylmethylcarbonyl-methylamino)-2-(pyrrolidinocarbonyl)-prop-2-yl]-benzimidazol
Ausbeute: 66% der Theorie,
$R_f$-Wert: 0.45 (Kieselgel; Methylenchlorid/Ethanol = 9:1) $C_{34}H_{45}N_7O_6$ (647.77)
Massenspektrum: $(M+H)^+$ = 648
$(M-H)^-$ = 646
$(M+Na)^+$ = 670

(11) 2-[4-(N-Phenylcarbonylamidino)-phenylaminomethyl]-1-methyl-5-[1-(N-ethoxycarbonylmethylcarbonyl-methylamino)-2-(pyrrolidinocarbonyl)-prop-2-yl]-benzimidazol
Ausbeute: 23% der Theorie,
$R_f$-Wert: 0.45 (Kieselgel; Methylenchlorid/Ethanol = 9:1) $C_{37}H_{43}N_7O_5$ (665.79)
Massenspektrum: $(M+H)^+$ = 666
$(M-H)^-$ = 664
$(M+Na)^+$ = 688
$(M+H+Cl)^+$ = 700/2 (Cl)

(12) 2-[4-(N-Phenylcarbonylamidino)-phenylaminomethyl]-1-methyl-5-[1-(N-methoxycarbonylmethylcarbonyl-methylamino)-2-(pyrrolidinocarbonyl)-prop-2-yl]-benzimidazol
Ausbeute: 67% der Theorie,
$R_f$-Wert: 0.60 (Kieselgel; Methylenchlorid/Ethanol = 9:1)
$C_{36}H_{41}N_7O_5$ (651.76)
Massenspektrum: $(M+H)^+$ = 652
$(M-H)^-$ = 650
$(M+Na)^+$ = 674

(13) 2-[4-(N-n-Butyloxycarbonylamidino)-phenylaminomethyl]-1-methyl-5-[1-(N-methoxycarbonylmethylcarbonyl-methylamino)-2-(pyrrolidinocarbonyl)-prop-2-yl]-benzimidazol
Ausbeute: 45% der Theorie,
$R_f$-Wert: 0.50 (Kieselgel; Methylenchlorid/Ethanol = 9:1) $C_{34}H_{45}N_7O_6$ (647.77)
Massenspektrum: $(M+H)^+$ = 648
$(M-H)^-$ = 646
$(M+Na)^+$ = 670
$(M-H+HCl)^-$ = 682/4 (Cl)

(14) 2-[4-(N-Ethyloxycarbonylamidino)-phenylaminomethyl]-1-methyl-5-[1-(N-ethoxycarbonylmethylcarbonyl-methylamino)-2-(pyrrolidinocarbonyl)-prop-2-yl]-benzimidazol
Ausbeute: 54% der Theorie,
$R_f$-Wert: 0.40 (Kieselgel; Methylenchlorid/Ethanol 9:1) $C_{33}H_{43}N_7O_6$ (633.75)
Massenspektrum: $(M+H)^+$ = 634
$(M-H)^-$ = 632
$(M+Na)^+$ = 656

(15) 2-[4-(N-Ethyloxycarbonylamidino)-phenylaminomethyl]-1-methyl-5-[1-(N-methoxycarbonylmethylcarbonyl-methylamino)-2-(pyrrolidinocarbonyl)-prop-2-yl]-benzimidazol
Ausbeute: 53% der Theorie,
$R_f$-Wert: 0.45 (Kieselgel; Methylenchlorid/Ethanol = 9:1) $C_{32}H_{41}N_7O_6$ (619.72)
Massenspektrum: $(M+H)^+$ = 620
$(M-H)^-$ = 618
$(M+Na)^+$ = 642

(16) 2-[4-(N-Pyridin-3-yl-carbonylamidino)-phenylaminomethyl]-1-methyl-5-[1-(N-ethoxycarbonylmethylcarbonyl-methylamino)-2-(pyrrolidinocarbonyl)-prop-2-yl]-benzimidazol
Ausbeute: 16% der Theorie,
$R_f$-Wert: 0,45 (Kieselgel; Methylenchlorid/Ethanol = 9:1) $C_{36}H_{42}N_8O_5$ (666.78)
Massenspektrum: $(M-H)^-$ = 665
$(M+Na)^+$ = 689

(17)2-[4-(N-n-Butyloxycarbonylamidino)-phenylaminomethyl]-1-methyl-5-[1-(ethoxycarbonylmethylamino)-1-(pyrrolidinocarbonyl)-ethyl]-benzimidazol
Ausbeute: 52% der Theorie,
$R_f$-Wert: 0.42 (Kieselgel; Methylenchlorid/Ethanol = 9:1) $C_{32}H_{43}N_7O_5$ (605.74)
Massenspektrum: $(M+H)^+$ = 606
$(M+Na)^+$ = 628
$(M-H)^-$ = 604

(18)2-[4-(N-Ethyloxycarbonylamidino)-phenylaminomethyl]-1-methyl-5-[1-(ethoxycarbonylmethylamino)-1-(pyrrolidinocarbonyl)-ethyl]-benzimidazol
Ausbeute: 30% der Theorie,
$R_f$-Wert: 0.44 (Kieselgel; Methylenchlorid/Ethanol = 9:1) $C_{30}H_{39}N_7O_5$ (577.68)
Massenspektrum: $(M+H)^+ = 578$
$(M+Na)^+ = 600$
$(M-H)^- = 576$

(19)2-[4-(N-Benzyloxycarbonylamidino)-phenylaminomethyl]-1-methyl-5-[1-(ethoxycarbonylmethylamino)-1-(pyrrolidinocarbonyl)-ethyl]-benzimidazol
Ausbeute: 51% der Theorie,
$R_f$-Wert: 0.50 (Kieselgel; Methylenchlorid/Ethanol = 9:1) $C_{35}H_{41}N_7O_5$ (639.75)
Massenspektrum: $(M+Na)^+ = 662$
$(M-H)^- = 638$

(20) 2-[4-(N-Pyridin-3-yl-carbonylamidino)-phenylaminomethyl]-1-methyl-5-[1-(ethoxycarbonylmethylamino)-1-(pyrrolidinocarbonyl)-ethyl]-benzimidazol
Ausbeute: 84 % der Theorie,
$R_f$-Wert: 0.20 (Kieselgel; Essigester/Ethanol = 4:1)
$C_{33}H_{38}N_8O_4$ (610.72)
Massenspektrum: $(M+H)^+ = 611$
$(M-H)^- = 609$
$(M-HCOO)^- = 611$

(21) 2-[4-(N-Acetoxymethyloxycarbonylamidino)-phenylaminomethyl]-1-methyl-5-[1-(ethoxycarbonylmethylamino)-1-(pyrrolidinocarbonyl)-ethyl]-benzimidazol
Ausbeute: 42% der Theorie,
$R_f$-Wert: 0.44 (Kieselgel; Methylenchlorid/Ethanol = 9:1) $C_{31}H_{39}N_7O7_5$ (621.09)
Massenspektrum: $(M+Na)^+ = 644$
$(M-H)^- = 620$

(22)2-[4-(N-(2,2,2-Trichlorethyloxycarbonyl)-amidino)-phenylaminomethyl]-1-methyl-5-[1-(ethoxycarbonylmethylamino)-1-(pyrrolidinocarbonyl)-ethyl]-benzimidazol
Ausbeute: 73% der Theorie,
$R_f$-Wert: 0.54 (Kieselgel; Methylenchlorid/Ethanol = 9:1) $C_{30}H_{36}Cl_3N_7O_5$ (681)
Massenspektrum: $M^+ = 679/81/3$ $(Cl_3)$
$(M+Na)^+ = 702/4/6$ $(Cl_3)$
$(M-H)^- = 678/80/2$ $(Cl_3)$

Beispiel 18

2-[4-(N-n-Octyloxycarbonylamidino)-phenylaminomethyl]-1-methyl-5-[1-(carboxymethylamino)-1-(pyrrolidinocarbonyl)-ethyl]-benzimidazol

Eine Lösung von 0.2 g (0.3 mMol) 2-[4-(N-n-Octyloxycarbonylamidino)-phenylaminomethyl]-1-methyl-5-[1-(ethoxycarbonylmethylamino)-1-(pyrrolidinocarbonyl)-ethyl]-benzimidazol in 3 ml Tetrahydrofuran und 2.5 ml Ethanol wird mit 1.1 ml 1N Natronlauge versetzt und 4 Stunden bei Raumtemperatur gerührt. Das Reaktionsgemisch wird eingedampft und mit 1 ml 1N Salzsäure versetzt. Nach 12 Stunden bei Raumtemperatur (pH 4) werden 2 Tropfen Ammoniak (33%ig) zugesetzt, wobei ein hellgelber Niederschlag ausfällt. Nach Absaugen des gebildeten Feststoffes wird das Filtrat mit 1 ml 1 N Salzsäure versetzt und unter Zusatz von Toluol eingedampft. Der Rückstand wird mit Aceton verrieben, abgesaugt, mit Diethylether gewaschen und getrocknet.
Ausbeute: 0.1 g (50% der Theorie),
$R_f$-Wert: 0.35 (Reversed Phase RP 8; Methanol/5%ige Kochsalzlösung = 2:1)
$C_{34}H_{47}N_7O_5$ (633.79)
Massenspektrum: $(M+H)^+ = 634$
$(M+H+Na)^{++} = 328,5$

Beispiel 19

2-[4-(N-Hydroxyamidino)-phenylaminomethyl]-1-methyl-5-[1-(ethoxycarbonylmethylamino)-1-(pyrrolidinocarbonyl)-ethyl]-benzimidazol

Eine Suspension von 0.6 g (1,2 mMol) 2-(4-Cyanophenylaminomethyl)-1-methyl-5-[1-(ethoxycarbonylmethylamino)-1-(pyrrolidinocarbonyl)-ethyl]-benzimidazol in 50 ml Ethanol wird mit 0.47 g (7.8 mMol) Hydroxylamin-hydrochlorid und 0.35 g (3.5 mMol) Natriumcarbonat versetzt und 17 Stunden unter Rückfluß erhitzt. Nach Abkühlung wird der Rückstand abfiltriert, das Filtrat wird eingedampft und in Wasser aufgenommen. Nach zweifacher Extraktion mit Dichlormethan werden die vereinigten organischen Phasen getrocknet und eingedampft. Das Rohprodukt wird an Kieselgel gereinigt, wobei mit Dichlormethan/Ethanol (19/1 und 7/1) eluiert wird. Die einheitlichen Fraktionen werden vereinigt, eingedampft, mit Diisopropylether verrieben und getrocknet.
Ausbeute: 0.025 g (4% der Theorie),
$R_f$-Wert: 0.68 (Kieselgel; Methylenchlorid/Ethanol = 4:1) $C_{27}H_{35}N_7O_4$ (521.62)
Massenspektrum: $(M-H)^-$ = 520
$(M+Na)^+$ = 544

Beispiel 20

2-[4-(N-Phenylcarbonylamidino)-phenylaminomethyl]-1-methyl-5-[1-(isopropyloxycarbonylmethylamino)-1-(pyrrolidinocarbonyl)-ethyl]-benzimidazol

a. 4-(5-Methyl-1,2,4-oxadiazol-3-yl)-phenylamino-essigsäureethylester

Hergestellt analog Beispiel 9a aus 4-(5-Methyl-1,2,4-oxadiazol-3-yl)-anilin und Bromessigsäureethylester in N-Ethyl-diisopropylamin.
Ausbeute: 78% der Theorie,
$R_f$-Wert: 0.60 (Kieselgel; Essigester/Petrolether = 1:1)

b. 4-(5-Methyl-1,2,4-oxadiazol-3-yl)-phenylamino-essigsäure

Hergestellt analog Beispiel 4 aus 4-(5-Methyl-1,2,4-oxadiazol-3-yl)-phenylamino-essigsäureethylester und Natronlauge in Ethanol.
Ausbeute: 75% der Theorie,
$R_f$-Wert: 0.15 (Kieselgel; Methylenchlorid/Ethanol = 9:1)

c. 2-[4-(5-Methyl-1,2,4-oxadiazol-3-yl)-phenylaminomethyl]-1-methyl-5-[1-amino-(pyrrolidin-1-yl-carbonyl)-ethyl]-benzimidazol

Hergestellt analog Beispiel 1e/f aus 2-(4-Methylamino-3-aminophenyl)-2-tert.butyloxycarbonyl-amino-1-pyrrolidin-1-yl-propanon, 4-(5-Methyl-1,2,4-oxadiazol-3-yl)-phenylamino-essigsäure und Carbonyldiimidazol in Tetrahydrofuran und anschließender Behandlung mit Eisessig.
Ausbeute: 34% der Theorie,
$R_f$-Wert: 0.10 (Kieselgel; Methylenchlorid/Ethanol = 9:1)

d. 2-[4-(5-Methyl-1,2,4-oxadiazol-3-yl)-phenylaminomethyl]-1-methyl-5-[1-(isopropyloxycarbonylmethylamino)-1-(pyrrolidinocarbonyl)-ethyl]-benzimidazol

Hergestellt analog Beispiel 11 aus 2-[4-(5-Methyl-1,2,4-oxadiazol-3-yl)-phenylaminomethyl]-1-methyl-5-[1-amino-1-(pyrrolidinocarbonyl)-ethyl]-benzimidazol, Bromessigsäureisopropylester und Kaliumcarbonat in Isopropanol/Methylenchlorid.
Ausbeute: 42% der Theorie,
$R_f$-Wert: 0.60 (Kieselgel; Methylenchlorid/Ethanol = 9:1)

e. 2-(4-Amidinophenylaminomethyl)-1-methyl-5-[1-(isopropyloxycarbonylmethylamino)-1-(pyrrolidinocarbonyl)-ethyl]-benzimidazol-acetat

Hergestellt analog Beispiel 1d aus 2-[4-(5-Methyl-1,2,4-oxadiazol-3-yl)-phenylaminomethyl]-1-methyl-5-[1-(isopropyloxycarbonylmethylamino)-1-(pyrrolidinocarbonyl)-ethyl]-benzimidazol und Wasserstoff/Palladium (10% auf Aktivkohle) in Ethanol/Eisessig.
Ausbeute: 69% der Theorie,
$R_f$-Wert: 0.30 (Kieselgel; Methylenchlorid/Ethanol = 7:3)

f. 2-[4-(N-Phenylcarbonylamidino)-phenylaminomethyl]-1-methyl- 5-[1-(isopropyloxycarbonylmethylamino)-1-(pyrrolidinocarbonyl)-ethyl]-benzimidazol

Hergestellt analog Beispiel 17 aus 2-(4-Amidinophenylaminomethyl)-1-methyl-5-[1-(isopropyloxycarbonylmethylamino)-1-(pyrrolidinocarbonyl)-ethyl]-benzimidazol-acetat und Benzoesäure-4-nitrophenylester in N-Ethyl-diisopropylamin/Dimethylformamid.
Ausbeute: 26% der Theorie,
$R_f$-Wert: 0.50 (Kieselgel; Methylenchlorid/Ethanol = 9:1)
$C_{35}H_{41}N_7O_4$ (623.75)
Massenspektrum: $(M+Na)^+ = 646$
$(M-H)^- = 622$

Analog Beispiel 20 werden folgende Verbindungen hergestellt:

(1) 2-[4-(N-Phenylcarbonylamidino)-phenylaminomethyl]-1-methyl-5-[1-(n-butyloxycarbonylmethylamino)-1-(pyrrolidinocarbonyl)-ethyl]-benzimidazol

(2) 2-[4-(N-Phenylcarbonylamidino)-phenylaminomethyl]-1-methyl-5-[1-(2-phenylethyloxycarbonylmethylamino)-1-(pyrrolidinocarbonyl)-ethyl]-benzimidazol

(3) 2-[4-(N-n-Hexyloxycarbonylamidino)-phenylaminomethyl]-1-methyl-5-[1-(isopropyloxycarbonylmethylamino)-1-(pyrrolidinocarbonyl)-ethyl]-benzimidazol
Ausbeute: 40% der Theorie,
$R_f$-Wert: 0.45 (Kieselgel; Methylenchlorid/Ethanol = 9:1)
$C_{35}H_{49}N_7O_5$ (647.82)
Massenspektrum: $(M+H)^+ = 648$
$(M-H)^- = 646$
$(M+Na)^+ = 670$

(4) 2-[4-(N-n-Octyloxycarbonylamidino)-phenylaminomethyl]-1-methyl-5-[1-(isopropyloxycarbonylmethylamino)-1-(pyrrolidinocarbonyl)-ethyl]-benzimidazol
Ausbeute: 31 % der Theorie,
$R_f$-Wert: 0.48 (Kieselgel; Methylenchlorid/Ethanol = 9:1)
$C_{37}H_{53}N_7O_5$ (675.88)
Massenspektrum: $(M+H)^+ = 674$
$(M+Na)^+ = 698$

(5) 2-[4-(N-(2,2,2-Trichlorethyloxycarbonyl)-amidino)-phenylaminomethyl]-1-methyl-5-[1-(isopropyloxycarbonylmethylamino)-1-(pyrrolidinocarbonyl)-ethyl]-benzimidazol
Ausbeute: 43 % der Theorie,
$R_f$-Wert: 0.50 (Kieselgel; Methylenchlorid/Ethanol = 9:1)
$C_{31}H_{38}Cl_3N_7O_5$ (695.05)
Massenspektrum: $(M-H)^- = 692/694/696/698$ ($Cl_3$)

(6) 2-[4-(N-Phenylcarbonylamidino)-phenylaminomethyl]-1-methyl-5-[1-(n-propyloxycarbonylmethylamino)-1-(pyrrolidinocarbonyl)-ethyl]-benzimidazol
Ausbeute: 79 % der Theorie,
$R_f$-Wert: 0.35 (Kieselgel; Essigester/Ethanol = 9:1)
$C_{35}H_{41}N_7O_4$ (623.76)
Massenspektrum: $(M+H)^+ = 624$
$(M-H)^- = 622$
$(M+HCOO)^- = 668$

(7) 2-[4-(N-n-Octyloxycarbonylamidino)-phenylaminomethyl]-1-methyl-5-[1-(n-propyloxycarbonylmethylamino)-1-(pyrrolidinocarbonyl)-ethyl]-benzimidazol
Ausbeute: 53 % der Theorie,
$R_f$-Wert: 0.39 (Kieselgel; Essigester/Ethanol = 9:1)
$C_{37}H_{53}N_7O_5$ (675.88)
Massenspektrum: $(M+H)^+ = 676$
$(M+Na)^+ = 698$
$(M-H)^- = 674$

Beispiel 21

(R)-2-(4-Amidinophenylaminomethyl)-1-methyl-5-[1-(carboxymethylamino)-1-(pyrrolidinocarbonyl)-ethyl]-benzimidazol-hydrochlorid

a. 2-Amino-2-(4-chlor-3-nitro-phenyl)-propionsäureethylester

Eine Mischung von 28 g (0.11 Mol) 2-Amino-2-(4-chlor-3-nitrophenyl)-propionsäure in 200 ml 5.6N ethanolischer Salzsäure wird 36 Stunden unter Rückfluß erhitzt. Nach Abdampfen des Lösungsmittels wird der Rückstand in 300 ml Essigester aufgeschlämmt und mit 300 ml gesättigter Natriumhydrogencarbonatlösung versetzt. Die organische Phase wird zweimal mit gesättigter Natriumhydrogencarbonatlösung und einmal mit Wasser gewaschen, über Natriumsulfat getrocknet und eingedampft.
Ausbeute: 21.1 g (68% der Theorie) hellbraunes Öl.

b. (R)-(+)-2-Amino-2-(4-chlor-3-nitro-phenyl)-propionsäureethylester

17.33 g (63.6 mMol) 2-Amino-2-(4-chlor-3-nitro-phenyl)-propionsäureethylester werden in 247 ml Isopropanol und 207 ml Methanol gelöst und mit 9.54 g (63.6 mMol) L-(+)-Weinsäure versetzt. Das Reaktionsgemisch wird auf 100°C erhitzt, wobei eine klare Lösung entsteht. Die Lösung wird innerhalb von 3 Stunden auf 27°C abgekühlt, der gebildete Niederschlag wird abgesaugt, mit Ethanol gewaschen und getrocknet. Anschließend wird der gebildete Feststoff (21.5 g) in 400 ml Essigester aufgeschlämmt und mit 400 ml gesättigter Natriumhydrogencarbonatlösung versetzt. Nach Extraktion und Phasentrennung wird die organische Phase mit Wasser gewaschen, getrocknet und eingedampft.
Ausbeute: 7.68 g (44.4% der Theorie) hellgelben Öl,
$[\alpha]^{20} = +4.38°$ (Essigester)
HPLC-Analyse: ee-Wert >98.6%

c. (R)-(-)-2-Amino-2-(4-chlor-3-nitro-phenyl)-propionsäure

Hergestellt analog Beispiel 4 aus (R)-(+)-2-Amino-2-(4-chlor-3-nitro-phenyl)-propionsäureethylester und Natronlauge in Tetrahydrofuran.
Ausbeute: 63% der Theorie,
$[\alpha]^{20} = -59.6°$ (Methanol/Wasser 1:1)

d. (R)-2-tert.Butyloxycarbonylamino-2-(4-chlor-3-nitrophenyl)-propionsäure

Hergestellt analog Beispiel 5d aus (R)-(-)-2-Amino-2-(4-chlor-3-nitro-phenyl)-propionsäure und Pyrokohlensäure-di-tert.butyldicarbonat und Triethylamin in Dioxan.
Ausbeute: 100% der Theorie,

e. (R)-2-tert.Butyloxycarbonylamino-2-(4-methylamino-3-nitrophenyl)-propionsäure

Hergestellt analog Beispiel 1b aus (R)-2-tert.Butyloxycarbonylamino-2-(4-chlor-3-nitro-phenyl)-propionsäure und Methylamin.
Ausbeute: 69% der Theorie.

f. (R)-2-(4-Methylamino-3-nitro-phenyl)-2-tert.butyloxycarbonylamino-1-pyrrolidino-propanon

Hergestellt analog Beispiel 1c aus (R)-2-tert.Butyloxycarbonylamino-2- (4-methylamino-3-nitro-phenyl)-propionsäure, Pyrrolidin und Carbonyldiimidazol in Tetrahydrofuran.
Ausbeute: 96% der Theorie.

g. (R)-2-(4-Methylamino-3-amino-phenyl)-2-tert.butyloxycarbonylamino-1-pyrrolidino-propanon

Hergestellt analog Beispiel 1c aus (R)-2-(4-Methylamino-3-nitro-phenyl)-2-tert.butyloxycarbonylamino-1-pyrrolidino-propanon und Wasserstoff/Palladium auf Aktivkohle in Methanol. Ausbeute: 99% der Theorie.

h. (R)-2-(4-Cyanophenylaminomethyl)-1-methyl-5-[1-(N-tert.butyloxycarbonylamino)-1-(pyrrolidinocarbonyl)-ethyl]-benzimidazol

Hergestellt analog Beispiel 1c/1f aus (R)-2-(4-Methylamino-3-amino-phenyl)-2-tert.butyloxycarbonylamino-1-pyrrolidinopropanon, 4-Cyanophenylglycin, Carbonyldiimidazol in Tetrahydrofuran und anschließendem Ringschluß in Eisessig.
Ausbeute: 100% der Theorie.

i. (R)-2-(4-Cyanophenylaminomethyl)-1-methyl-5-[1-amino-1-(pyrrolidinocarbonyl)-ethyl]-benzimidazol

Hergestellt analog Beispiel 6i aus (R)-2-(4-Cyanophenylaminomethyl)-1-methyl-5-[1-(N-tert.butyloxycarbonylamino)-1-(pyrrolidinocarbonyl)-ethyl]-benzimidazol und 6N Salzsäure in Dioxan.
Ausbeute: 76% der Theorie.

k. (R)-2-(4-Cyanophenylaminomethyl)-1-methyl-5-[1-(ethoxycarbonylmethylamino)-1-(pyrrolidinocarbonyl)-ethyl]-benzimidazol

Hergestellt analog Beispiel 6k aus (R)-2-(4-Cyanophenylaminomethyl)-1-methyl-5-[1-amino-1-(pyrrolidinocarbonyl)-ethyl]-benzimidazol und Jodessigsäureethylester/Kaliumcarbonat in Aceton.
Ausbeute: 75% der Theorie.

l. (R)-2-(4-Amidinophenylaminomethyl)-1-methyl-5-[1-(ethoxycarbonylmethylamino)-1-(pyrrolidinocarbonyl)-ethyl]-benzimidazol-hydrochlorid

Hergestellt analog Beispiel 1g aus (R)-2-(4-Cyanophenylaminomethyl)-1-methyl-5-[1-(ethoxycarbonylmethylamino)-1-(pyrrolidinocarbonyl)-ethyl]-benzimidazol und Salzsäure/Ammoniumcarbonat in Ethanol.
Ausbeute: 95% der Theorie.

m. (R)-2-(4-Amidinophenylaminomethyl)-1-methyl-5-[1-(carboxymethylamino)-1-(pyrrolidinocarbonyl)-ethyl]-benzimidazolhydrochlorid

Hergestellt analog Beispiel 4 aus (R)-2-(4-Amidinophenylaminomethyl)-1-methyl-5-[1-(ethoxycarbonylmethylamino)-1-(pyrrolidinocarbonyl)-ethyl]-benzimidazol und Natronlauge in Ethanol.
Ausbeute: 100% der Theorie,
$C_{25}H_{31}N_7O_3$ x 2HCl (477.57/550.5)
Massenspektrum: $(M+H)^+$ = 478
$(M-H+HCl)^-$ = 512/514 (Cl)
$(M-H+2HCl)^-$ = 448/550/552 ($Cl_2$)

Beispiel 22

Trockenampulle mit 75 mg Wirkstoff pro 10 ml

Zusammensetzung:

| | |
|---|---|
| Wirkstoff | 75,0 mg |
| Mannitol | 50,0 mg |
| Wasser für Injektionszwecke | ad 10,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 23

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 24

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 9 mm.

Beispiel 25

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |

(fortgesetzt)

| | |
|---|---|
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 12 mm.

Beispiel 26

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

Beispiel 27

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 0 abgefüllt.

Beispiel 28

Suppositorien mit 100 mg Wirkstoff

1 Zäpfchen enthält:

| | |
|---|---|
| Wirkstoff | 100,0 mg |
| Polyethylenglykol (M.G. 1500) | 600,0 mg |
| Polyethylenglykol (M.G. 6000) | 460,0 mg |
| Polyethylensorbitanmonostearat | 840,0 mg |
| | 2 000,0 mg |

Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

**1.** Benzimidazole der allgemeinen Formel

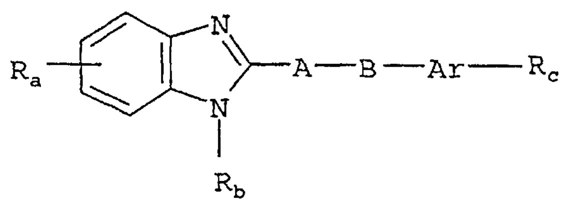

(I),

in der
Ar eine gegebenenfalls durch ein Fluor-, Chlor- oder Bromatom, durch eine Trifluormethyl-, $C_{1-3}$-Alkyl- oder $C_{1-3}$-Alkoxygruppe substituierte Phenylen- oder Naphthylengruppe,
eine gegebenenfalls im Kohlenstoffgerüst durch eine $C_{1-3}$-Alkylgruppe substituierte Thienylen-, Thiazolylen-, Pyridinylen-, Pyrimidinylen-, Pyrazinylen- oder Pyridazinylengruppe,
A eine $C_{1-3}$-Alkylengruppe,
B ein Sauerstoff- oder Schwefelatom, eine Methylen-, Carbonyl-, Sulfinyl- oder Sulfonylgruppe, eine gegebenenfalls durch eine $C_{1-3}$-Alkylgruppe substituierte Iminogruppe, in der der Alkylteil durch eine Carboxygruppe mono- oder disubstituiert sein kann,
$R_a$ eine $R_1$-CO-$C_{3-5}$-cycloalkylgruppe, in der
$R_1$ eine $C_{1-3}$-Alkoxy-, Amino-, $C_{1-4}$-Alkylamino- oder Di-($C_{1-4}$-Alkyl)-aminogruppe, in denen jeweils der Alkylteil durch eine Carboxygruppe substituiert sein kann,
eine 4- bis 7-gliedrige Cycloalkylenimino- oder Cycloalkenyleniminogruppe, die durch eine oder zwei $C_{1-3}$-Alkylgruppen substituiert sein können, wobei ein Alkylsubstituent gleichzeitig durch eine Hydroxy-, $C_{1-3}$-Alkoxy-, Carboxy-, Carboxy-$C_{1-3}$-alkoxy-, Carboxy-$C_{1-3}$-alkylamino-, N-($C_{1-3}$-Alkyl)-N-(carboxy-$C_{1-3}$-alkyl)-amino-, Carboxy-$C_{1-3}$-alkylaminocarbonyl-, N-($C_{1-3}$-Alkyl)-N-(carboxy-$C_{1-3}$-alkyl)-aminocarbonyl-, Carboxy-$C_{1-3}$-alkylaminocarbonylamino-, 1-($C_{1-3}$-Alkyl)-3-(carboxy-$C_{1-3}$-alkyl)-aminocarbonylamino-, 3- ($C_{1-3}$-Alkyl)-3-(carboxy-$C_{1-3}$-alkyl)-aminocarbonylamino- oder 1, 3-Di-($C_{1-3}$-alkyl)-3-(carboxy-$C_{1-3}$-alkyl)-aminocarbonylaminogruppe substituiert sein kann,
eine durch eine Hydroxygruppe substituierte 4- bis 7-gliedrige Cycloalkyleniminogruppe,
eine gegebenenfalls durch eine $C_{1-3}$-Alkylgruppe substituierte 5- bis 7-gliedrige Cycloalkyleniminogruppe, an die über zwei benachbarte Kohlenstoffatome ein Phenylring ankondensiert ist,
eine Morpholino-, Piperazino-, N-($C_{1-3}$-Alkyl)-piperazino-, Pyrrolino-, 3,4-Dehydro-piperidino- oder Pyrrol-1-yl-Gruppe darstellt,
eine $R_2$-CX-$C_{3-5}$-cycloalkylgruppe, in der

$R_2$ eine gegebenenfalls durch eine $C_{1-3}$-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine $C_{1-3}$-Alkylgruppe substituierte Iminogruppe, ein Sauerstoff- oder Schwefelatom oder eine gegebenenfalls durch eine $C_{1-3}$-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-$C_{1-3}$-alkoxy-, Carboxy-$C_{1-3}$-alkylamino- oder N-($C_{1-3}$-Alkyl)-carboxy-$C_{1-3}$-alkylaminogruppe substituiert sein kann, und

X ein Sauerstoffatom, eine $C_{1-3}$-Alkylimino-, $C_{1-3}$-Alkoxyimino-, $C_{1-3}$-Alkylhydrazino-, Di-($C_{1-3}$-Alkyl)-hydrazino-, $C_{2-4}$-Alkanoylhydrazino-, N-($C_{1-3}$-Alkyl)-$C_{2-4}$-alkanoylhydrazino- oder $C_{1-3}$-Alkylidengruppe, die jeweils im Alkyl- oder Alkanoylteil oder im Alkyl- und Alkanoylteil durch eine Carboxygruppe substituiert sein können, darstellen,

eine durch eine Imidazol- oder Imidazolongruppe substituierte $C_{1-3}$-Alkyl- oder $C_{3-5}$-Cycloalkylgruppe, in denen

der Imidazolring durch eine Phenyl- oder Carboxygruppe und durch eine oder zwei $C_{1-3}$-Alkylgruppen oder durch eine, zwei oder drei $C_{1-3}$-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl)-$C_{1-3}$-alkylamino- oder Di-($C_{1-3}$-Alkyl)-aminogruppe substituiert sein kann, und

der Imidazolonring durch eine $C_{1-3}$-Alkylgruppe substituiert sein kann, wobei der Alkylsubstituent durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl)-$C_{1-3}$-alkylamino- oder Di-($C_{1-3}$-Alkyl)-aminogruppe substituiert sein kann, und

zusätzlich an die vorstehend erwähnten Imidazol- und Imidazolonringe über zwei benachbarte Kohlenstoffatome ein Phenyl- oder Pyridinring ankondensiert sein kann,

eine Imidazolidin-2,4-dion-5-yl-Gruppe, die durch eine oder zwei $C_{1-3}$-Alkylgruppen substituiert sein kann, wobei gleichzeitig ein Alkylsubstituent durch eine Carboxygruppe substituiert sein kann,

eine $C_{1-4}$-Alkylgruppe, die

durch eine $C_{1-3}$-Alkyl-$Y_1$-$C_{1-3}$-alkyl-, HOOC-$C_{1-3}$-alkyl-$Y_1$-$C_{1-3}$-alkyl-, Tetrazolyl-$C_{1-3}$-alkyl-$Y_2$-, $R_3NR_4$- oder $R_3NR_4$-$C_{1-3}$-alkyl-Gruppe und

durch eine gegebenenfalls durch eine $C_{1-3}$-Alkylgruppe substituierte Isoxazolidinylcarbonylgruppe, durch eine Pyrrolinocarbonyl-, 3,4-Dehydro-piperidinocarbonyl-, Pyrrol-1-yl-carbonyl-, Carboxy-, Aminocarbonyl-, $C_{1-3}$-Alkylaminocarbonyl-, Di-($C_{1-3}$-Alkyl)-aminocarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei $C_{1-3}$-Alkylgruppen substituiert und gleichzeitig jeweils ein Alkylteil oder Alkylsubstituent der vorstehend erwähnten $C_{1-3}$-Alkylaminocarbonyl-, Di-($C_{1-3}$-Alkyl)-aminocarbonyl- oder Cycloalkyleniminocarbonylgruppen durch eine Carboxygruppe substituiert sein kann, und die verbleibenden Wasserstoffatome der $C_{1-4}$-Alkylgruppe ganz oder teilweise durch Fluoratome ersetzt sein können, in denen

$R_3$ ein Wasserstoffatom oder eine gegebenenfalls durch eine Carboxygruppe substituierte $C_{1-3}$-Alkylgruppe und

$R_4$ ein Wasserstoffatom, eine $C_{1-3}$-Alkyl-$Y_1$-$C_{1-3}$-alkyl-$Y_2$-, Carboxy-$C_{1-3}$-alkyl-$Y_1$-$C_{1-3}$-alkyl-$Y_2$-, $C_{1-3}$-Alkyl-$Y_2$- oder Carboxy-$C_{1-3}$-alkyl-$Y_2$-Gruppe oder

$R_3$ und $R_4$ zusammen mit dem dazwischen liegenden Stickstoffatom eine gegebenenfalls durch eine Carboxy-, $C_{1-3}$-Alkyl- oder Carboxy-$C_{1-3}$-alkylgruppe substituierte 4- bis 7-gliedrige Cycloalkyleniminogruppe darstellen, in denen

$Y_1$ eine Kohlenstoff-Kohlenstoff-Bindung, ein Sauerstoffatom, eine Sulfenyl-, Sulfinyl-, Sulfonyl-, -NH-, -NH-CO- oder -NH-CO-NH-Gruppe und

$Y_2$ eine Kohlenstoff-Stickstoffbindung oder eine Carbonyl-, Sulfonyl-, Imino- oder -NH-CO-Gruppe darstellen, wobei die Carbonylgruppe der -NH-CO-Gruppe mit dem Stickstoffatom der $R_3NR_4$-Gruppe verknüpft ist, und die bei der Definition der Reste $Y_1$ und $Y_2$ vorkommenden Iminogruppen jeweils zusätzlich durch eine $C_{1-3}$-Alkyl- oder Carboxy-$C_{1-3}$-alkylgruppe substituiert sein können,

eine durch eine $R_5NR_6$-Gruppe substituierte $C_{1-3}$-Alkyl- oder $C_{3-5}$-Cycloalkylgruppe, in denen

$R_5$ ein Wasserstoffatom, eine $C_{1-3}$-Alkyl-, $C_{5-7}$-Cycloalkyl-, Phenylcarbonyl-, Phenylsulfonyl- oder Pyridinylgruppe und

$R_6$ eine $C_{1-3}$-Alkyl-, Carboxy-$C_{1-3}$-alkyl- oder Carboxy-$C_{1-3}$-alkylcarbonylgruppe darstellen,

eine $C_{1-3}$-Alkylgruppe, die durch $C_{2-4}$-Alkanoyl- oder $C_{5-7}$-Cycloalkanoylgruppe und durch eine durch ein Chlor-, Brom- oder Jodatom substituierte $C_{1-3}$-Alkylgruppe substituiert, ist,

$R_b$ ein Wasserstoffatom oder eine $C_{1-3}$-Alkylgruppe und

$R_c$ eine Cyanogruppe oder eine gegebenenfalls durch eine oder zwei $C_{1-3}$-Alkylgruppen substituierte Amidinogruppe bedeuten, worin

die bei der Definition der vorstehend erwähnten Reste erwähnten Carboxygruppen außerdem durch eine in-vivo in eine Carboxygruppe überführbare Gruppe oder durch eine unter physiologischen Bedingungen negativ geladene Gruppe ersetzt sein können oder

die bei der Definition der vorstehend erwähnten Reste erwähnten Amino- und Iminogruppen außerdem durch einen in vivo abspaltbaren Rest substituiert sein können, wobei

unter einer in-vivo in eine Carboxygruppe überführbaren Gruppe eine Hydroxmethylgruppe, eine mit einem Alkohol veresterte Carboxygruppe, in der der alkoholische Teil ein $C_{1-6}$-Alkanol, ein Phenyl-$C_{1-3}$-alkanol, ein $C_{3-9}$-Cycloalkanol, wobei ein $C_{5-8}$-Cycloalkanol zusätzlich durch ein oder zwei $C_{1-3}$-Alkylgruppen substituiert sein kann, ein $C_{5-8}$-Cycloalkanol, in dem eine Methylengruppe in 3- oder 4-Stellung durch ein Sauerstoffatom oder durch eine gegebenenfalls durch eine $C_{1-3}$-Alkyl-, Phenyl-$C_{1-3}$-alkyl-, Phenyl-$C_{1-3}$-alkoxycarbonyl- oder $C_{2-6}$-Alkanoylgruppe substituierte Iminogruppe ersetzt ist und der Cycloalkanolteil zusätzlich durch ein oder zwei $C_{1-3}$-Alkylgruppen substituiert sein kann, ein $C_{4-7}$-Cycloalkenol, ein $C_{3-5}$-Alkenol, ein Phenyl-$C_{3-5}$-alkenol, ein $C_{3-5}$-Alkinol oder Phenyl- $C_{3-5}$-alkinol mit der Maßgabe, daß keine Bindung an das Sauerstoffatom von einem Kohlenstoffatom ausgeht, welches eine Doppel- oder Dreifachbindung trägt, ein $C_{3-8}$-Cycloalkyl-$C_{1-3}$-alkanol, ein Bicycloalkanol mit insgesamt 8 bis 10 Kohlenstoffatomen, das im Bicycloalkylteil zusätzlich durch eine oder zwei $C_{1-3}$-Alkylgruppen substituiert sein kann, ein 1,3-Dihydro-3-oxo-1-isobenzfuranol oder ein Alkohol der Formel

$$R_d\text{-CO-O-}(R_eCR_f)\text{-OH},$$

verstanden wird, in dem

$R_d$ eine $C_{1-8}$-Alkyl-, $C_{5-7}$-Cycloalkyl-, Phenyl- oder Phenyl-$C_{1-3}$-alkylgruppe,

$R_e$ ein Wasserstoffatom, eine $C_{1-3}$-Alkyl-, $C_{5-7}$-Cycloalkyl- oder Phenylgruppe und

$R_f$ ein Wasserstoffatom oder eine $C_{1-3}$-Alkylgruppe darstellen,

unter einer unter physiologischen Bedingungen negativ geladenen Gruppe eine Tetrazol-5-yl-, Phenylcarbonylaminocarbonyl-, Trifluormethylcarbonylaminocarbonyl-, $C_{1-6}$-Alkylsulfonylamino-, Phenylsulfonylamino-, Benzylsulfonylamino-, Trifluormethylsulfonylamino-, $C_{1-6}$-Alkylsulfonylaminocarbonyl-, Phenylsulfonylaminocarbonyl-, Benzylsulfonylaminocarbonyl- oder Perfluor-$C_{1-6}$-alkylsulfonylaminocarbonylgruppe

und unter einem von einer Imino- oder Aminogruppe in-vivo abspaltbaren Rest eine Hydroxygruppe, eine gegebenenfalls durch Fluor-, Chlor-, Brom- oder Jodatome, durch $C_{1-3}$-Alkyl- oder $C_{1-3}$-Alkoxygruppen mono- oder disubstituierte Benzoylgruppe, wobei die Substituenten gleich oder verschieden sein können, eine Pyridinoylgruppe oder eine $C_{1-16}$-Alkanoylgruppe, eine 3,3,3-Trichlorpropionyl- oder Allyloxycarbonylgruppe, eine $C_{1-16}$-Alkoxycarbonyl- oder $C_{1-16}$-Alkylcarbonyloxygruppe, in denen Wasserstoffatome ganz oder teilweise durch Fluor- oder Chloratome ersetzt sein können, eine Phenyl-$C_{1-6}$-alkoxycarbonylgruppe, eine 3-Aminopropionylgruppe, in der die Aminogruppe durch $C_{1-6}$-Alkyl- oder $C_{3-7}$-Cycloalkylgruppen mono- oder disubstituiert und die Substituenten gleich oder verschieden sein können, eine $C_{1-3}$-Alkylsulfonyl-$C_{2-4}$-alkoxycarbonyl-, $C_{1-3}$-Alkoxy-$C_{2-4}$-alkoxy-$C_{2-4}$-alkoxycarbonyl-, $R_d$-CO-O-($R_dCR_f$)-O-CO-, $C_{1-6}$-Alkyl-CO-NH-($R_gCR_h$)-O-CO- oder $C_{1-6}$-Alkyl-CO-O-($R_gCR_h$)-($R_gCR_h$)-O-CO-Gruppe, in denen $R_d$ bis $R_f$ wie vorstehend erwähnt definiert sind und

$R_g$ und $R_h$, die gleich oder verschieden sein können, Wasserstoffatome oder $C_{1-3}$-Alkylgruppen darstellen, verstanden werden,

deren Tautomere, deren Stereoisomere, deren Gemische und deren Salze.

**2.** Benzimidazole der allgemeinen Formel

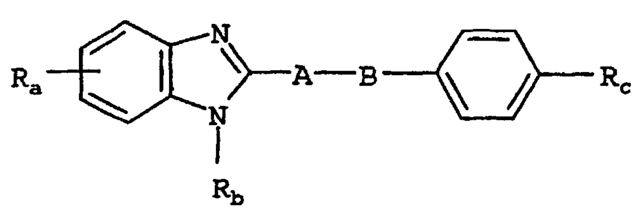

(Ia),

in der

A eine $C_{1-3}$-Alkylengruppe,

B ein Sauerstoff- oder Schwefelatom, eine Methylen-, Carbonyl-, Sulfinyl- oder Sulfonylgruppe, eine gegebenenfalls durch eine $C_{1-3}$-Alkylgruppe substituierte Iminogruppe, in der der Alkylteil durch eine Carboxygruppe mono- oder disubstituiert sein kann,

$R_a$ eine $R_1$-CO-$C_{3-5}$-cycloalkylgruppe, in der

$R_1$ eine $C_{1-3}$-Alkoxy-, Amino-, $C_{1-4}$-Alkylamino- oder Di-($C_{1-4}$-Alkyl)-aminogruppe, in denen jeweils der Alkylteil durch eine Carboxygruppe substituiert sein kann,

eine 4- bis 7-gliedrige Cycloalkylenimino- oder Cycloalkenylenyiminogruppe, die durch eine oder zwei $C_{1-3}$-Alkylgruppe substituiert sein können, wobei ein Alkylsubstituent gleichzeitig durch eine Hydroxy-, $C_{1-3}$-Alkoxy-, Carboxy-, Carboxy-$C_{1-3}$-alkoxy-, Carboxy-$C_{1-3}$-alkylamino-, N-($C_{1-3}$-Alkyl)-N-(carboxy-$C_{1-3}$-alkyl)-amino-, Carboxy-$C_{1-3}$-alkylaminocarbonyl-, N-($C_{1-3}$-Alkyl)-N-(carboxy-$C_{1-3}$-alkyl)-aminocarbonyl-, Carboxy-$C_{1-3}$-alkylaminocarbonylamino-, 1-($C_{1-3}$-Alkyl)-3-(carboxy-$C_{1-3}$-alkyl)-aminocarbonylamino-, 3-($C_{1-3}$-Alkyl)-3-(carboxy-$C_{1-3}$-alkyl)-aminocarbonylamino- oder 1,3-Di-($C_{1-3}$-alkyl)-3-(carboxy-$C_{1-3}$-alkyl)-aminocarbonylaminogruppe substituiert sein kann,

eine durch eine Hydroxygruppe substituierte 4- bis 7-gliedrige Cycloalkyleniminogruppe,

eine gegebenenfalls durch eine $C_{1-3}$-Alkylgruppe substituierte 5- bis 7-gliedrige Cycloalkyleniminogruppe, an die über zwei benachbarte Kohlenstoffatome ein Phenylring ankondensiert ist,

eine Morpholino-, Piperazino-, N-($C_{1-3}$-Alkyl)-piperazino-, Pyrrolino-, 3,4-Dehydro-piperidino- oder Pyrrol-1-yl-Gruppe darstellt,

eine $R_2$-CX-$C_{3-5}$-cycloalkylgruppe, in der

$R_2$ eine gegebenenfalls durch eine $C_{1-3}$-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine $C_{1-3}$-Alkylgruppe substituierte Iminogruppe, ein Sauerstoff- oder Schwefelatom oder eine gegebenenfalls durch eine $C_{1-3}$-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-$C_{1-3}$-alkoxy-, Carboxy-$C_{1-3}$-alkylamino- oder N-($C_{1-3}$-Alkyl)-carboxy-$C_{1-3}$-alkylaminogruppe substituiert sein kann, und

X ein Sauerstoffatom, eine $C_{1-3}$-Alkylimino-, $C_{1-3}$-Alkoxyimino-, $C_{1-3}$-Alkylhydrazino-, Di-($C_{1-3}$-Alkyl)-hydrazino-, $C_{2-4}$-Alkanoylhydrazino-, N-($C_{1-3}$-Alkyl)-$C_{2-4}$-alkanoylhydrazino- oder $C_{1-3}$-Alkylidengruppe, die jeweils im Alkyl- oder Alkanoylteil oder im Alkyl- und Alkanoylteil durch eine Carboxygruppe substituiert sein können, darstellen,

eine durch eine Imidazol- oder Imidazolongruppe substituierte $C_{1-3}$-Alkyl- oder $C_{3-5}$-Cycloalkylgruppe, in denen

der Imidazolring durch eine Phenyl- oder Carboxygruppe und durch eine oder zwei $C_{1-3}$-Alkylgruppen oder durch eine, zwei oder drei $C_{1-3}$-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl)-$C_{1-3}$-alkylamino- oder Di-($C_{1-3}$-Alkyl)-aminogruppe substituiert sein kann, und

der Imidazolonring durch eine $C_{1-3}$-Alkylgruppe substituiert sein kann, wobei der Alkylsubstituent durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl)-$C_{1-3}$-alkylamino- oder Di-($C_{1-3}$-Alkyl)-aminogruppe substituiert sein kann, und

zusätzlich an die vorstehend erwähnten Imidazol- und Imidazolonringe über zwei benachbarte Kohlenstoffatome ein Phenyl- oder Pyridinring ankondensiert sein kann,

eine Imidazolidin-2,4-dion-5-yl-Gruppe, die durch eine oder zwei $C_{1-3}$-Alkylgruppen substituiert sein kann, wobei gleichzeitig ein Alkylsubstituent durch eine Carboxygruppe substituiert sein kann,

eine $C_{1-4}$-Alkylgruppe, die

durch eine $C_{1-3}$-Alkyl-$Y_1$-$C_{1-3}$-alkyl-, HOOC-$C_{1-3}$-alkyl-$Y_1$-$C_{1-3}$-alkyl-, Tetrazolyl-$C_{1-3}$-alkyl-$Y_2$-, $R_3NR_4$- oder $R_3NR_4$-$C_{1-3}$-alkyl-Gruppe und

durch eine gegebenenfalls durch eine $C_{1-3}$-Alkylgruppe substituierte Isoxazolidin-1-ylcarbonylgruppe, durch eine Pyrrolinocarbonyl-, 2,3-Dehydro-piperidinocarbonyl-, Pyrrol-1-yl-carbonyl-, Carboxy-, Aminocarbonyl-, $C_{1-3}$-Alkylaminocarbonyl-, Di-($C_{1-3}$-Alkyl)-aminocarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei $C_{1-3}$-Alkylgruppen substituiert und gleichzeitig jeweils ein Alkylteil oder Alkylsubstituent der vorstehend erwähnten $C_{1-3}$-Alkylaminocarbonyl-, Di-($C_{1-3}$-Alkyl)-aminocarbonyl- oder Cycloalkyleniminocarbonylgruppen durch eine Carboxygruppe substituiert sein kann, und die verbleibenden Wasserstoffatome der $C_{1-4}$-Alkylgruppe ganz oder teilweise durch Fluoratome ersetzt sein können, in denen

$R_3$ ein Wasserstoffatom oder eine gegebenenfalls durch eine Carboxygruppe substituierte $C_{1-3}$-Alkylgruppe und

$R_4$ ein Wasserstoffatom, eine $C_{1-3}$-Alkyl-$Y_1$-$C_{1-3}$-alkyl-$Y_2$-, Carboxy-$C_{1-3}$-alkyl-$Y_1$-$C_{1-3}$-alkyl-$Y_2$-, $C_{1-3}$-Alkyl-$Y_2$- oder Carboxy-$C_{1-3}$-alkyl-$Y_2$-Gruppe oder

$R_3$ und $R_4$ zusammen mit dem dazwischen liegenden Stickstoffatom eine gegebenenfalls durch eine Carboxy-, $C_{1-3}$-Alkyl- oder Carboxy-$C_{1-3}$-alkylgruppe substituierte 4- bis 7-gliedrige Cycloalkyleniminogruppe darstellen, in denen

$Y_1$ eine Kohlenstoff-Kohlenstoff-Bindung, ein Sauerstoffatom, eine Sulfenyl-, Sulfinyl-, Sulfonyl-, -NH-,

-NH-CO- oder -NH-CO-NH-Gruppe und

$Y_2$ eine Kohlenstoff-Stickstoffbindung oder eine Carbonyl-, Sulfonyl-, Imino- oder -NH-CO-Gruppe darstellen, wobei die Carbonylgruppe der -NH-CO-Gruppe mit dem Stickstoffatom der $R_3NR_4$-Gruppe verknüpft ist, und die bei der Definition der Reste $Y_1$ und $Y_2$ vorkommenden Iminogruppen jeweils zusätzlich durch eine $C_{1-3}$-Alkyl- oder Carboxy-$C_{1-3}$-alkylgruppe substituiert sein können,

eine durch eine $R_5NR_6$-Gruppe substituierte $C_{1-3}$-Alkyl- oder $C_{3-5}$-Cycloalkylgruppe, in denen

$R_5$ ein Wasserstoffatom, eine $C_{1-3}$-Alkyl-, $C_{5-7}$-Cycloalkyl-, Phenylcarbonyl-, Phenylsulfonyl- oder Pyridinylgruppe und

$R_6$ eine $C_{1-3}$-Alkyl-, Carboxy-$C_{1-3}$-alkyl- oder Carboxy-$C_{1-3}$-alkylcarbonylgruppe darstellen,

eine $C_{1-3}$-Alkylgruppe, die durch $C_{2-4}$-Alkanoyl- oder $C_{5-7}$-Cycloalkanoylgruppe und durch eine durch ein Chlor-, Brom- oder Jodatom substituierte $C_{1-3}$-Alkylgruppe substituiert ist,

$R_b$ ein Wasserstoffatom oder eine $C_{1-3}$-Alkylgruppe und

$R_c$ eine Cyanogruppe oder eine Amidinogruppe, die durch eine Hydroxygruppe, durch eine oder zwei $C_{1-3}$-Alkylgruppen, durch eine oder zwei $C_{1-8}$-Alkoxycarbonylgruppen substituiert sein kann, bedeuten,

wobei die bei der Definition der vorstehend erwähnten Reste erwähnten Carboxy-, Amino- und Iminogruppen außerdem durch einen wie in Anspruch 1 definierten in vivo abspaltbaren Rest substituiert sein können,

deren Tautomere, deren Stereoisomere und deren Salze.

**3.** Benzimidazole der. allgemeinen Formel Ia gemäß Anspruch 2, in der

A eine $C_{1-3}$-Alkylengruppe,

B ein Sauerstoffatom, eine Methylen-, Imino- oder N-($C_{1-3}$-Alkyl)-iminogruppe, in der der Alkylteil durch eine Carboxygruppe substituiert sein kann,

$R_a$ eine in 1-Stellung durch den $R_1$-CO-Rest substituierte $C_{3-5}$-Cycloalkylgruppe, in der

$R_1$ eine $C_{1-3}$-Alkoxy-, Amino-, $C_{1-4}$-Alkylamino- oder Di-($C_{1-4}$-Alkyl)-aminogruppe, in denen jeweils der Alkylteil durch eine Carboxygruppe substituiert sein kann,

eine 4- bis 7-gliedrige Cycloalkyleniminogruppe, die durch eine Hydroxygruppe oder durch eine oder zwei $C_{1-3}$-Alkylgruppe substituiert sein kann, wobei ein Alkylsubstituent gleichzeitig durch eine Hydroxy-, $C_{1-3}$-Alkoxy-, Carboxy-, Carboxy-$C_{1-3}$-alkoxy-, Carboxy-$C_{1-3}$-alkylamino-, N-($C_{1-3}$-Alkyl)-N-(carb oxy-$C_{1-3}$-alkyl)-amino-, Carboxy-$C_{1-3}$-alkylaminocarbonyl-, N-($C_{1-3}$-Alkyl)-N-(carboxy-$C_{1-3}$-alkyl)-aminocarbonyl-, Carboxy-$C_{1-3}$-alkylaminocarbonylamino-, 1-($C_{1-3}$-Alkyl)-3-(carboxy-$C_{1-3}$-alkyl)-aminocarbonylamino-, 3-($C_{1-3}$-Alkyl)-3-(carboxy-$C_{1-3}$-alkyl)-aminocarbonylamino- oder 1,3-Di-($C_{1-3}$-alkyl)-3-(carboxy-$C_{1-3}$-alkyl)-aminocarbonylaminogruppe substituiert sein kann,

eine gegebenenfalls durch eine $C_{1-3}$-Alkylgruppe substituierte 5- bis 7-gliedrige Cycloalkyleniminogruppe, an die über zwei benachbarte Kohlenstoffatome ein Phenylring ankondensiert ist,

eine Morpholino-, Piperazino-, N-($C_{1-3}$-Alkyl)-piperazino-, Pyrrolino-, 3,4-Dehydro-piperidino- oder Pyrrol-1-yl-Gruppe darstellt,

eine in 1-Stellung durch den $R_2$-CX-Rest substituierte $C_{3-5}$-Cycloalkylgruppe, in der

$R_2$ eine gegebenenfalls durch eine $C_{1-3}$-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine $C_{1-3}$-Alkylgruppe substituierte Iminogruppe, ein Sauerstoff- oder Schwefelatom oder eine gegebenenfalls durch eine $C_{1-3}$-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-$C_{1-3}$-alkoxy-, Carboxy-$C_{1-3}$-alkylamino- oder N-($C_{1-3}$-Alkyl)-carboxy-$C_{1-3}$-alkylaminogruppe substituiert sein kann, und

X ein Sauerstoffatom, eine $C_{1-3}$-Alkylimino-, $C_{1-3}$-Alkoxyiminooder $C_{1-3}$-Alkylidengruppe, die jeweils im Alkyl- oder Alkoxyteil durch eine Carboxygruppe substituiert sein können, darstellen,

eine in 1-Stellung durch eine Imidazol- oder Imidazolongruppe substituierte $C_{1-3}$-Alkylgruppe, in denen

der Imidazolring durch eine Phenyl- oder Carboxygruppe und durch eine oder zwei $C_{1-3}$-Alkylgruppen oder durch eine, zwei oder drei $C_{1-3}$-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl)-$C_{1-3}$-alkylamino- oder Di-($C_{1-3}$-Alkyl)-aminogruppe substituiert sein kann, und

der Imidazolonring durch eine $C_{1-3}$-Alkylgruppe substituiert sein kann, wobei der Alkylsubstituent durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl)-$C_{1-3}$-alkylamino- oder Di-($C_{1-3}$-Alkyl)-aminogruppe substituiert sein kann, und

zusätzlich an die vorstehend erwähnten Imidazol- und Imidazolonringe über zwei benachbarte Kohlenstoffatome ein Phenyl- oder Pyridinring ankondensiert sein kann,

eine Imidazolidin-2,4-dion-5-yl-Gruppe, die durch eine oder zwei $C_{1-3}$-Alkylgruppen substituiert sein kann, wobei

gleichzeitig ein Alkylsubstituent durch eine Carboxygruppe substituiert sein kann,
eine $C_{1-4}$-Alkylgruppe, die in 1-Stellung
durch eine $R_3NR_4$- oder $R_3NR_4$-$C_{1-3}$-alkyl-Gruppe und
durch eine Pyrrolinocarbonyl-, 2,3-Dehydro-piperidinocarbonyl-, Imidazol-1-yl-carbonyl-, Carboxy-, Aminocarbonyl-, $C_{1-3}$-Alkylaminocarbonyl-, Di- ($C_{1-3}$-Alkyl) -aminocarbonyl-, Isoxazolidin-1-ylcarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei $C_{1-3}$-Alkylgruppen substituiert und gleichzeitig jeweils ein Alkylteil oder Alkylsubstituent der vorstehend erwähnten $C_{1-3}$-Alkylaminocarbonyl-, Di-($C_{1-3}$-Alkyl)-aminocarbonyl- oder Cycloalkyleniminocarbonylgruppen durch eine Carboxygruppe substituiert sein kann, und die verbleibenden Wasserstoffatome der $C_{1-4}$-Alkylgruppe ganz oder teilweise durch Fluoratome ersetzt sein können, in denen
$R_3$ ein Wasserstoffatom oder eine gegebenenfalls durch eine Carboxygruppe substituierte $C_{1-3}$-Alkylgruppe und
$R_4$ ein Wasserstoffatom, $C_{1-3}$-Alkyl-$Y_2$- oder Carboxy-$C_{1-3}$-alkyl-$Y_2$-Gruppe oder
$R_3$ und $R_4$ zusammen mit dem dazwischen liegenden Stickstoffatom eine gegebenenfalls in 1-Stellung durch eine Carboxy-, $C_{1-3}$-Alkyl- oder Carboxy-$C_{1-3}$-alkylgruppe substituierte 4-bis 7-gliedrige Cycloalkyleniminogruppe darstellen, in denen
$Y_2$ eine Kohlenstoff-Stickstoffbindung oder eine Carbonyl-, Imino- oder -NH-CO-Gruppe darstellt, wobei die Carbonylgruppe der -NH-CO-Gruppe mit dem Stickstoffatom der $R_3NR_4$-Gruppe verknüpft ist, und die bei der Definition des Restes $Y_2$ vorkommende Iminogruppe zusätzlich durch eine $C_{1-3}$-Alkyl- oder Carboxy-$C_{1-3}$-alkylgruppe substituiert sein können,
eine in 1-Stellung durch eine $R_5NR_6$-Gruppe substituierte $C_{1-3}$-Alkyl- oder $C_{3-5}$-Cycloalkylgruppe, in denen
$R_5$ ein Wasserstoffatom, eine $C_{1-3}$-Alkyl-, $C_{5-7}$-Cycloalkyl-, Phenylcarbonyl-, Phenylsulfonyl- oder Pyridinylgruppe und
$R_6$ eine $C_{1-3}$-Alkyl-, Carboxy-$C_{1-3}$-alkyl- oder Carboxy-$C_{1-3}$-alkylcarbonylgruppe darstellen,
eine $C_{1-3}$-Alkylgruppe, die durch $C_{2-4}$-Alkanoyl- oder $C_{5-7}$-Cycloalkanoylgruppe und durch eine durch ein Chlor-, Brom- oder Jodatom substituierte $C_{1-3}$-Alkylgruppe substituiert ist,
$R_b$ eine $C_{1-3}$-Alkylgruppe und
$R_c$ eine gegebenenfalls durch eine 2,2,2-Trichlorethoxycarbonyl-, $C_{1-8}$-Alkoxycarbonyl-, Acetoxymethyloxycarbonyl-, Benzyloxycarbonyl- oder Benzoylgruppe substituierte Amidinogruppe bedeuten, wobei der Benzoylteil durch Fluor-, Chlor-, Brom- oder Jodatome, durch $C_{1-3}$-Alkyl- oder $C_{1-3}$-Alkoxygruppen mono- oder disubstituiert und die Substituenten gleich oder verschieden sein können,
deren $C_{1-3}$-Alkanolester, deren Tautomere, deren Stereoisomere und deren Salze.

**4.** Benzimidazole der allgemeinen Formel Ia gemäß Anspruch 2, in der
A eine Methylengruppe,
B ein Sauerstoffatom oder eine Iminogruppe,
$R_a$ eine in 1-Stellung durch den $R_1$-CO-Rest substituierte Cyclopropylgruppe, in der
$R_1$ eine gegebenenfalls durch eine Methyl- oder Ethylgruppe substituierte Pyrrolidino- oder Piperidinogruppe, in denen jeweils der Methyl- oder Ethylteil durch eine Carboxy-, Carboxy-$C_{1-3}$-alkoxy-, Carboxy-$C_{1-3}$-alkylamino- oder N-($C_{1-3}$-Alkyl)-carboxy-$C_{1-3}$-alkylaminogruppe substituiert sein kann, darstellt,
eine in 1-Stellung durch den $R_2$-CX-Rest substituierte Cyclopropylgruppe, in der
$R_2$ eine gegebenenfalls durch eine $C_{1-3}$-Alkylgruppe substituierte Phenyl-, Pyridyl- Pyrazolylgruppe und
X ein Sauerstoffatom, eine $C_{1-3}$-Alkoxyimino- oder $C_{1-3}$-Alkylidengruppe, die jeweils im Alkyl- oder Alkoxyteil durch eine Carboxygruppe substituiert sind, darstellen,
eine in 1-Stellung durch eine Imidazolgruppe substituierte $C_{1-2}$-Alkylgruppe, in der der Imidazolring durch eine Phenyl- oder Carboxygruppe und durch eine oder zwei $C_{1-3}$-Alkylgruppen oder durch eine, zwei oder drei $C_{1-3}$-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, $C_{2-4}$-Alkanoylamino-, $C_{1-3}$-Alkylamino-, N-($C_{2-4}$-Alkanoyl)-$C_{1-3}$-alkylamino- oder Di-($C_{1-3}$-Alkyl)-aminogruppe substituiert sein kann, wobei zusätzlich an die vorstehend erwähnten Imidazolringe über zwei benachbarte Kohlenstoffatome ein Phenyl- oder Pyridinring ankondensiert sein kann,
eine in 1-Stellung durch eine Benzimidazolon-1-yl-Gruppe substituierte $C_{1-2}$-Alkylgruppe, wobei der Imidazolonring durch eine gegebenenfalls durch eine Carboxygruppe substituierte Methyl- oder Ethylgruppe substituiert sein kann, darstellen,
eine Methyl- oder Ethylgruppe, die in 1-Stellung
durch eine $R_3NR_4$- oder $R_3NR_4$-$C_{1-3}$-alkyl-Gruppe und
durch eine Di-($C_{1-3}$-Alkyl)-aminocarbonylgruppe, durch eine Isoxazolidin-1-ylcarbonylgruppe, durch eine gegebenenfalls durch eine $C_{1-3}$-Alkylgruppe substituierte Pyrrolidinocarbonyl- oder Piperidinocarbonylgruppe, wobei

bei den vorstehend erwähnten Gruppen jeweils ein Alkylteil oder Alkylsubstituent durch eine Carboxygruppe substituiert sein kann, in denen

$R_3$ ein Wasserstoffatom oder eine gegebenenfalls durch eine Carboxygruppe substituierte $C_{1-3}$-Alkylgruppe und

$R_4$ ein Wasserstoffatom, eine $C_{1-3}$-Alkyl-$Y_2$- oder Carboxy-$C_{1-3}$-alkyl-$Y_2$-Gruppe oder

$R_3$ und $R_4$ zusammen mit dem dazwischen liegenden Stickstoffatom eine gegebenenfalls durch eine Carboxygruppe substituierte 4- bis 7-gliedrige Cycloalkyleniminogruppe darstellen, in denen

$Y_2$ eine Kohlenstoff-Stickstoffbindung, eine Carbonylgruppe oder eine gegebenenfalls durch eine $C_{1-3}$-Alkylgruppe substituierte Iminogruppe darstellt,

eine in 1-Stellung durch eine $R_5NR_6$-Gruppe substituierte $C_{1-2}$-Alkylgruppe, in der

$R_5$ eine Pyridinyl-, Phenylcarbonyl- oder Phenylsulfonylgruppe und

$R_6$ eine $C_{1-3}$-Alkyl- oder Carboxy-$C_{1-3}$-alkylgruppe darstellen,

eine durch eine in 3-Stellung durch ein Chloratom substituierte n-Propylgruppe, die in 1-Stellung durch eine Cyclopentylcarbonylgruppe substituiert ist,

eine in 1-Stellung durch eine Cyclopentylaminogruppe substituierte Cyclopropylgruppe, die am Stickstoffatom durch eine Carboxy-$C_{1-3}$-alkylcarbonylgruppe substituiert ist,

$R_b$ eine Methylgruppe und

$R_c$ eine gegebenenfalls durch eine $C_{1-8}$-Alkoxycarbonyl-, Acetoxymethyloxycarbonyl-, 2,2,2-Trichlorethoxycarbonyl-, Benzyloxycarbonyl- oder Benzoylgruppe substituierte Amidinogruppe bedeuten,

deren $C_{1-3}$-Alkanolester, deren Tautomere, deren Stereoisomere und deren Salze.

**5.** Benzimidazole der allgemeinen Formel Ia gemäß Anspruch 2, in der

A eine Methylengruppe,

B eine Iminogruppe,

$R_a$ eine in 1-Stellung durch den $R_1$-CO-Rest substituierte Cyclopropylgruppe, in der

$R_1$ eine gegebenenfalls durch eine Methyl- oder Ethylgruppe substituierte Pyrrolidino- oder Piperidinogruppe, in denen jeweils der Methyl- oder Ethylteil durch eine Carboxy-, Carboxy-$C_{1-3}$-alkoxy-, Carboxy-$C_{1-3}$-alkylamino- oder N-($C_{1-3}$-Alkyl)-carboxy-$C_{1-3}$-alkylaminogruppe substituiert sein kann, darstellt,

eine in 1-Stellung durch den $R_2$-CX-Rest substituierte Cyclopropylgruppe, in der

$R_2$ eine gegebenenfalls durch eine $C_{1-3}$-Alkylgruppe substituierte Phenyl-, Pyridyl- Pyrazolylgruppe und

X ein Sauerstoffatom, eine $C_{1-3}$-Alkoxyimino- oder $C_{1-3}$-Alkylidengruppe, die jeweils im Alkyl- oder Alkoxyteil durch eine Carboxygruppe substituiert sind, darstellen,

eine in 1-Stellung durch eine Imidazolgruppe substituierte $C_{1-2}$-Alkylgruppe, in der der Imidazolring durch 1 bis 3 Methylgruppen substituiert sein kann oder durch zwei Methylgruppen und eine Ethylgruppe substituiert ist, wobei zusätzlich einer der vorstehend erwähnten Methyl- oder Ethylsubstituenten gleichzeitig durch eine Carboxygruppe substituiert sein kann,

eine Methyl- oder Ethylgruppe, die in 1-Stellung

durch eine $R_3NR_4$- oder $R_3NR_4$-$CH_2$-Gruppe und

durch eine Di-($C_{1-3}$-Alkyl)-aminocarbonylgruppe, eine gegebenenfalls durch eine $C_{1-3}$-Alkylgruppe substituierte Pyrrolidinocarbonyl- oder Piperidinocarbonylgruppe, wobei bei den vorstehend erwähnten Gruppen jeweils ein Alkylteil oder Alkylsubstituent durch eine Carboxygruppe substituiert sein kann, in denen

$R_3$ ein Wasserstoffatom oder eine gegebenenfalls durch eine Carboxygruppe substituierte $C_{1-3}$-Alkylgruppe und

$R_4$ eine $C_{1-3}$-Alkyl-$Y_2$- oder Carboxy-$C_{1-3}$-alkyl-$Y_2$-Gruppe darstellen, in denen

$Y_2$ eine Kohlenstoff-Stickstoffbindung, eine Carbonylgruppe oder eine gegebenenfalls durch eine $C_{1-3}$-Alkylgruppe substituierte Iminogruppe darstellt,

$R_b$ eine Methylgruppe und

$R_c$ eine gegebenenfalls durch eine $C_{1-8}$-Alkoxycarbonyl-, Acetoxymethyloxycarbonyl-, 2,2,2-Trichlorethoxycarbonyl-, Benzyloxycarbonyl- oder Benzoylgruppe substituierte Amidinogruppe bedeuten,

deren $C_{1-3}$-Alkanolester, deren Tautomere, deren Stereoisomere und deren Salze.

**6.** Benzimidazole der allgemeinen Formel I nach mindestens einem der Ansprüche 1 bis 5, in denen der Rest $R_a$ in 5-Stellung steht,

deren Tautomere, deren Stereoisomere und deren Salze.

**7.** Folgende Verbindungen der allgemeinen Formel Ia:

(a) 2-(4-Amidinophenylaminomethyl)-1-methyl-5-[1-(pyrrolidin-1-yl-carbonyl)cyclopropyl]-benzimidazol,

(b) (E/Z)-2-(4-Amidinophenylaminomethyl)-1-methyl-5-[1-[(pyridin-2-yl)-(carboxymethyloxyimino)methylen] cyclopropyl]-benzimidazol,

(c) 2-(4-Amidinophenylaminomethyl)-1-methyl-5-[1-(2-carboxyethylamino)-1-(pyrrolidin-1-yl-carbonyl)-ethyl]-benzimidazol,

(d) 2-(4-Amidinophenylaminomethyl)-1-methyl-5-[1-[2-(2-carboxyethyl)-pyrrolidin-1-yl-carbonyl]cyclopropyl]-benzimidazol,

(e) 2-(4-Amidinophenylaminomethyl)-1-methyl-5-[2-(2-carboxyethyl)-4,5-dimethyl-imidazol-1-yl-methyl]-benzimidazol

(f) 2-(4-Amidinophenylaminomethyl)-1-methyl-5-[1-(carboxymethylamino)-1-(pyrrolidin-1-yl-carbonyl)-ethyl]-benzimidazol und

(g) 2-(4-Amidinophenylaminomethyl)-1-methyl-5-[1-(N-methylcarboxymethylcarbonylaminomethyl)-1-methyl-1-(pyrrolidin-1-ylcarbonyl)-ethyl]-benzimidazol

sowie deren $C_{1-3}$-Alkanolester, deren N-($C_{1-8}$-Alkoxycarbonyl)-, N-Benzyloxycarbonyl- und N-Benzoyl-amidine, deren Tautomere, deren Stereoisomere und deren Salze.

**8.** 2-(4-Amidinophenylaminomethyl)-1-methyl-5-[1-(carboxymethylamino)-1-(pyrrolidin-1-yl-carbonyl)-ethyl]-benzimidazol, dessen $C_{1-3}$-Alkanolester und deren N-($C_{1-8}$-Alkoxycarbonyl)-N-Benzyloxycarbonyl- und N-Benzoyl-amidine, deren Tautomere, Stereoisomere und Salze.

**9.** (R)-2-(4-Amidinophenylaminomethyl)-1-methyl-5-[1-(carboxymethylamino)-1-(pyrrolidinocarbonyl)-ethyl]-benzimidazol, dessen $C_{1-3}$-Alkanolester und deren N-($C_{1-8}$-Alkoxycarbonyl)-, N-Benzyloxycarbonyl- und N-Benzoyl-amidine und deren Salze.

**10.** Physiologisch verträgliche Salze der Verbindungen gemäß den Ansprüchen 1 bis 9, in denen $R_c$ eine der in den Ansprüchen 1 bis 9 erwähnten Amidinogruppen darstellt.

**11.** Arzneimittel, enthaltend eine Verbindung nach mindestens einem der Ansprüche 1 bis 9, in denen $R_c$ eine der in den Ansprüchen 1 bis 9 erwähnten Amidinogruppen darstellt, oder ein Salz gemäß Anspruch 10 neben gegebenenfalls einem oder mehreren inerten Trägerstoffen und/oder Verdünnungsmitteln.

**12.** Verwendung einer Verbindung nach mindestens einem der Ansprüche 1 bis 9, in denen $R_c$ eine der in den Ansprüchen 1 bis 9 erwähnten Amidinogruppen darstellt, oder ein Salz gemäß Anspruch 10 zur Herstellung eines Arzneimittels mit einer die Thrombinzeit verlängernder Wirkung, einer thrombinhemmender Wirkung und einer Hemmwirkung auf verwandte Serinproteasen.

**13.** Verfahren zur Herstellung eines Arzneimittels gemäß Anspruch 11, **dadurch gekennzeichnet, daß** auf nichtchemischem Wege eine Verbindung nach mindestens einem der Ansprüche 1 bis 9, in denen $R_c$ eine der in den Ansprüchen 1 bis 9 erwähnten Amidinogruppen darstellt, oder ein Salz gemäß Anspruch 10 in einen oder mehrere inerte Trägerstoffe und/oder Verdünnungsmittel eingearbeitet wird.

**14.** Verfahren zur Herstellung der Verbindungen gemäß den Ansprüchen 1 bis 10, **dadurch gekennzeichnet, daß**

a) zur Herstellung einer Verbindung der allgemeinen Formel I, in der $R_c$ eine Cyanogruppe darstellt, eine gegebenenfalls im Reaktionsgemisch gebildete Verbindung der allgemeinen Formel

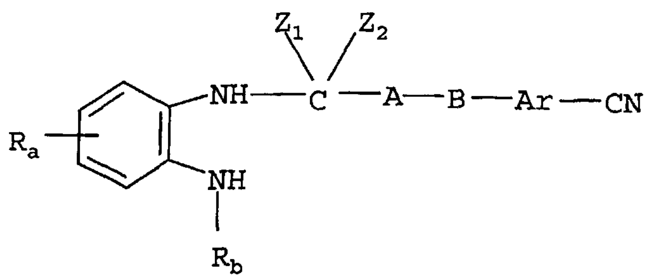

(II),

in der

$R_a$, $R_b$, Ar, A und B wie in den Ansprüchen 1 bis 9 erwähnt definiert sind,

$Z_1$ und $Z_2$, die gleich oder verschieden sein können, gegebenenfalls durch Alkylgruppen mit 1 bis 6 Kohlenstoffatomen substituierte Amino-, Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung einer Verbindung der allgemeinen Formel I, in der $R_a$ eine $R_2$-CX'-$C_{3-5}$-cycloalkylengruppe bedeutet, in der $R_2$ wie in den Ansprüchen 1 bis 9 erwähnt definiert ist und X' einen der für X in den Ansprüchen 1 bis 9 erwähnten Iminoreste darstellt, eine Verbindung der allgemeinen Formel

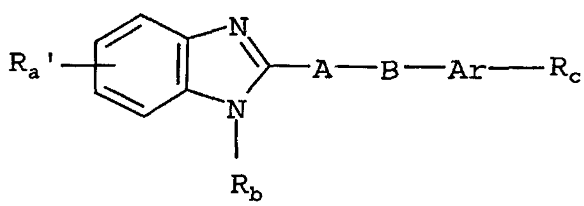

(IV),

in der

$R_b$, $R_c$, Ar, A und B wie in den Ansprüchen 1 bis 9 erwähnt definiert sind und

$R_a$' eine $R_2$-CO-$C_{3-5}$-cycloalkylengruppe darstellt, wobei

$R_2$ wie in den Ansprüchen 1 bis 9 erwähnt definiert ist,

mit einem Amin der allgemeinen Formel

$$H_2X' \quad (V),$$

in der

X' eine der für X in den Ansprüchen 1 bis 9 erwähnten Iminoreste darstellt, umgesetzt wird oder

c) zur Herstellung einer Verbindung der allgemeinen Formel I, in der $R_a$ eine $R_2$-CX"-$C_{3-5}$-cycloalkylengruppe bedeutet, in der $R_2$ wie in den Ansprüchen 1 bis 9 erwähnt definiert ist und X" einen der für X in den Ansprüchen 1 bis 9 erwähnten Alkylidenreste darstellt, eine Verbindung der allgemeinen Formel

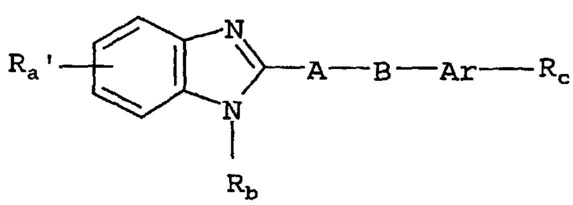

(IV),

in der
$R_b$, $R_c$, Ar, A und B wie in den Ansprüchen 1 bis 9 erwähnt definiert sind und
$R_a$' eine $R_2$-CO-$C_{3-5}$-cycloalkylengruppe darstellt, wobei
$R_2$ wie in den Ansprüchen 1 bis 9 erwähnt definiert ist,
mit einem Phosphon der allgemeinen Formel

$$Z_3\text{-HX''} \qquad (VI),$$

in der X" einen der für X in den Ansprüchen 1 bis 9 erwähnten Alkylidenreste und
$Z_3$ eine Triphenylphospono- oder Di-($C_{1-3}$-alkoxy)phosphonogruppe darstellt, umgesetzt wird oder

d) zur Herstellung einer Verbindung der allgemeinen Formel I, in der $R_c$ eine Amidinogruppe, die durch eine oder zwei $C_{1-3}$-Alkylgruppen substituiert sein kann, eine gegebenenfalls im Reaktionsgemisch gebildete Verbindung der allgemeinen Formel

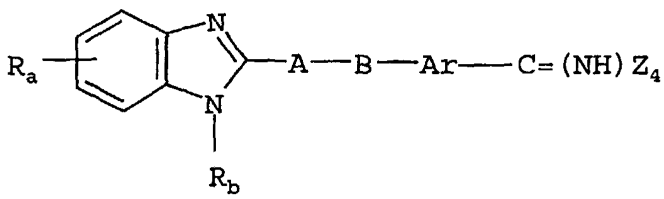

(VII),

in der
$R_a$, $R_b$, Ar, A und B wie in den Ansprüchen 1 bis 9 erwähnt definiert sind und
$Z_4$ eine Alkoxy-, Aralkoxy-, Alkylthio- oder Aralkylthiogruppe darstellt, mit einem Amin der allgemeinen Formel

$$H\text{ - }R_7NR_8\text{ ,} \qquad (VIII)$$

in der
$R_7$ und $R_8$, die gleich oder verschieden sein können, jeweils ein Wasserstoffatom oder eine $C_{1-3}$-Alklylgruppe bedeuten, oder mit dessen Salzen umgesetzt wird oder

e) zur Herstellung einer Verbindung der allgemeinen Formel I, in der $R_a$ eine Imidazolidin-2,4-dion-5-yl-Gruppe, die durch eine oder zwei $C_{1-3}$-Alkylgruppen substituiert sein kann, wobei gleichzeitig ein Alkylsubstituent durch eine Carboxy- oder $C_{1-3}$-Alkoxycarbonylgruppe substituiert sein kann, eine gegebenenfalls im Reationsgemisch gebildete Verbindung der allgemeinen Formel

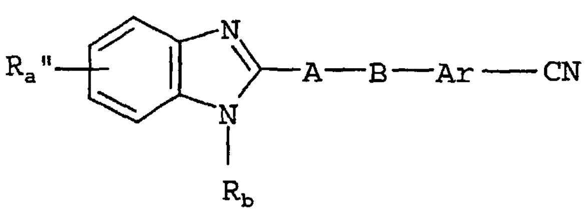

(IX),

in der
$R_b$, Ar, A und B wie in den Ansprüchen 1 bis 9 erwähnt definiert sind und
$R_a$" eine Aminocarbonyaminogruppe, die in 3-Stellung durch eine $C_{1-3}$-Alkoxycarbonyl-$C_{1-3}$-alkylgruppe substituiert ist, bedeutet, cyclisiert wird oder

f) zur Herstellung einer Verbindung der allgemeinen Formel I, in der $R_c$ eine Hydroxyamidinogruppe darstellt, ein Nitril der allgemeinen Formel

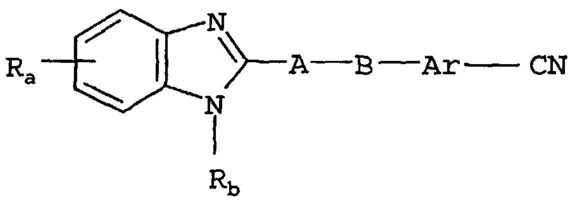

(X),

in der
$R_a$, $R_b$, Ar, A und B wie in den Ansprüchen 1 bis 9 erwähnt definiert sind, mit Hydroxylamin oder dessen Salzen umgesetzt wird oder

g) zur Herstellung einer Verbindung der allgemeinen Formel I, in der $R_a$ eine Carboxygruppe enthält und $R_c$ wie in den Ansprüchen 1 bis 9 erwähnt definiert ist oder $R_a$ wie in den Ansprüchen 1 bis 9 erwähnt definiert ist und $R_c$ eine gegebenenfalls durch eine Hydroxygruppe oder durch eine oder zwei $C_{1-3}$-Alkylgruppen substituierte Amidinogruppe darstellt, eine Verbindung der allgemeinen Formel

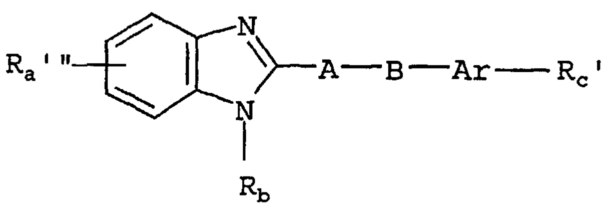

(XI),

in der
$R_b$, Ar, A und B wie in den Ansprüchen 1 bis 9 erwähnt definiert sind und
$R_a$' " und $R_c$' die für $R_a$ und $R_c$ in den Ansprüchen 1 bis 9 erwähnten Bedeutungen mit der Maßgabe besitzen, daß $R_a$ eine durch Hydrolyse, Behandeln mit einer Säure oder Base, Thermolyse oder Hydrogenolyse in eine Carboxygruppe überführbare Gruppe enthält und $R_c$ wie in den Ansprüchen 1 bis 9 erwähnt definiert ist oder $R_c$ eine durch Hydrolyse, Behandeln mit einer Säure oder Base, Thermolyse oder Hydrogenolyse in eine gegebenenfalls durch eine Hydroxygruppe oder durch eine oder zwei $C_{1-3}$-Alkylgruppen substituierte Amidinogruppe überführbare Gruppe darstellt und $R_a$ wie in den Ansprüchen 1 bis 9 erwähnt definiert ist,
mittels Hydrolyse, Behandeln mit einer Säure oder Base, Thermolyse oder Hydrogenolyse in eine Verbindung der allgemeinen Formel I übergeführt wird, in der $R_a$ eine Carboxygruppe enthält und $R_c$ wie in den Ansprüchen 1 bis 9 erwähnt definiert ist oder $R_a$ wie in den Ansprüchen 1 bis 9 erwähnt definiert ist und $R_c$ eine gegebenenfalls durch eine Hydroxygruppe oder durch eine oder zwei $C_{1-3}$-Alkylgruppen substituierte Amidinogruppe darstellt, oder

h) zur Herstellung einer Verbindung der allgemeinen Formel I, in der $R_c$ eine Amidinogruppe, die durch eine oder zwei $C_{1-8}$-Alkoxycarbonylgruppen oder durch einen in vivo abspaltbaren Rest substituiert ist, eine Verbindung der allgemeinen Formel

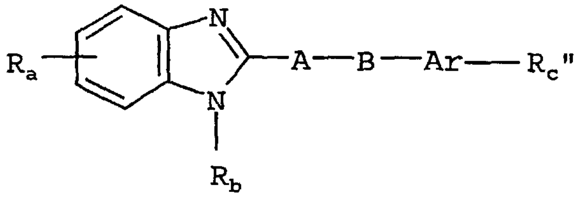

(XII),

in der
$R_a$, $R_b$, Ar, A und B wie in den Ansprüchen 1 bis 9 erwähnt definiert sind und
$R_c$" eine Amidinogruppe bedeutet, mit einer Verbindung der allgemeinen Formel

$$Z_5 - R_9 \qquad (XIII),$$

in der
$R_9$ eine $C_{1-8}$-Alkoxycarbonylgruppe oder den Acylrest einer der eingangs erwähnten in vivo abspaltbaren Reste und
$Z_5$ eine nukleofuge Austrittsgruppe bedeuten, umgesetzt wird und
gewünschtenfalls anschließend eine so erhaltene Verbindung der allgemeinen Formel I, die eine ($R_3NR_4$) -$C_{1-3}$-alkylgruppe enthält, in der mindestens einer der Reste $R_3$ oder $R_4$ ein Wasserstoffatom darstellt, mit einem entsprechenden Isocyanat oder Carbamoylhalogenid in eine entsprechende Harnstoffverbindung der allgemeinen Formel I übergeführt wird und/oder
eine so erhaltene Verbindung der allgemeinen Formel I, die eine $NH_2$-$C_{1-3}$-alkylgruppe enthält, mit einem entsprechenden Acrylsäureester in eine entsprechende 2-($C_{1-3}$-Alkoxycarbonyl)-ethyl-Verbindung der allgemeinen Formel I übergeführt wird und/oder
eine so erhaltene Verbindung der allgemeinen Formel I, die eine ($R_3NR_4$)-$C_{1-3}$-alkylgruppe enthält, in der $R_3$ und $R_4$ jeweils ein Wasserstoffatom darstellen, mit einem entsprechenden Dihalogenalkan in eine entsprechende Verbindung der allgemeinen Formel I, in der $R_3$ und $R_4$ zusammen mit dem dazwischen liegenden Stickstoffatom eine entsprechende 4- bis 7-gliedrig Cycloalkyleniminogruppe darstellt, übergeführt wird und/oder
eine so erhaltene Verbindung der allgemeinen Formel I, in der $R_c$ eine Amidinogruppe darstellt, mit einem Halogenessigsäurederivat sowie anschließender Hydrolyse und Decarboxylierung in eine durch eine oder zwei Methylgruppen substituierte entsprechende Amidinoverbindung übergeführt wird und/oder
eine so erhaltene Verbindung der allgemeinen Formel I, in der $R_c$ eine Hydroxyamidinogruppe darstellt, mittels katalytischer Hydrierung in eine entsprechende Amidinoverbindung übergeführt wird und/oder
eine so erhaltene Verbindung der allgemeinen Formel I, in der $R_a$ eine Carboxygruppe enthält, mittels Veresterung in einen entsprechenden Ester übergeführt wird und/oder
ein während den Umsetzungen zum Schutze von reaktiven Gruppen verwendeter Schutzrest abgespalten wird und/oder
eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

1. Benzimidazoles of general formula

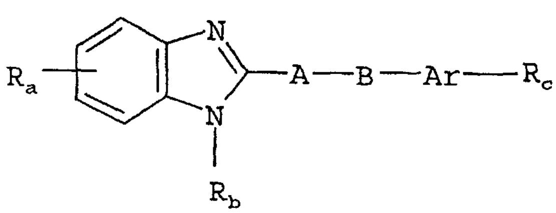

(I),

wherein
Ar denotes a phenylene or naphthylene group optionally substituted by a fluorine, chlorine or bromine atom, by a trifluoromethyl, $C_{1-3}$-alkyl or $C_{1-3}$-alkoxy group,
a thienylene, thiazolylene, pyridinylene, pyrimidinylene, pyrazinylene or pyridazinylene group optionally substituted in the carbon skeleton by a $C_{1-3}$-alkyl group,
A denotes a $C_{1-3}$-alkylene group,
B denotes an oxygen or sulphur atom, a methylene, carbonyl, sulphinyl or sulphonyl group, an imino group optionally substituted by a $C_{1-3}$-alkyl group wherein the alkyl moiety may be mono- or disubstituted by a carboxy group,
$R_a$ denotes an $R_1$-CO-$C_{3-5}$-cycloalkyl group wherein

$R_1$ denotes a $C_{1-3}$-alkoxy, amino, $C_{1-4}$-alkylamino or di-($C_{1-4}$-alkyl)-amino group wherein each alkyl moiety may be substituted by a carboxy group,

a 4- to 7-membered cycloalkyleneimino or cycloalkenyleneimino group which may be substituted by a hydroxy group or by one or two $C_{1-3}$-alkyl groups, whilst an alkyl substituent may simultaneously be substituted by a hydroxy, $C_{1-3}$-alkoxy, carboxy, carboxy-$C_{1-3}$-alkoxy, carboxy-$C_{1-3}$-alkylamino, N-($C_{1-3}$-alkyl)-N- (carboxy-$C_{1-3}$-alkyl)-amino, carboxy-$C_{1-3}$-alkylaminocarbonyl, N-($C_{1-3}$-alkyl)-N-(carboxy-$C_{1-3}$-alkyl)-aminocarbonyl, carboxy-$C_{1-3}$-alkylamino-carbonylamino, 1- ($C_{1-3}$-alkyl)-3-(carboxy-$C_{1-3}$-alkyl)-aminocarbonylamino, 3-($C_{1-3}$-alkyl)-3-(carboxy-$C_{1-3}$-alkyl)-aminocarbonylamino or 1,3-di-($C_{1-3}$-alkyl)-3-(carboxy-$C_{1-3}$-alkyl)-aminocarbonylamino group,

a 4- to 7-membered cycloalkenyleneimino group substituted by a hydroxy group,

a 5- to 7-membered cycloalkyleneimino group optionally substituted by a $C_{1-3}$-alkyl group, to which a phenyl ring is fused via two adjacent carbon atoms,

a morpholino, piperazino, N-($C_{1-3}$-alkyl)-piperazino, pyrrolino, 3,4-dehydro-piperidino or pyrrol-1-yl group,

an $R_2$-CX-$C_{3-5}$-cycloalkyl group wherein

$R_2$ denotes a phenyl, naphthyl or monocyclic 5- or 6-membered heteroaryl group optionally substituted by a $C_{1-3}$-alkyl group, wherein the 6-membered heteroaryl group contains one, two or three nitrogen atoms and the 5-membered heteroaryl group contains an imino group optionally substituted by a $C_{1-3}$-alkyl group, an oxygen or sulphur atom or an imino group optionally substituted by a $C_{1-3}$-alkyl group and an oxygen or sulphur atom or one or two nitrogen atoms and the abovementioned alkyl substituent may be substituted by a carboxy, carboxy-$C_{1-3}$-alkoxy, carboxy-$C_{1-3}$-alkylamino or N-($C_{1-3}$-alkyl)-carboxy-$C_{1-3}$-alkylamino group, and

X denotes an oxygen atom, a $C_{1-3}$-alkylimino, $C_{1-3}$-alkoxyimino, $C_{1-3}$-alkylhydrazino, di-($C_{1-3}$-alkyl)-hydrazino, $C_{2-4}$-alkanoylhydrazino, N-($C_{1-3}$-alkyl)-$C_{2-4}$-alkanoylhydrazino or $C_{1-3}$-alkylidene group each of which may be substituted in the alkyl or alkanoyl moiety or in the alkyl and alkanoyl moieties by a carboxy group,

a $C_{1-3}$-alkyl or $C_{3-5}$-cycloalkyl group substituted by an imidazole or imidazolone group wherein

the imidazole ring may be substituted by a phenyl or carboxy group and by one or two $C_{1-3}$-alkyl groups or by one, two or three $C_{1-3}$-alkyl groups, wherein the substituents may be identical or different and one of the abovementioned alkyl substituents may simultaneously be substituted by a carboxy group or may be substituted in the 2 or 3 position by an amino, $C_{2-4}$-alkanoylamino, $C_{1-3}$-alkylamino, N-($C_{2-4}$-alkanoyl) -$C_{1-3}$-alkylamino or di-($C_{1-3}$-alkyl)-amino group, and

the imidazolone ring may be substituted by a $C_{1-3}$-alkyl group, whilst the alkyl substituent may be substituted by a carboxy group or in the 2 or 3 position by an amino, $C_{2-4}$-alkanoylamino, $C_{1-3}$-alkylamino, N-($C_{2-4}$-alkanoyl) -$C_{1-3}$-alkylamino or di-($C_{1-3}$-alkyl)-amino group, and

additionally a phenyl or pyridine ring may be fused to the abovementioned imidazole or imidazolone rings via two adjacent carbon atoms,

an imidazolidine-2,4-dion-5-yl group which may be substituted by one or two $C_{1-3}$-alkyl groups, whilst at the same time an alkyl substituent may be substituted by a carboxy group,

a $C_{1-4}$-alkyl group which is substituted

by a $C_{1-3}$-alkyl-$Y_1$-$C_{1-3}$-alkyl, HOOC-$C_{1-3}$-alkyl-$Y_1$-$C_{1-3}$-alkyl, tetrazolyl-$C_{1-3}$-alkyl-$Y_2$, $R_3NR_4$- or $R_3NR_4$-$C_{1-3}$-alkyl group and

by an isoxazolidinylcarbonyl group optionally substituted by a $C_{1-3}$-alkyl group, by a pyrrolinocarbonyl, 3,4-dehydro-piperidinocarbonyl, pyrrol-1-yl-carbonyl, carboxy, aminocarbonyl, $C_{1-3}$-alkylaminocarbonyl, di-($C_{1-3}$-alkyl)-aminocarbonyl or 4- to 7-membered cycloalkyleneiminocarbonyl group, whilst in the abovementioned groups the cycloalkyleneimino moiety may be substituted by one or two $C_{1-3}$-alkyl groups and at the same time each alkyl moiety or alkyl substituent in the abovementioned $C_{1-3}$-alkylaminocarbonyl, di-($C_{1-3}$-alkyl)-aminocarbonyl or cycloalkyleneiminocarbonyl groups may be substituted by a carboxy group, and the remaining hydrogen atoms of the $C_{1-4}$-alkyl group may be wholly or partially replaced by fluorine atoms wherein

$R_3$ denotes a hydrogen atom or a $C_{1-3}$-alkyl group optionally substituted by a carboxy group and

$R_4$ denotes a hydrogen atom, a $C_{1-3}$-alkyl-$Y_1$-$C_{1-3}$-alkyl-$Y_2$, carboxy-$C_{1-3}$-alkyl-$Y_1$-$C_{1-3}$-alkyl-$Y_2$, $C_{1-3}$-alkyl-$Y_2$ or carboxy-$C_{1-3}$-alkyl-$Y_2$ group or

$R_3$ and $R_4$ together with the nitrogen atom between them denote an 4- to 7-membered cycloalkyleneimino group optionally substituted by a carboxy, $C_{1-3}$-alkyl or carboxy-$C_{1-3}$-alkyl group wherein

$Y_1$ denotes a carbon-carbon bond, an oxygen atom, a sulphenyl, sulphinyl, sulphonyl, -NH-, -NH-CO- or -NH-CO-NH- group and

$Y_2$ denotes a carbon-nitrogen bond or a carbonyl, sulphonyl, imino or -NH-CO- group, wherein the carbonylgroup of the -NH-CO- group is linked to the nitrogen atom of the $R_3NR_4$- group, and the imino groups occurring in the definition of the groups $Y_1$ and $Y_2$ may each additionally be substituted by a $C_{1-3}$-alkyl or carboxy-$C_{1-3}$-alkyl group,

a $C_{1-3}$-alkyl or $C_{3-5}$-cycloalkyl group substituted by a $R_5NR_6$- group wherein

$R_5$ denotes a hydrogen atom, a $C_{1-3}$-alkyl, $C_{5-7}$-cycloalkyl, phenylcarbonyl, phenylsulphonyl or pyridinyl group

and

$R_6$ denotes a $C_{1-3}$-alkyl, carboxy-$C_{1-3}$-alkyl or carboxy-$C_{1-3}$-alkylcarbonyl group, a $C_{1-3}$-alkyl group which is substituted by a $C_{2-4}$-alkanoyl or $C_{5-7}$-cycloalkanoyl group and by a $C_{1-3}$-alkyl group substituted by a chlorine, bromine or iodine atom,

$R_b$ denotes a hydrogen atom or a $C_{1-3}$-alkyl group and

$R_c$ denotes a cyano group or an amidino group optionally substituted by one or two $C_{1-3}$-alkyl groups, wherein the carboxy groups mentioned in the definitions of the abovementioned groups may also be replaced by a group which can be converted *in vivo* into a carboxy group or by a group which is negatively charged under physiological conditions, or

the amino and imino groups mentioned in the definitions of the abovementioned groups may also be substituted by a group which can be cleaved *in vivo*, while

by a group which can be converted *in vivo* into a carboxy group is meant a hydroxmethyl group, a carboxy group esterified with an alcohol, wherein the alcoholic moiety is a $C_{1-6}$-alkanol, a phenyl-$C_{1-3}$-alkanol, a $C_{3-9}$-cycloalkanol, whilst a $C_{5-8}$-cycloalkanol may additionally be substituted by one or two $C_{1-3}$-alkyl groups, a $C_{5-8}$-cycloalkanol wherein a methylene group in the 3- or 4-position is replaced by an oxygen atom or by an imino group optionally substituted by a $C_{1-3}$-alkyl, phenyl-$C_{1-3}$-alkyl, phenyl-$C_{1-3}$-alkoxycarbonyl or $C_{2-6}$-alkanoyl group and the cycloalkanol moiety may additionally be substituted by one or two $C_{1-3}$-alkyl groups, a $C_{4-7}$-cycloalkenol, a $C_{3-5}$-alkenol, a phenyl-$C_{3-5}$-alkenol, a $C_{3-5}$-alkynol or phenyl-$C_{3-5}$-alkynol, with the proviso that no bond to the oxygen atom starts from a carbon atom which carries a double or triple bond, a $C_{3-8}$-cycloalkyl-$C_{1-3}$-alkanol, a bicycloalkanol with a total of 8 to 10 carbon atoms, which may additionally be substituted in the bicycloalkyl moiety by one or two $C_{1-3}$-alkyl groups, a 1,3-dihydro-3-oxo-1-isobenzofuranol or an alcohol of formula

$$R_d\text{-CO-O-}(R_eCR_f)\text{-OH},$$

wherein

$R_d$ denotes a $C_{1-8}$-alkyl, $C_{5-7}$-cycloalkyl, phenyl or phenyl-$C_{1-3}$-alkyl group,

$R_e$ denotes a hydrogen atom, a $C_{1-3}$-alkyl, $C_{5-7}$-cycloalkyl or phenyl group and

$R_f$ denotes a hydrogen atom or a $C_{1-3}$-alkyl group,

by a group which is negatively charged under physiological conditions is meant a tetrazol-5-yl, phenylcarbonylaminocarbonyl, trifluormethylcarbonylaminocarbonyl, $C_{1-6}$-alkylsulphonylamino, phenylsulphonylamino, benzylsulphonylamino, trifluoromethylsulphonylamino, $C_{1-6}$-alkylsulphonylaminocarbonyl, phenylsulphonylaminocarbonyl, benzylsulphonylaminocarbonyl or perfluoro-$C_{1-6}$-alkylsulphonylaminocarbonyl group

and by a group which can be cleaved *in vivo* from an imino or amino group is meant a hydroxy group, a benzoyl group optionally mono- or disubstituted by fluorine, chlorine, bromine or iodine atoms, by $C_{1-3}$-alkyl or $C_{1-3}$-alkoxy groups, wherein the substituents may be identical or different, a pyridinoyl group or a $C_{1-16}$-alkanoyl group, a 3,3,3-trichloropropionyl or allyloxycarbonyl group, a $C_{1-16}$-alkoxycarbonyl or $C_{1-16}$-alkylcarbonyloxy group wherein hydrogen atoms may be wholly or partially replaced by fluorine or chlorine atoms, a phenyl-$C_{1-6}$-alkoxycarbonyl group, a 3-amino-propionyl group wherein the amino group may be mono- or disubstituted by $C_{1-6}$-alkyl or $C_{3-7}$-cycloalkyl groups and the substituents may be identical or different, a $C_{1-3}$-alkylsulphonyl-$C_{2-4}$-alkoxycarbonyl, $C_{1-3}$-alkoxy-$C_{2-4}$-alkoxy-$C_{2-4}$-alkoxycarbonyl, $R_d$-CO-O-($R_d$C$R_f$)-O-CO-, $C_{1-6}$-alkyl-CO-NH-($R_g$C$R_h$)-O-CO- or $C_{1-6}$-alkyl-CO-O-($R_g$C$R_h$)-($R_g$C$R_h$)-O-CO- group wherein $R_d$ to $R_f$ are as hereinbefore defined,

$R_g$ and $R_h$, which may be identical or different, denote hydrogen atoms or $C_{1-3}$-alkyl groups,

the tautomers. stereoisomers, mixtures thereof and the salts thereof.

**2.** Benzimidazoles of general formula

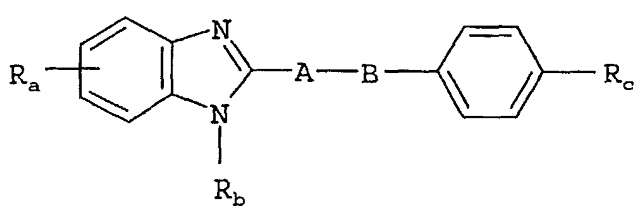

(Ia),

wherein

A denotes a $C_{1-3}$-alkylene group,

B denotes an oxygen or sulphur atom, a methylene, carbonyl, sulphinyl or sulphonyl group, an imino group optionally substituted by a $C_{1-3}$-alkyl group wherein the alkyl moiety may be mono- or disubstituted by a carboxy group,
$R_a$ denotes an $R_1$-CO-$C_{3-5}$-cycloalkyl group wherein

$R_1$ denotes a $C_{1-3}$-alkoxy, amino, $C_{1-4}$-alkylamino or di-($C_{1-4}$-alkyl)-amino group wherein each alkyl moiety may be substituted by a carboxy group,

a 4- to 7-membered cycloalkyleneimino or cycloalkenyleneimino group which may be substituted by one or two $C_{1-3}$-alkyl groups, whilst an alkyl substituent may simultaneously be substituted by a hydroxy, $C_{1-3}$-alkoxy, carboxy, carboxy-$C_{1-3}$-alkoxy, carboxy-$C_{1-3}$-alkylamino, N-($C_{1-3}$-alkyl)-N-(carboxy-$C_{1-3}$-alkyl)-amino, carboxy-$C_{1-3}$-alkylaminocarbonyl, N-($C_{1-3}$-alkyl)-N-(carboxy-$C_{1-3}$-alkyl)-aminocarbonyl, carboxy-$C_{1-3}$-alkylaminocarbonylamino, 1-($C_{1-3}$-alkyl)-3-(carboxy-$C_{1-3}$-alkyl)-aminocarbonylamino, 3-($C_{1-3}$-alkyl)-3-(carboxy-$C_{1-3}$-alkyl)-aminocarbonylamino or 1, 3-di-($C_{1-3}$-alkyl)-3-(carboxy-$C_{1-3}$-alkyl)-aminocarbonylamino group,

a 4- to 7-membered cycloalkenyleneimino group substituted by a hydroxy group,

a 5- to 7-membered cycloalkyleneimino group optionally substituted by a $C_{1-3}$-alkyl group, to which a phenyl ring is fused via two adjacent carbon atoms,

a morpholino, piperazino, N-($C_{1-3}$-alkyl)-piperazino, pyrrolino, 3,4-dehydro-piperidino or pyrrol-1-yl group,

an $R_2$-CX-$C_{3-5}$-cycloalkyl group wherein

$R_2$ denotes a phenyl, naphthyl or monocyclic 5- or 6-membered heteroaryl group optionally substituted by a $C_{1-3}$-alkyl group, wherein the 6-membered heteroaryl group contains one, two or three nitrogen atoms and the 5-membered heteroaryl group contains an imino group optionally substituted by a $C_{1-3}$-alkyl group, an oxygen or sulphur atom or an imino group optionally substituted by a $C_{1-3}$-alkyl group and an oxygen or sulphur atom or one or two nitrogen atoms and the abovementioned alkyl substituent may be substituted by a carboxy, carboxy-$C_{1-3}$-alkoxyl carboxy-$C_{1-3}$-alkylamino or N-($C_{1-3}$-alkyl)-carboxy-$C_{1-3}$-alkylamino group, and

X denotes an oxygen atom, a $C_{1-3}$-alkylimino, $C_{1-3}$-alkoxyimino, $C_{1-3}$-alkylhydrazino, di-($C_{1-3}$-alkyl)-hydrazino, $C_{2-4}$-alkanoylhydrazino, N-($C_{1-3}$-alkyl)-$C_{2-4}$-alkanoylhydrazino or $C_{1-3}$-alkylidene group each of which may be substituted in the alkyl or alkanoyl moiety or in the alkyl and alkanoyl moieties by a carboxy group,

a $C_{1-3}$-alkyl or $C_{3-5}$-cycloalkyl group substituted by an imidazole or imidazolone group wherein

the imidazole ring may be substituted by a phenyl or carboxy group and by one or two $C_{1-3}$-alkyl groups or by one, two or three $C_{1-3}$-alkyl groups, wherein the substituents may be identical or different and one of the abovementioned alkyl substituents may simultaneously be substituted by a carboxy group or may be substituted in the 2 or 3 position by an amino, $C_{2-4}$-alkanoylamino, $C_{1-3}$-alkylamino, N-($C_{2-4}$-alkanoyl)-$C_{1-3}$-alkylamino or di-($C_{1-3}$-alkyl)-amino group, and

the imidazolone ring may be substituted by a $C_{1-3}$-alkyl group, whilst the alkyl substituent may be substituted by a carboxy group or in the 2 or 3 position by an amino, $C_{2-4}$-alkanoylamino, $C_{1-3}$-alkylamino, N-($C_{2-4}$-alkanoyl) -$C_{1-3}$-alkylamino or di-($C_{1-3}$-alkyl)-amino group, and

additionally a phenyl or pyridine ring may be fused to the abovementioned imidazole or imidazolone rings via two adjacent carbon atoms,

an imidazolidine-2,4-dion-5-yl group which may be substituted by one or two $C_{1-3}$-alkyl groups, whilst at the same time an alkyl substituent may be substituted by a carboxy group,

a $C_{1-4}$-alkyl group which is substituted

by a $C_{1-3}$-alkyl-$Y_1$-$C_{1-3}$-alkyl, HOOC-$C_{1-3}$-alkyl-$Y_1$-$C_{1-3}$-alkyl, tetrazolyl-$C_{1-3}$-alkyl-$Y_2$, $R_3NR_4$- or $R_3NR_4$-$C_{1-3}$-alkyl group and

by an isoxazolidinylcarbonyl group optionally substituted by a $C_{1-3}$-alkyl group, by a pyrrolinocarbonyl, 3,4-dehydro-piperidinocarbonyl, pyrrol-1-yl-carbonyl, carboxy, aminocarbonyl, $C_{1-3}$-alkylaminocarbonyl, di-($C_{1-3}$-alkyl)-aminocarbonyl or 4- to 7-membered cycloalkyleneiminocarbonyl group, whilst in the abovementioned groups the cycloalkyleneimino moiety may be substituted by one or two $C_{1-3}$-alkyl groups and at the same time each alkyl moiety or alkyl substituent in the abovementioned $C_{1-3}$-alkylaminocarbonyl, di-($C_{1-3}$-alkyl)-aminocarbonyl or cycloalkyleneiminocarbonyl groups may be substituted by a carboxy group, and the remaining hydrogen atoms of the $C_{1-4}$-alkyl group may be wholly or partially replaced by fluorine atoms wherein

$R_3$ denotes a hydrogen atom or a $C_{1-3}$-alkyl group optionally substituted by a carboxy group and

$R_4$ denotes a hydrogen atom, a $C_{1-3}$-alkyl-$Y_1$-$C_{1-3}$-alkyl-$Y_2$, carboxy-$C_{1-3}$-alkyl-$Y_1$-$C_{1-3}$-alkyl-$Y_2$, $C_{1-3}$-alkyl-$Y_2$ or carboxy-$C_{1-3}$-alkyl-$Y_2$ group or

$R_3$ and $R_4$ together with the nitrogen atom between them denote an 4- to 7-membered cycloalkyleneimino group optionally substituted by a carboxy, $C_{1-3}$-alkyl or carboxy-$C_{1-3}$-alkyl group wherein

$Y_1$ denotes a carbon-carbon bond, an oxygen atom, a sulphenyl, sulphinyl, sulphonyl, -NH-, -NH-CO- or -NH-CO-NH- group and

$Y_2$ denotes a carbon-nitrogen bond or a carbonyl, sulphonyl, imino or -NH-CO- group, wherein the carbonyl group of the -NH-CO- group is linked to the nitrogen atom of the $R_3NR_4$- group, and the imino groups occurring in the definition of the groups $Y_1$ and $Y_2$ may each additionally be substituted by a $C_{1-3}$-alkyl or carboxy-$C_{1-3}$-alkyl

group,

a $C_{1-3}$-alkyl or $C_{3-5}$-cycloalkyl group substituted by a $R_5NR_6$- group wherein

$R_5$ denotes a hydrogen atom, a $C_{1-3}$-alkyl, $C_{5-7}$-cycloalkyl, phenylcarbonyl, phenylsulphonyl or pyridinyl group and

$R_6$ denotes a $C_{1-3}$-alkyl, carboxy-$C_{1-3}$-alkyl or carboxy-$C_{1-3}$-alkylcarbonyl group,

a $C_{1-3}$-alkyl group which is substituted by a $C_{2-4}$-alkanoyl or $C_{5-7}$-cycloalkanoyl group and by a $C_{1-3}$-alkyl group substituted by a chlorine, bromine or iodine atom,

$R_b$ denotes a hydrogen atom or a $C_{1-3}$-alkyl group and

$R_c$ denotes a cyano group or an amidino group which may be substituted by a hydroxy group, by one or two $C_{1-3}$-alkyl groups, or by one or two $C_{1-8}$-alkoxycarbonyl groups,

wherein the carboxy, amino and imino groups mentioned in the definition of the abovementioned groups may also be substituted by a group which can be cleaved *in vivo* as defined in claim 1,

the tautomers, stereoisomers and salts thereof.

**3.** Benzimidazoles of general formula Ia according to claim 2, wherein

A denotes a $C_{1-3}$-alkylene group,

B denotes an oxygen atom, a methylene, imino or N-($C_{1-3}$-alkyl)-imino group wherein the alkyl moiety may be substituted by a carboxy group,

$R_a$ denotes an $C_{3-5}$-cycloalkyl group substituted by the $R_1$-CO group in the 1 position wherein

$R_1$ denotes a $C_{1-3}$-alkoxy, amino, $C_{1-4}$-alkylamino or di-($C_{1-4}$-alkyl)-amino group wherein each alkyl moiety may be substituted by a carboxy group,

a 4- to 7-membered cycloalkyleneimino group which may be substituted by a hydroxy group or by one or two $C_{1-3}$-alkyl groups, whilst an alkyl substituent may simultaneously be substituted by a hydroxy, $C_{1-3}$-alkoxy, carboxy, carboxy-$C_{1-3}$-alkoxy, carboxy-$C_{1-3}$-alkylamino, N-($C_{1-3}$-alkyl)-N-(carboxy-$C_{1-3}$-alkyl)-amino, carboxy-$C_{1-3}$-alkylaminocarbonyl, N-($C_{1-3}$-alkyl)-N-(carboxy-$C_{1-3}$-alkyl)-aminocarbonyl, carboxy-$C_{1-3}$-alkylaminocarbonylamino, 1-($C_{1-3}$-alkyl)-3-(carboxy-$C_{1-3}$-alkyl)-aminocarbonylamino, 3-($C_{1-3}$-alkyl)-3-(carboxy-$C_{1-3}$-alkyl)-aminocarbonylamino or 1, 3-di-($C_{1-3}$-alkyl)-3-(carboxy-$C_{1-3}$-alkyl)-aminocarbonylamino group,

a 5- to 7-membered cycloalkyleneimino group optionally substituted by a $C_{1-3}$-alkyl group, to which a phenyl ring is fused via two adjacent carbon atoms,

a morpholino, piperazino, N-($C_{1-3}$-alkyl)-piperazino, pyrrolino, 3,4-dehydro-piperidino or pyrrol-1-yl group,

a $C_{3-5}$-cycloalkyl group substituted in the 1 position by the $R_2$-CX- group, wherein

$R_2$ denotes a phenyl, naphthyl or monocyclic 5- or 6-membered heteroaryl group optionally substituted by a $C_{1-3}$-alkyl group, wherein the 6-membered heteroaryl group contains one, two or three nitrogen atoms and the 5-membered heteroaryl group contains an imino group optionally substituted by a $C_{1-3}$-alkyl group, an oxygen or sulphur atom or an imino group optionally substituted by a $C_{1-3}$-alkyl group and an oxygen or sulphur atom or one or two nitrogen atoms and the abovementioned alkyl substituent may be substituted by a carboxy, carboxy-$C_{1-3}$-alkoxy, carboxy-$C_{1-3}$-alkylamino or N-($C_{1-3}$-alkyl)-carboxy-$C_{1-3}$-alkylamino group, and

X denotes an oxygen atom, a $C_{1-3}$-alkylimino, $C_{1-3}$-alkoxyimino or $C_{1-3}$-alkylidene group, each of which may be substituted in the alkyl or alkanoyl moiety by a carboxy group,

a $C_{1-3}$-alkyl group substituted in the 1 position by an imidazole or imidazolone group wherein

the imidazole ring may be substituted by a phenyl or carboxy group and by one or two $C_{1-3}$-alkyl groups or by one, two or three $C_{1-3}$-alkyl groups, wherein the substituents may be identical or different and one of the abovementioned alkyl substituents may simultaneously be substituted by a carboxy group or may be substituted in the 2 or 3 position by an amino, $C_{2-4}$-alkanoylamino, $C_{1-3}$-alkylamino, N-($C_{2-4}$-alkanoyl)-$C_{1-3}$-alkylamino or di-($C_{1-3}$-alkyl)-amino group, and

the imidazolone ring may be substituted by a $C_{1-3}$-alkyl group, whilst the alkyl substituent may be substituted by a carboxy group or in the 2 or 3 position by an amino, $C_{2-4}$-alkanoylamino, $C_{1-3}$-alkylamino, N-($C_{2-4}$-alkanoyl) -$C_{1-3}$-alkylamino or di-($C_{1-3}$-alkyl)-amino group, and

additionally a phenyl or pyridine ring may be fused to the abovementioned imidazole or imidazolone rings via two adjacent carbon atoms,

an imidazolidine-2,4-dion-5-yl group which may be substituted by one or two $C_{1-3}$-alkyl groups, whilst at the same time an alkyl substituent may be substituted by a carboxy group,

a $C_{1-4}$-alkyl group which is substituted in the 1 position

by an $R_3NR_4$- or $R_3NR_4$-$C_{1-3}$-alkyl group and

by a pyrrolinocarbonyl, 2,3-dehydro-piperidinocarbonyl, imidazol-1-yl-carbonyl, carboxy, aminocarbonyl, $C_{1-3}$-alkylaminocarbonyl, di- ($C_{1-3}$-alkyl)-aminocarbonyl, isoxazolidin-1-ylcarbonyl or 4- to 7-membered cycloalkyleneiminocarbonyl group, whilst in the abovementioned groups the cycloalkyleneimino moiety may be substituted by one or two $C_{1-3}$-alkyl groups and at the same time each alkyl moiety or alkyl substituent in the above-

mentioned $C_{1-3}$-alkylaminocarbonyl, di-($C_{1-3}$-alkyl)-aminocarbonyl or cycloalkyleneiminocarbonyl groups may be substituted by a carboxy group, and the remaining hydrogen atoms of the $C_{1-4}$-alkyl group may be wholly or partially replaced by fluorine atoms, wherein

$R_3$ denotes a hydrogen atom or a $C_{1-3}$-alkyl group optionally substituted by a carboxy group and

$R_4$ denotes a hydrogen atom, a $C_{1-3}$-alkyl-$Y_2$ or carboxy-$C_{1-3}$-alkyl-$Y_2$ group or

$R_3$ and $R_4$ together with the nitrogen atom between them denote a 4- to 7-membered cycloalkyleneimino group optionally substituted in the 1 position by a carboxy, $C_{1-3}$-alkyl or carboxy-$C_{1-3}$-alkyl group, wherein

$Y_2$ denotes a carbon-nitrogen bond or a carbonyl, imino or -NH-CO- group, wherein the carbonyl group of the -NH-CO- group is linked to the nitrogen atom of the $R_3NR_4$- group, and the imino group occurring in the definition of the groups $Y_2$ may additionally be substituted by a $C_{1-3}$-alkyl or carboxy-$C_{1-3}$-alkyl group, a $C_{1-3}$-alkyl or $C_{3-5}$-cycloalkyl group substituted in the 1 position by an $R_5NR_6$- group, wherein

$R_5$ denotes a hydrogen atom, a $C_{1-3}$-alkyl, $C_{5-7}$-cycloalkyl, phenylcarbonyl, phenylsulphonyl or pyridinyl group and

$R_6$ denotes a $C_{1-3}$-alkyl, carboxy-$C_{1-3}$-alkyl or carboxy-$C_{1-3}$-alkylcarbonyl group,

a $C_{1-3}$-alkyl group which is substituted by a $C_{2-4}$-alkanoyl or $C_{5-7}$-cycloalkanoyl group and by a $C_{1-3}$-alkyl group substituted by a chlorine, bromine or iodine atom,

$R_b$ denotes a $C_{1-3}$-alkyl group and

$R_c$ denotes an amidino group which may optionally be substituted by a 2,2,2-trichloroethoxycarbonyl, $C_{1-8}$-alkoxycarbonyl, acetoxymethyloxycarbonyl, benzyloxycarbonyl or benzoyl group, whilst the benzoyl moiety may be mono- or disubstituted by fluorine, chlorine, bromine or iodine atoms, by $C_{1-3}$-alkyl or $C_{1-3}$-alkoxy groups and the substituents may be identical or different,

the $C_{1-3}$-alkanol esters, the tautomers, stereoisomers and salts thereof.

**4.** Benzimidazoles of general formula Ia according to claim 2, wherein

A denotes a methylene group,

B denotes an oxygen atom or an imino group,

$R_a$ denotes a cyclopropyl group substituted by the $R_1$-CO-group in the 1 position, wherein

R denotes a pyrrolidino or piperidino group optionally substituted by a methyl or ethyl group wherein each methyl or ethyl moiety may be substituted by a carboxy, carboxy-$C_{1-3}$-alkoxy, carboxy-$C_{1-3}$-alkylamino or N-($C_{1-3}$-alkyl)-carboxy-$C_{1-3}$-alkylamino group,

a cyclopropyl group substituted in the 1 position by the $R_2$-CX- group, wherein

$R_2$ denotes a phenyl, pyridyl, pyrazolyl group optionally substituted by a $C_{1-3}$-alkyl group and

X denotes an oxygen atom, a $C_{1-3}$-alkoxyimino or $C_{1-3}$-alkylidene group, each of which is substituted in the alkyl or alkoxy moiety by a carboxy group,

a $C_{1-2}$-alkyl group substituted in the 1 position by an imidazole group wherein the imidazole ring may be substituted by a phenyl or carboxy group and by one or two $C_{1-3}$-alkyl groups or by one, two or three $C_{1-3}$-alkyl groups, wherein the substituents may be identical or different and one of the abovementioned alkyl substituents may simultaneously be substituted by a carboxy group or may be substituted in the 2 or 3 position by an amino, $C_{2-4}$-alkanoylamino, $C_{1-3}$-alkylamino, N- ($C_{2-4}$-alkanoyl)-$C_{1-3}$-alkylamino or di- ($C_{1-3}$-alkyl)-amino group, whilst additionally a phenyl or pyridine ring may be fused to the abovementioned imidazole rings via two adjacent carbon atoms,

a $C_{1-2}$-alkyl substituted in the 1 position by a benzimidazolon-1-yl group, whilst the imidazolone ring may be substituted by a methyl or ethyl group optionally substituted by a carboxy group,

a methyl or ethyl group which is substituted in the 1 position

by an $R_3NR_4$ or $R_3NR_4$-$C_{1-3}$-alkyl group and

by a di-($C_{1-3}$-alkyl)-aminocarbonyl group, by a isoxazolidin-1-ylcarbonyl group, by a pyrrolidinocarbonyl or piperidinocarbonyl group substituted by a $C_{1-3}$-alkyl group, whilst in the abovementioned groups each alkyl moiety or alkyl substituent in the abovementioned groups may be substituted by a carboxy group, wherein

$R_3$ denotes a hydrogen atom or a $C_{1-3}$-alkyl group optionally substituted by a carboxy group and

$R_4$ denotes a hydrogen atom, a $C_{1-3}$-alkyl-$Y_2$ or carboxy-$C_{1-3}$-alkyl-$Y_2$ group or

$R_3$ and $R_4$ together with the nitrogen atom between them denote a 4- to 7-membered cycloalkyleneimino group optionally substituted by a carboxy group, wherein

$Y_2$ denotes a carbon-nitrogen bond, a carbonyl group or an imino group optionally substituted by a $C_{1-3}$-alkyl group,

a $C_{1-2}$-alkyl group substituted in the 1 position by an $R_5NR_6$- group, wherein

$R_5$ denotes a pyridinyl, phenylcarbonyl or phenylsulphonyl group and

$R_6$ denotes a $C_{1-3}$-alkyl or carboxy-$C_{1-3}$-alkyl group,

an n-propyl group substituted in the 3 position by a chlorine atom, which is substituted in the 1 position by a

cyclopentylcarbonyl group,
a cyclopropyl group substituted in the 1 position by a cyclopentylamino group, which is substituted at the nitrogen atom by a carboxy-$C_{1-3}$-alkylcarbonyl group,
$R_b$ denotes a methyl group and
$R_c$ denotes an amidino group which may optionally be substituted by a $C_{1-8}$-alkoxycarbonyl, acetoxymethyloxycarbonyl, 2,2,2-trichloroethoxycarbonyl, benzyloxycarbonyl or benzoyl group,
the $C_{1-3}$-alkanol esters thereof, the tautomers, stereoisomers and salts thereof.

**5.** Benzimidazoles of general formula Ia according to claim 2, wherein
A denotes a methylene group,
B denotes an imino group,
$R_a$ denotes a cyclopropyl group substituted by the $R_1$-CO-group in the 1 position, wherein
$R_1$ denotes a pyrrolidino or piperidino group optionally substituted by a methyl or ethyl group wherein each methyl or ethyl moiety may be substituted by a carboxy, carboxy-$C_{1-3}$-alkoxy, carboxy-$C_{1-3}$-alkylamino or N-($C_{1-3}$-alkyl)-carboxy-$C_{1-3}$-alkylamino group,
a cyclopropyl group substituted in the 1 position by the $R_2$-CX group, wherein
$R_2$ denotes a phenyl, pyridyl, pyrazolyl group optionally substituted by a $C_{1-3}$-alkyl group and
X denotes an oxygen atom, a $C_{1-3}$-alkoxyimino or $C_{1-3}$-alkylidene group, each of which is substituted in the alkyl or alkoxy moiety by a carboxy group,
a $C_{1-2}$-alkyl group substituted in the 1 position by an imidazole group wherein the imidazole ring may be substituted by one to three methyl groups or by two methyl groups and an ethyl group, whilst additionally one of the abovementioned methyl or ethyl substituents may simultaneously be substituted by a carboxy group,
a methyl or ethyl group which is substituted in the 1 position
by an $R_3NR_4$- or $R_3NR_4$-$CH_2$- group and
by a di-($C_{1-3}$-alkyl)-aminocarbonyl, by a pyrrolidinocarbonyl or piperidinocarbonyl group optionally substituted by a $C_{1-3}$-alkyl group, whilst in the abovementioned groups each alkyl moiety or alkyl substituent may be substituted by a carboxy group, wherein
$R_3$ denotes a hydrogen atom or a $C_{1-3}$-alkyl group optionally substituted by a carboxy group and
$R_4$ denotes a $C_{1-3}$-alkyl-$Y_2$ or carboxy- $C_{1-3}$-alkyl-$Y_2$ group wherein
$Y_2$ denotes a carbon-nitrogen bond, a carbonyl group or an imino group optionally substituted by a $C_{1-3}$-alkyl group,
$R_b$ denotes a methyl group and
$R_c$ denotes an amidino group which may optionally be substituted by a $C_{1-8}$-alkoxycarbonyl, acetoxymethyloxycarbonyl, 2,2,2-trichloroethoxycarbonyl, benzyloxycarbonyl or benzoyl group,
the $C_{1-3}$-alkanol esters thereof, the tautomers, stereoisomers and salts thereof.

**6.** Benzimidazoles of general formula I according to at least one of claims 1 to 5 wherein the group $R_a$ is in the 5 position,
the tautomers, stereoisomers and salts thereof.

**7.** The following compounds of general formula Ia:

(a) 2-(4-amidinophenylaminomethyl)-1-methyl-5-[1-(pyrrolidin-1-yl-carbonyl)cyclopropyl]-benzimidazole,

(b) (E/Z)-2-(4-amidinophenylaminomethyl)-1-methyl-5-[1-[(pyridin-2-yl)-(carboxymethyloxyimino)methylene]-cyclopropyl]-benzimidazole,

(c) 2-(4-amidinophenylaminomethyl)-1-methyl-5-[1-(2-carboxyethylamino)-1-(pyrrolidin-1-yl-carbonyl)-ethyl]-benzimidazole,

(d) 2-(4-amidinophenylaminomethyl)-1-methyl-5-[1-[2-(2-carboxyethyl)-pyrrolidin-1-yl-carbonyl]cyclopropyl]-benzimidazole,

(e) 2-(4-amidinophenylaminomethyl)-1-methyl-5-[2-(2-carboxyethyl)-4,5-dimethyl-imidazol-1-yl-methyl]-benzimidazole

(f) 2-(4-amidinophenylaminomethyl)-1-methyl-5-[1-(carboxymethylamino)-1-(pyrrolidin-1-yl-carbonyl)-ethyl]-benzimidazole and

(g) 2-(4-amidinophenylaminomethyl)-1-methyl-5-[1-(N-methyl carboxymethylcarbonylaminomethyl)-1-methyl-1-(pyrrolidin-1-yl-carbonyl)-ethyl]-benzimidazole

and the $C_{1-3}$-alkanolesters, the N-($C_{1-8}$-alkoxycarbonyl), N-benzyloxycarbonyl and N-benzoyl-amidines thereof, the tautomers, stereoisomers and salts thereof.

**8.** 2-(4-amidinophenylaminomethyl)-1-methyl-5-[1-(carboxymethylamino)-1-(pyrrolidin-1-yl-carbonyl)-ethyl]-benzimidazole, the $C_{1-3}$-alkanolesters thereof, and their N-($C_{1-8}$-alkoxycarbonyl), N-benzyloxycarbonyl and N-benzoylamidines, tautomers, stereoisomers and salts.

**9.** (R)-2-(4-amidinophenylaminomethyl)-1-methyl-5-[1-(carboxymethylamino)-1-(pyrrolidinocarbonyl)-ethyl]-benzimidazole, the $C_{1-3}$-alkanolesters thereof, and their N-($C_{1-8}$-alkoxycarbonyl), N-benzyloxycarbonyl- and N-benzoylamidines and salts.

**10.** Physiologically acceptable salts of the compounds according to claims 1 to 9 wherein $R_c$ denotes one of the amidino groups mentioned in claims 1 to 9.

**11.** Pharmaceutical compositions containing a compound according to at least one of claims 1 to 9 wherein $R_c$ denotes one of the amidino groups mentioned in claims 1 to 9, or a salt according to claim 10, optionally together with one or more inert carriers and/or diluents.

**12.** Use of a compound according to at least one of claims 1 to 9 wherein $R_c$ denotes one of the amidino groups mentioned in claims 1 to 9, or a salt according to claim 10, for preparing a pharmaceutical composition with a thrombin time prolonging effect, a thrombin-inhibiting effect and an inhibiting effect on related serine proteases.

**13.** Process for preparing a pharmaceutical composition according to claim 11, **characterised in that** a compound according to at least one of claims 1 to 9 wherein $R_c$ denotes one of the amidino groups mentioned in claims 1 to 9, or a salt according to claim 10, is incorporated in one or more inert carriers and/or diluents by a non-chemical method.

**14.** Process for preparing the compounds according to claims 1 to 10, **characterised in that**

a) in order to prepare a compound of general formula I wherein $R_c$ denotes a cyano group:

a compound of general formula

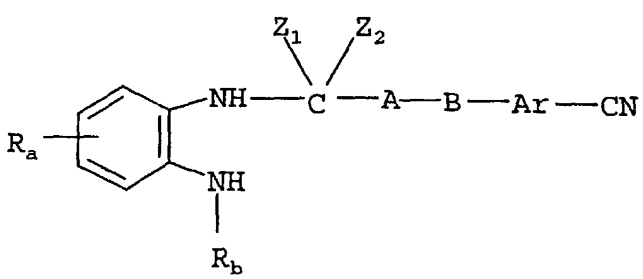

(II),

optionally formed in the reaction mixture,

wherein
$R_a$, $R_b$, Ar, A and B are defined as in claims 1 to 9,
$Z_1$ and $Z_2$, which may be identical or different, denote amino, hydroxy or mercapto groups optionally substituted by alkyl groups with 1 to 6 carbon atoms or
$Z_1$ and $Z_2$ together represent an oxygen or sulphur atom, an imino group optionally substituted by an alkyl group with 1 to 3 carbon atoms, an alkylenedioxy or alkylenedithio group with 2 or 3 carbon atoms, is cyclised, or

b) in order to prepare a compound of general formula I wherein $R_a$ denotes an $R_2$-CX'-$C_{3-5}$-cycloalkylene

group, wherein $R_2$ is defined as in claims 1 to 9 and X' denotes one of the imino groups mentioned for X in claims 1 to 9:

a compound of general formula

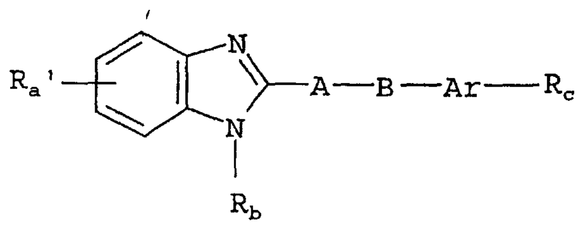

(IV),

wherein
$R_b$, $R_c$, Ar, A and B are defined as in claims 1 to 9 and
$R_a$' denotes an $R_2$-CO-$C_{3-5}$-cycloalkylene group, where
$R_2$ is defined as in claims 1 to 9,
is reacted with an amine of general formula

$H_2X'$ (V),

wherein
X' denotes one of the imino groups mentioned for X in claims 1 to 9, or

c) in order to prepare a compound of general formula I wherein $R_a$ denotes an $R_2$-CX"-$C_{3-5}$-cycloalkylene group,
wherein $R_2$ is defined as in claims 1 to 9 and X" denotes one of the alkylidene groups mentioned for X in claims 1 to 9, a compound of general formula

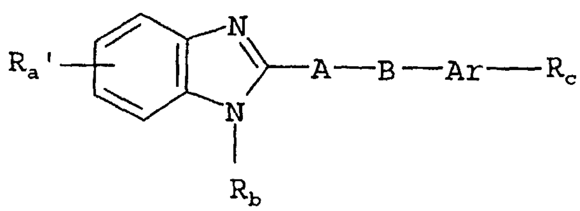

(IV),

wherein
$R_b$, $R_c$, Ar, A and B are defined as in claims 1 to 9 and
$R_a$' denotes an $R_2$-CO-$C_{3-5}$-cycloalkylene group, where
$R_2$ is defined as in claims 1 to 9,
is reacted with a phosphone of general formula

$Z_3$-HX" (VI),

wherein
X" denotes one of the alkylidene groups mentioned for X in claims 1 to 9 and
$Z_3$ denotes a triphenylphosphono or di-($C_{1-3}$-alkoxy)phosphono group, or

d) in order to prepare a compound of general formula I wherein $R_c$ denotes an amidino group which may be substituted by one or two $C_{1-3}$-alkyl groups:

a compound of general formula

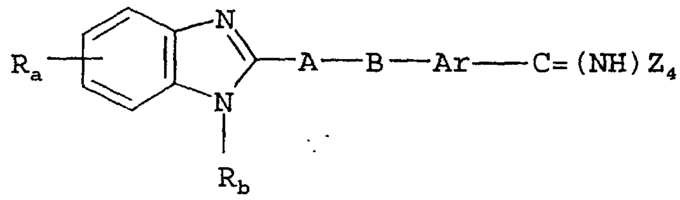

(VII),

optionally formed in the reaction mixture

wherein
$R_a$, $R_b$, Ar, A and B are defined as in claims 1 to 9 and
$Z_4$ denotes an alkoxy, aralkoxy, alkylthio or aralkylthio group, is reacted with an amine of general formula

$$H - R_7NR_8 , \qquad (VIII)$$

wherein
$R_7$ and $R_8$, which may be identical or different, each denote a hydrogen atom or a $C_{1-3}$-alkyl group, or with the salts thereof, or

e) in order to prepare a compound of general formula I wherein $R_a$ denotes an imidazolidin-2,4-dion-5-yl group which may be substituted by one or two $C_{1-3}$-alkyl groups, whilst at the same time an alkyl substituent may be substituted by a carboxy or $C_{1-3}$-alkoxycarbonyl group:

a compound of general formula

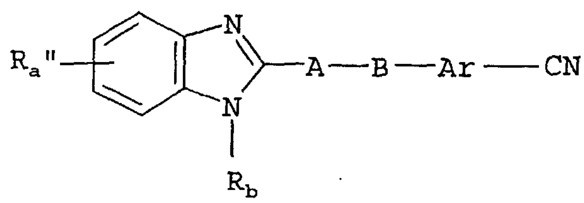

(IX),

optionally formed in the reaction mixture

wherein
$R_b$, Ar, A and B are defined as in claims 1 to 9 and
$R_a$" denotes an aminocarbonylamino group, substituted in the 3 position by a $C_{1-3}$-alkoxycarbonyl-$C_{1-3}$-alkyl group, is cyclised, or

f) in order to prepare a compound of general formula I wherein $R_c$ denotes a hydroxyamidino group:

a nitrile of general formula

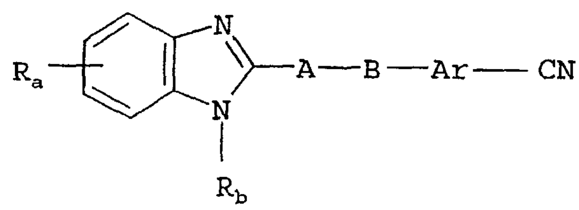 (X),

wherein

$R_a$, $R_b$, Ar, A and B are defined as in claims 1 to 9, is reacted with hydroxylamine or the salts thereof, or

g) in order to prepare a compound of general formula I wherein $R_a$ contains a carboxy group and $R_c$ is defined as in claims 1 to 9 or $R_a$ is defined as in claims 1 to 9 and $R_c$ denotes an amidino group optionally substituted by a hydroxy group or by one or two $C_{1-3}$-alkyl groups:

a compound of general formula

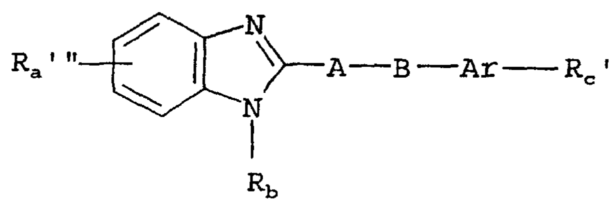 (XI),

wherein

$R_b$, Ar, A and B are defined as in claims 1 to 9 and

$R_a$''' and $R_c$' have the meanings given for $R_a$ and $R_c$ in claims 1 to 9, with the proviso that $R_a$ contains a group which may be converted into a carboxy group by hydrolysis, treatment with an acid or base, thermolysis or hydrogenolysis and $R_c$ is defined as in claims 1 to 9 or $R_c$ denotes a group which may optionally be converted by hydrolysis, treatment with an acid or base, thermolysis or hydrogenolysis into an amidino group substituted by a hydroxy group or by one or two $C_{1-3}$-alkyl groups and $R_a$ is defined as in claims 1 to 9,

is converted by hydrolysis, treatment with an acid or base, thermolysis or hydrogenolysis into a compound of general formula I wherein $R_a$ contains a carboxy group and $R_c$ is defined as in claims 1 to 9 or $R_a$ is defined as in claims 1 to 9 and $R_c$ denotes an amidino group optionally substituted by a hydroxy group or by one or two $C_{1-3}$-alkyl groups, or

h) in order to prepare a compound of general formula I wherein $R_c$ denotes an amidino group which is substituted by one or two $C_{1-8}$-alkoxycarbonyl groups or by a group which can be cleaved *in vivo*:

a compound of general formula I

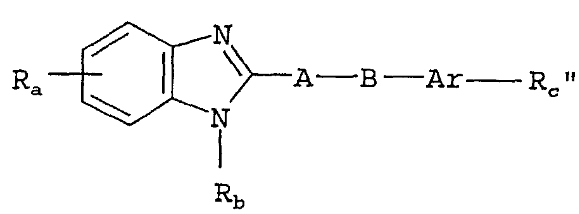 (XII),

wherein

$R_a$, $R_b$, Ar, A and B are defined as in claims 1 to 9 and

$R_c$'' denotes an amidino group, is reacted with a compound of general formula

$$Z_5 - R_9 \quad \text{(XIII)},$$

wherein

$R_9$ denotes a $C_{1-8}$-alkoxycarbonyl group or the acyl group of one of the groups which can be cleaved *in vivo* mentioned hereinbefore and

$Z_5$ denotes a nucleofugic leaving group, and

subsequently if desired a compound of general formula I thus obtained which contains an $(R_3NR_4)$-$C_{1-3}$-alkyl group in which at least one of the groups $R_3$ or $R_4$ denotes a hydrogen atom, is converted with a corresponding isocyanate or carbamoyl halide into a corresponding urea compound of general formula I, and/or

a compound of general formula I thus obtained which contains a $NH_2$-$C_{1-3}$-alkyl group is converted with a corresponding acrylic acid ester into a corresponding 2-($C_{1-3}$-alkoxycarbonyl)-ethyl compound of general formula I and/or

a compound of general formula I thus obtained which contains an $(R_3NR_4)$-$C_{1-3}$-alkyl group in which $R_3$ and $R_4$ each denote a hydrogen atom is converted with a corresponding dihaloalkane into a corresponding compound of general formula I wherein $R_3$ and $R_4$ together with the nitrogen atom between them denote a corresponding 4- to 7-membered cycloalkyleneimino group and/or

a compound of general formula I thus obtained wherein $R_c$ denotes an amidino group is converted with a haloacetic acid derivative and subsequent hydrolysis and decarboxylation into a corresponding amidino compound substituted by one or two methyl groups and/or

a compound of general formula I thus obtained wherein $R_c$ denotes a hydroxyamidino group is converted by catalytic hydrogenation into a corresponding amidino compound and/or

a compound of general formula I thus obtained wherein $R_a$ contains a carboxy group is converted by esterification into a corresponding ester and/or

a protecting group used to protect reactive groups during the reactions is cleaved and/or

a compound of general formula I thus obtained is resolved into its stereoisomers and/or

a compound of general formula I thus obtained is converted into the salts thereof, more particularly for pharmaceutical use into the physiologically acceptable salts thereof with an inorganic or organic acid or base.

## Revendications

**1.** Benzimidazoles de la formule générale :

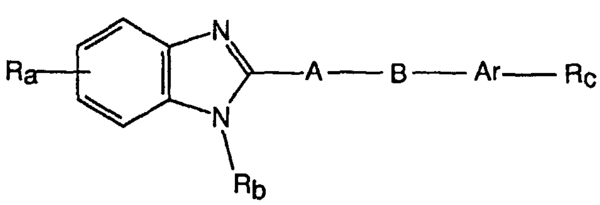

(I)

dans laquelle :

Ar représente un radical phénylène ou naphtylène le cas échéant substitué par un atome de fluor, de chlore ou de brome, par un radical trifluorométhyle, alkyle en $C_{1-3}$ ou alcoxy en $C_{1-3}$,

un radical thiénylène, thiazolylène, pyridinylène, pyrimidinylène, pyrazinylène ou pyridazinylène, le cas échéant substitué dans le squelette carboné, par un radical alkyle en $C_{1-3}$ ;

A représente un radical alkylène en $C_{1-3}$ ;

B représente un atome d'oxygène ou de soufre, un radical méthylène, carbonyle, sulfinyle ou sulfonyle, un radical imino le cas échéant substitué par un radical alkyle en $C_{1-3}$, dans lequel la partie alkyle peut être mono- ou disubstituée par un radical carboxy ;

$R_a$ représente un radical $R_1$-CO-(cycloalkyle en $C_{3-5}$), dans lequel :

$R_1$ représente un radical alcoxy en $C_{1-3}$, amino, (alkyl en $C_{1-4}$)amino ou di(alkyl en $C_{1-4}$)amino, dans

lesquels chacune des parties alkyle peut être substituée par un radical carboxy,
un radical cycloalkylènimino ou cycloalcénylènimino ayant 4 à 7 membres, qui peut être substitué par un ou deux radicaux alkyle en $C_{1-3}$, où un substituant alkyle peut être simultanément substitué par un radical hydroxy, alcoxy en $C_{1-3}$, carboxy, carboxy(alcoxy en $C_{1-3}$), carboxy(alkylamino en $C_{1-3}$), N-(alkyl en $C_{1-3}$) -N-carboxy(alkyl en $C_{1-3}$)amino, carboxy(alkylamino en $C_{1-3}$)carbonyle, -N(alkyl en $C_{1-3}$)-N-(carboxy(alkyl en $C_{1-3}$)aminocarbonyle, carboxy(alkylamino en $C_{1-3}$) carbonylamino, 1-(alkyl en $C_{1-3}$)-3-carboxy(alkyl en $C_{1-3}$) aminocarbonylamino, 3-(alkyl en $C_{1-3}$)-3-carboxy(alkyl en $C_{1-3}$)aminocarbonylamino ou 1,3-di(alkyl en $C_{1-3}$)-3-carboxy(alkyl en $C_{1-3}$)aminocarbonylamino,
un radical cycloalkylènimino ayant 4 à 7 membres, substitué par un radical hydroxy,
un radical cycloalkylènimino ayant 5 à 7 membres, le cas échéant substitué par un radical alkyle en $C_{1-3}$, sur lequel un cycle phényle est condensé sur deux atomes de carbone voisins,
un radical morpholino, pipérazino, N-(alkyl en $C_{1-3}$)pipérazino, pyrrolino, 3,4-déshydropipéridino ou pyrrol-1-yle ;
un radical $R_2$-CX-(cycloalkyle en $C_{3-5}$), dans lequel :

$R_2$ représente un radical phényle, naphtyle ou hétéroaryle monocyclique ayant 5 ou 6 membres le cas échéant substitué par un radical alkyle en $C_{1-3}$, où le radical hétéroaryle à 6 membres contient un, deux ou trois atomes d'azote et le radical hétéroaryle à 5 membres contient un radical imino le cas échéant substitué par un radical alkyle en $C_{1-3}$, un atome d'oxygène ou de soufre ou un radical imino le cas échéant substitué par un radical alkyle en $C_{1-3}$, et un atome d'oxygène ou de soufre ou un ou deux atomes d'azote, et le substituant alkyle mentionné précédemment peut être substitué par un radical carboxy, carboxy(alcoxy en $C_{1-3}$), carboxy(alkylamino en $C_{1-3}$), N-(alkyl en $C_{1-3}$)-N-carboxy (alkyl en $C_{1-3}$)amino, et
X représente un atome d'oxygène, un radical (alkyl en $C_{1-3}$)imino, (alcoxy en $C_{1-3}$)imino, (alkyl en $C_{1-3}$)hydrazino, di(alkyl en $C_{1-3}$)hydrazino, (alkanoyl en $C_{2-4}$)hydrazino, N-(alkyl en $C_{1-3}$)(alkanoyl en $C_{2-4}$)hydrazino, ou alkylidène en $C_{1-3}$, qui peuvent être chaque fois substitués, dans la partie alkyle ou alkanoyle ou dans les parties alkyle et alkanoyle, par un radical carboxy ;
un radical alkyle en $C_{1-3}$ ou cycloalkyle en $C_{3-5}$ substitué par un radical imidazole ou imidazolone, dans lequel
le cycle imidazole peut être substitué par un radical phényle ou carboxy et par un ou deux radicaux alkyle en $C_{1-3}$ ou par un, deux ou trois radicaux alkyle en $C_{1-3}$, où les substituants peuvent être identiques ou différents et l'un des substituants alkyle mentionnés précédemment peut être simultanément substitué par un radical carboxy ou en la position 2 ou 3, par un radical amino, (alcanoyl en $C_{2-4}$) amino, (alkyl en $C_{1-3}$)amino, N-(alcanoyl en $C_{2-4}$) (alkyl en $C_{1-3}$)amino, ou di(alkyl en $C_{1-3}$)amino, et
le cycle imidazolone peut être substitué par un radical alkyle en $C_{1-3}$, où le substituant alkyle peut être substitué par un radical carboxy ou en la position 2 ou 3, par un radical amino, (alcanoyl en $C_{2-4}$) amino, (alkyl en $C_{1-3}$)amino, N-(alcanoyl en $C_{2-4}$) (alkyl en $C_{1-3}$)amino, ou di(alkyl en $C_{1-3}$)amino, et en outre, sur les cycles imidazole et imidazolone indiqués précédemment, un cycle phényle ou pyridine peut être condensé sur deux atomes de carbone voisins ;
un radical imidazolidin-2,4-dion-5-yle, qui peut être substitué par un ou deux radicaux alkyle en $C_{1-3}$, où simultanément, un substituant alkyle peut être substitué par un radical carboxy ;
un radical alkyle en $C_{1-4}$, qui est substitué
par un radical (alkyl en $C_{1-3}$)-$Y_1$-(alkyle en $C_{1-3}$), HOOC-(alkyl en $C_{1-3}$)-$Y_1$-(alkyle en $C_{1-3}$), tétrazolyl-(alkyl en $C_{1-3}$)-$Y_2$-, $R_3NR_4$- ou $R_3NR_4$-(alkyle en $C_{1-3}$), et
par un radical isoxazolidinylcarbonyle le cas échéant substitué par un radical alkyle en $C_{1-3}$ ; par un radical pyrrolinocarbonyle, 3,4-déshydropipéridinocarbonyle, pyrrol-1-ylcarbonyle, carboxy, aminocarbonyle, (alkyl en $C_{1-3}$)aminocarbonyle, di(alkyl en $C_{1-3}$)aminocarbonyle ou (cycloalkylènimino à 4 à 7 membres)carbonyle, où dans les radicaux mentionnés précédemment, la partie cycloalkylènimino peut être substituée par un ou deux radicaux alkyle en $C_{1-3}$ et simultanément, chaque fois, une partie alkyle ou un substituant alkyle des radicaux (alkyle en $C_{1-3}$)aminocarbonyle, di(alkyle en $C_{1-3}$) aminocarbonyle ou cycloalkylèniminocarbonyle mentionnés précédemment, peut être substitué par un radical carboxy, et les atomes d'hydrogène restants des radicaux alkyle en $C_{1-4}$ peuvent être complètement ou partiellement remplacés par des atomes de fluor, dans lesquels
$R_3$ représente un atome d'hydrogène ou un radical alkyle en $C_{1-3}$ le cas échéant substitué par un radical carboxy, et
$R_4$ représente un atome d'hydrogène, un radical (alkyl en $C_{1-3}$)-$Y_1$-(alkyl en $C_{1-3}$)-$Y_2$-, carboxy-(alkyl en $C_{1-3}$)-$Y_1$-(alkyle en $C_{1-3}$)-$Y_2$-, (alkyl en $C_{1-3}$)-$Y_2$- ou carboxy-(alkyl en $C_{1-3}$)-$Y_2$-; ou
$R_3$ et $R_4$ représentent ensemble, avec l'atome d'azote situé entre eux, un radical cycloalkylènimino

ayant 4 à 7 membres, le cas échéant substitué par un radical carboxy, alkyle en $C_{1-3}$ ou carboxy (alkyle en $C_{1-3}$), dans lesquels
$Y_1$ représente une liaison carbone-carbone, un atome d'oxygène, un radical sulfényle, sulfinyle, sulfonyle, -NH-, -NH-CO- ou -NH-CO-NH-, et
$Y_2$ représente une liaison carbone-azote, ou un radical carbonyle, sulfonyle, imino ou -NH-CO-, où le radical carbonyle du radical -NH-CO- est relié par l'atome d'azote du radical $R_3NR_4$, et les radicaux imino présents dans la définition des restes $Y_1$ et $Y_2$ peuvent être chaque fois, substitués de manière supplémentaire, par un radical alkyle en $C_{1-3}$ ou carboxy(alkyle en $C_{1-3}$),
un radical alkyle en $C_{1-3}$ ou cycloalkyle en $C_{3-5}$, substitué par un radical $R_5NR_6$, dans lesquels
$R_5$ représente un atome d'hydrogène, un radical alkyle en $C_{1-3}$, cycloalkyle en $C_{5-7}$, phénylcarbonyle, phénylsulfonyle ou pyridinyle, et
$R_6$ représente un radical alkyle en $C_{1-3}$, carboxy(alkyle en $C_{1-3}$) ou carboxy(alkyl en $C_{1-3}$)carbonyle ;
un radical alkyle en $C_{1-3}$, qui est substitué par un radical alcanoyle en $C_{2-4}$ ou cycloalkanoyle en $C_{5-7}$ et par un radical alkyle en $C_{1-3}$ substitué par un atome de chlore, de brome ou d'iode ;
$R_b$ représente un atome d'hydrogène ou un radical alkyle en $C_{1-3}$ ;
$R_c$ représente un radical cyano ou un radical amidino le cas échéant substitué par un ou deux radicaux alkyle en $C_{1-3}$, où
les radicaux carboxy mentionnés dans la définition des restes indiqués précédemment, peuvent être remplacés en outre, par un radical convertissable in vivo en un radical carboxy ou par un radical chargé négativement dans les conditions physiologiques, ou
les radicaux amino et imino mentionnés dans la définition des restes indiqués précédemment, peuvent être en outre, substitués par un reste clivable in vivo, où
par reste convertissable in vivo en radical carboxy, on entend un radical hydroxyméthyle, un reste carboxy estérifié avec un alcool, dans lequel la partie alcoolique est un alcanol en $C_{1-6}$, un phényl (alcanol en $C_{1-3}$), un cyclalcanol en $C_{3-9}$, où un cycloalcanol en $C_{5-8}$ peut être en outre substitué par un ou deux radicaux alkyle en $C_{1-3}$, un cyclalcanol en $C_{5-8}$, dans lequel un radical méthylène en position 3 ou 4 est remplacé par un atome d'oxygène ou un radical imino le cas échéant substitué par un radical alkyle en $C_{1-3}$, phényl(alkyle en $C_{1-3}$), phényl(alcoxy en $C_{1-3}$)carbonyle ou alcanoyle en $C_{2-6}$, et la partie cycloalcanol peut être substituée en outre, par un ou deux radicaux alkyle en $C_{1-3}$, un cycloalcénol en $C_{4-7}$, un alcénol en $C_{3-5}$, un phényl(alcénol en $C_{3-5}$), un alcynol en $C_{3-5}$ ou un phényl(alcynol en $C_{3-5}$), avec la condition qu'aucune liaison sur l'atome d'oxygène parte d'un atome de carbone, qui porte une double ou triple liaison, un (cycloalkyl en $C_{3-8}$)alcanol en $C_{1-3}$, un bicycloalcanol avec au total, 8 à 10 atomes de carbone, qui peut être en outre substitué dans la partie bicycloalkyle, par un ou deux radicaux alkyle en $C_{1-3}$, un 1,3-dihydro-3-oxo-1-isobenzfurannol ou un alcool de la formule :

$$Rd - CO - O - (R_eCR_f) - OH$$

dans laquelle:

$R_d$ représente un radical alkyle en $C_{1-8}$, cycloalkyle en $C_{5-7}$, phényle ou phényl(alkyle en $C_1$-$C_3$);
$R_e$ représente un atome d'hydrogène, un radical alkyle en $C_{1-3}$, cycloalkyle en $C_{5-7}$ ou phényle, et
$R_f$ représente un atome d'hydrogène ou un radical alkyle en $C_{1-3}$ ;
par groupe chargé négativement dans les conditions physiologiques, on entend un radical tétrazol-5-yle, phénylcarbonylaminocarbonyle, trifluorométhylcarbonylaminocarbonyle, (alkyl en $C_{1-6}$)sulfonylamino, phénylsulfonylamino, benzylsulfonylamino, trifluorométhylsulfonylamino, (alkyl en $C_{1-6}$)sulfonylaminocarbonyle, phénylsulfonylaminocarbonyle, benzylsulfonylaminocarbonyle ou (perfluoroalkyl en $C_{1-6}$)sulfonylaminocarbonyle, et
par reste clivable in vivo en un reste imino ou amino, on entend un radical hydroxy, un radical benzoyle le cas échéant mono- ou disubstitué par l'atome de fluor, de chlore, de brome ou d'iode, par un radical alkyle en $C_{1-3}$ ou alcoxy en $C_{1-3}$, où les substituants peuvent être identiques ou différents, un radical pyridinoyle ou un radical alcanoyle en $C_{1-16}$, un radical 3,3,3-trichloropropionyle ou allyloxycarbonyle, un radical (alcoxy en $C_{1-16}$)carbonyle ou (alkyle en $C_{1-16}$)carbonyloxy, dans lesquels les atomes d'hydrogène peuvent être totalement ou partiellement remplacés par des atomes de fluor ou de chlore, un radical phényl(alcoxy en $C_{1-6}$)carbonyle, un radical 3-aminopropionyle, dans lequel le radical amino peut être mono- ou disubstitué par des radicaux alkyle en $C_{1-6}$ ou cycloalkyle en $C_{3-7}$ et les substituants peuvent être identiques ou différents, un radical (alkyl en $C_{1-3}$)sulfonyl(alcoxy en $C_{2-4}$)carbo-

nyle, (alcoxy en $C_{1-3}$)(alcoxy en $C_{2-4}$)(alcoxy en $C_{2-4}$)carbonyle, $R_d$-CO-O-($R_dCR_f$)-O-CO-, (alkyl en $C_{1-6}$)-CO-NH-($R_gCR_h$)-O-CO- ou (alkyl en $C_{1-6}$)-CO-O-($R_gCR_h$)-($R_gCR_h$)-O-CO-, dans lesquels $R_d$ à $R_f$ sont définis comme indiqué précédemment, et

$R_g$ et $R_h$, qui peuvent être identiques ou différents, représentent l'atome d'hydrogène ou un radical alkyle en $C_{1-3}$,

leurs tautomères, leurs stéréoisomères, leurs mélanges et leurs sels.

**2.** Benzimidazoles de formule générale :

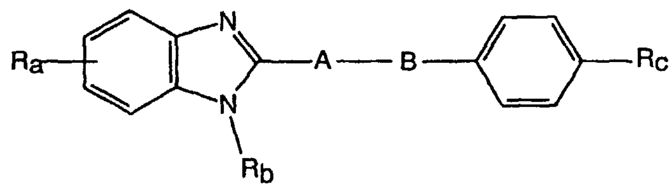

(Ia)

dans laquelle :

A représente un radical alkylène en $C_{1-3}$ ;

B représente un atome d'oxygène ou de soufre, un radical méthylène, carbonyle, sulfinyle ou sulfonyle, un radical imino le cas échéant substitué par un radical alkyle en $C_{1-3}$, dans lequel la partie alkyle peut être mono- ou disubstituée par un radical carboxy ;

$R_a$ représente un radical $R_1$-CO-(cycloalkyle en $C_{3-5}$), dans lequel :

$R_1$ représente un radical alcoxy en $C_{1-3}$, amino, (alkyl en $C_{1-4}$)amino ou di(alkyl en $C_{1-4}$)amino, dans lesquels chacune des parties alkyle peut être substituée par un radical carboxy,

un radical cycloalkylènimino ou cycloalcénylènimino ayant 4 à 7 membres, qui peut être substitué par un ou deux radicaux alkyle en $C_{1-3}$, où un substituant alkyle peut être simultanément substitué par un radical hydroxy, alcoxy en $C_{1-3}$, carboxy, carboxy(alcoxy en $C_{1-3}$), carboxy(alkylamino en $C_{1-3}$), N-(alkyl en $C_{1-3}$) -N-carboxy(alkyl en $C_{1-3}$)amino, carboxy(alkylamino en $C_{1-3}$)carbonyle, N-(alkyl en $C_{1-3}$)-N-carboxy(alkyl en $C_{1-3}$)aminocarbonyle, carboxy(alkylamino en $C_{1-3}$)carbonylamino, 1-(alkyl en $C_{1-3}$)-3-carboxy(alkyl en $C_{1-3}$)aminocarbonylamino, 3-(alkyl en $C_{1-3}$)-3-carboxy(alkyl en $C_{1-3}$)aminocarbonylamino ou 1,3-di(alkyl en $C_{1-3}$)-3-carboxy(alkyl en $C_{1-3}$)aminocarbonylamino,

un radical cycloalkylènimino ayant 4 à 7 membres, substitué par un radical hydroxy,

un radical cycloalkylènimino ayant 5 à 7 membres, le cas échéant substitué par un radical alkyle en $C_{1-3}$, sur lequel un cycle phényle est condensé par deux atomes de carbone voisins,

un radical morpholino, pipérazino, N-(alkyl en $C_{1-3}$)pipérazino, pyrrolino, 3,4-déshydropipéridino ou pyrrol-1-yle ;

un radical $R_2$-CX-(cycloalkyle en $C_{3-5}$), dans lequel :

$R_2$ représente un radical phényle, naphtyle ou hétéroaryle monocyclique ayant 5 ou 6 membres le cas échéant substitué par un radical alkyle en $C_{1-3}$, où le radical hétéroaryle à 6 membres contient un, deux ou trois atomes d'azote et le radical hétéroaryle à 5 membres contient un radical imino le cas échéant substitué par un radical alkyle en $C_{1-3}$, un atome d'oxygène ou de soufre ou un radical imino le cas échéant substitué par un radical alkyle en $C_{1-3}$, et un atome d'oxygène ou de soufre ou un ou deux atomes d'azote, et le substituant alkyle mentionné précédemment peut être substitué par un radical carboxy, carboxy(alcoxy en $C_{1-3}$), carboxy(alkylamino en $C_{1-3}$) ou N-(alkyl en $C_{1-3}$)-N-carboxy(alkyl en $C_{1-3}$)amino, et

X représente un atome d'oxygène, un radical (alkyl en $C_{1-3}$)imino, (alcoxy en $C_{1-3}$)imino, (alkyl en $C_{1-3}$)hydrazino, di(alkyl en $C_{1-3}$)hydrazino, (alkanoyl en $C_{2-4}$)hydrazino, N-(alkyl en $C_{1-3}$) (alkanoyl en $C_{2-4}$)hydrazino, ou alkylidène en $C_{1-3}$, qui peuvent être chaque fois substitués, dans la partie alkyle ou alkanoyle ou dans les parties alkyle et alkanoyle, par un radical carboxy ;

un radical alkyle en $C_{1-3}$ ou cycloalkyle en $C_{3-5}$ substitué par un radical imidazole ou imidazolone, dans lequel

le cycle imidazole peut être substitué par un radical phényle ou carboxy et par un ou deux radicaux alkyle en $C_{1-3}$ ou par un, deux ou trois radicaux alkyle en $C_{1-3}$, où les substituants peuvent être identiques ou différents et l'un des substituants alkyle mentionnés précédemment peut être simultanément substitué par un radical carboxy ou en la position 2 ou 3, par un radical amino, (alcanoyl en $C_{2-4}$) amino, (alkyl en $C_{1-3}$)amino, N-(alcanoyl en $C_{2-4}$) (alkyl en $C_{1-3}$)amino, ou di(alkyl en $C_{1-3}$)amino, et le cycle imidazolone peut être substitué par un radical alkyle en $C_{1-3}$, où le substituant alkyle peut être substitué par un radical carboxy ou en la position 2 ou 3, par un radical amino, (alcanoyl en $C_{2-4}$) amino, (alkyl en $C_{1-3}$)amino, N-(alcanoyl en $C_{2-4}$) (alkyl en $C_{1-3}$)amino, ou di(alkyl en $C_{1-3}$)amino, et en outre, sur les cycles imidazole et imidazolone indiqués précédemment, un cycle phényle ou pyridine peut être condensé par deux atomes de carbone voisins ;

un radical imidazolidin-2,4-dion-5-yle, qui peut être substitué par un ou deux radicaux alkyle en $C_{1-3}$, où simultanément, un substituant alkyle peut être substitué par un radical carboxy ;

un radical alkyle en $C_{1-4}$, qui est substitué

par un radical (alkyl en $C_{1-3}$)-$Y_1$-(alkyle en $C_{1-3}$), HOOC-(alkyl en $C_{1-3}$)-$Y_1$-(alkyle en $C_{1-3}$), tétrazolyl-(alkyl en $C_{1-3}$)-$Y_2$-, $R_3NR_4$- ou $R_3NR_4$-(alkyle en $C_{1-3}$), et

par un radical isoxazolidin-1-ylcarbonyle le cas échéant substitué par un radical alkyle en $C_{1-3}$, par un radical pyrrolinocarbonyle, 2,3-déshydropipéridinocarbonyle, pyrrol-1-ylcarbonyle, carboxy, aminocarbonyle, (alkyl en $C_{1-3}$)aminocarbonyle, di(alkyl en $C_{1-3}$)aminocarbonyle ou (cycloalkylènimino à 4 à 7 membres)carbonyle, où dans les radicaux mentionnés précédemment, la partie cycloalkylènimino peut être substituée par un ou deux radicaux alkyle en $C_{1-3}$ et simultanément, chaque fois, une partie alkyle ou un substituant alkyle des radicaux (alkyle en $C_{1-3}$)aminocarbonyle, di(alkyle en $C_{1-3}$) aminocarbonyle ou cycloalkylèniminocarbonyle mentionnés précédemment, peut être substitué par un radical carboxy, et les atomes d'hydrogène restants des radicaux alkyle en $C_{1-4}$ peuvent être complètement ou partiellement remplacés par des atomes de fluor, dans lesquels

$R_3$ représente un atome d'hydrogène ou un radical alkyle en $C_{1-3}$ le cas échéant substitué par un radical carboxy, et

$R_4$ représente un atome d'hydrogène, un radical (alkyl en $C_{1-3}$)-$Y_1$-(alkyl en $C_{1-3}$)-$Y_2$-, carboxy-(alkyl en $C_{1-3}$)-$Y_1$-(alkyle en $C_{1-3}$)-$Y_2$-, (alkyl en $C_{1-3}$)-$Y_2$- ou carboxy(alkyl en $C_{1-3}$)-$Y_2$-, ou

$R_3$ et $R_4$ représentent ensemble, avec l'atome d'azote situé entre eux, un radical cycloalkylènimino ayant 4 à 7 membres, le cas échéant substitué par un radical carboxy, alkyle en $C_{1-3}$ ou carboxy (alkyle en $C_{1-3}$), dans lesquels

$Y_1$ représente une liaison carbone-carbone, un atome d'oxygène, un radical sulfényle, sulfinyle, sulfonyle, -NH-, -NH-CO- ou -NH-CO-NH-, et

$Y_2$ représente une liaison carbone-azote, ou un radical carbonyle, sulfonyle, imino ou -NH-CO-, où le radical carbonyle du radical -NH-CO- est relié par l'atome d'azote du radical $R_3NR_4$, et les radicaux imino présents dans la définition des restes $Y_1$ et $Y_2$ peuvent être chaque fois, substitués de manière supplémentaire, par un radical alkyle en $C_{1-3}$ ou carboxy(alkyle en $C_{1-3}$),

un radical alkyle en $C_{1-3}$ ou cycloalkyle en $C_{3-5}$, substitué par un radical $R_5NR_6$, dans lesquels

$R_5$ représente un atome d'hydrogène, un radical alkyle en $C_{1-3}$, cycloalkyle en $C_{5-7}$, phénylcarbonyle, phénylsulfonyle ou pyridinyle, et

$R_6$ représente un radical alkyle en $C_{1-3}$, carboxy(alkyle en $C_{1-3}$) ou carboxy(alkyl en $C_{1-3}$)carbonyle ;

un radical alkyle en $C_{1-3}$, qui est substitué par un radical alcanoyle en $C_{2-4}$ ou cycloalkanoyle en $C_{5-7}$ et par un radical alkyle en $C_{1-3}$ substitué par un atome de chlore, de brome ou d'iode ;

$R_b$ représente un atome d'hydrogène ou un radical alkyle en $C_{1-3}$ ;

$R_c$ représente un radical cyano ou un radical amidino qui peut être substitué par un radical hydroxy, par un ou deux radicaux alkyle en $C_{1-3}$, par un ou deux radicaux (alcoxy en $C_{1-6}$)carbonyle,

où les radicaux carboxy, amino et imino mentionnés dans la définition des restes indiqués précédemment, peuvent être substitués en outre, par un reste clivable in vivo comme défini à la revendication 1,

leurs tautomères, leurs stéréoisomères et leurs sels.

**3.** Benzimidazoles de la formule générale (Ia) selon la revendication 2, dans laquelle :

A représente un radical alkylène en $C_{1-3}$ ;

B représente un atome d'oxygène, un radical méthylène, imino ou N-(alkyle en $C_{1-3}$)imino, dans lequel la partie alkyle peut être substituée par un radical carboxy ;

$R_a$ représente un radical cycloalkyle en $C_{3-5}$ substitué en position 1 par le reste $R_1$-CO-, dans lequel :

$R_1$ représente un radical alcoxy en $C_{1-3}$, amino, (alkyl en $C_{1-4}$)amino ou di(alkyl en $C_{1-4}$)amino, dans lesquels chacune des parties alkyle peut être substituée par un radical carboxy,
un radical cycloalkylènimino ayant 4 à 7 membres, qui peut être substitué par un radical hydroxy ou par un ou deux radicaux alkyle en $C_{1-3}$, où un substituant alkyle peut être simultanément substitué par un radical hydroxy, alcoxy en $C_{1-3}$, carboxy, carboxy(alcoxy en $C_{1-3}$), carboxy(alkylamino en $C_{1-3}$), N-(alkyl en $C_{1-3}$)-N-carboxy(alkyl en $C_{1-3}$)amino, carboxy(alkylamino en $C_{1-3}$)carbonyle, -N(alkyl en $C_{1-3}$)-N-(carboxy(alkyl en $C_{1-3}$)aminocarbonyle, carboxy(alkylamino en $C_{1-3}$)carbonylamino, 1-(alkyl en $C_{1-3}$)-3-carboxy(alkyl en $C_{1-3}$)aminocarbonylamino, 3-(alkyl en $C_{1-3}$)-3-carboxy(alkyl en $C_{1-3}$)aminocarbonylamino ou 1,3-di(alkyl en $C_{1-3}$)-3-carboxy(alkyl en $C_{1-3}$) aminocarbonylamino,
un radical cycloalkylènimino ayant 5 à 7 membres, le cas échéant substitué par un radical alkyle en $C_{1-3}$, sur lequel un cycle phényle est condensé par deux atomes de carbone voisins,
un radical morpholino, pipérazino, N-(alkyl en $C_{1-3}$)pipérazino, pyrrolino, 3,4-déshydropipéridino ou pyrrol-1-yle ;
un radical cycloalkyle en $C_{3-5}$ substitué en position 1 par le reste $R_2$-CX-, dans lequel :

$R_2$ représente un radical phényle, naphtyle ou hétéroaryle monocyclique ayant 5 ou 6 membres, le cas échéant substitué par un radical alkyle en $C_{1-3}$, où le radical hétéroaryle à 6 membres contient un, deux ou trois atomes d'azote et le radical hétéroaryle à 5 membres contient un radical imino la cas échéant substitué par un radical alkyle en $C_{1-3}$, un atome d'oxygène ou de soufre ou un radical imino le cas échéant substitué par un radical alkyle en $C_{1-3}$, et un atome d'oxygène ou de soufre ou un ou deux atomes d'azote, et le substituant alkyle mentionné précédemment peut être substitué par un radical carboxy, carboxy(alcoxy en $C_{1-3}$), carboxy(alkylamino en $C_{1-3}$) ou N-(alkyl en $C_{1-3}$)-N-carboxy(alkyl en $C_{1-3}$)amino, et
X représente un atome d'oxygène, un radical (alkyl en $C_{1-3}$)imino, (alcoxy en $C_{1-3}$)imino ou alkylidène en $C_{1-3}$, qui peuvent être chaque fois substitués, dans la partie alkyle ou alkoxy par un radical carboxy ;
un radical alkyle en $C_{1-3}$ substitué en position 1, par un radical imidazole ou imidazolone, dans lequel le cycle imidazole peut être substitué par un radical phényle ou carboxy et par un ou deux radicaux alkyle en $C_{1-3}$ ou par un, deux ou trois radicaux alkyle en $C_{1-3}$, où les substituants peuvent être identiques ou différents et l'un des substituants alkyle mentionnés précédemment peut être simultanément substitué par un radical carboxy ou en la position 2 ou 3, par un radical amino, (alcanoyl en $C_{2-4}$) amino, (alkyl en $C_{1-3}$)amino, N-(alcanoyl en $C_{2-4}$)(alkyl en $C_{1-3}$)amino, ou di(alkyl en $C_{1-3}$)amino, et
le cycle imidazolone peut être substitué par un radical alkyle en $C_{1-3}$, où le substituant alkyle peut être substitué par un radical carboxy ou en la position 2 ou 3, par un radical amino, (alcanoyl en $C_{2-4}$) amino, (alkyl en $C_{1-3}$)amino, N-(alcanoyl en $C_{2-4}$)(alkyl en $C_{1-3}$)amino, ou di(alkyl en $C_{1-3}$)amino, et
en outre, sur les cycles imidazole et imidazolone indiqués précédemment, un cycle phényle ou pyridine peut être condensé sur deux atomes de carbone voisins ;
un radical imidazolidin-2,4-dion-5-yle, qui peut être substitué par un ou deux radicaux alkyle en $C_{1-3}$, où simultanément, un substituant alkyle peut être substitué par un radical carboxy ;
un radical alkyle en $C_{1-4}$, qui est substitué en position 1
par un radical $R_3NR_4$- ou $R_3NR_4$-(alkyle en $C_{1-3}$), et
par un radical pyrrolinocarbonyle, 2,3-déshydropipéridinocarbonyle, imidazol-1-ylcarbonyle, carboxy, aminocarbonyle, (alkyl en $C_{1-3}$)aminocarbonyle, di(alkyl en $C_{1-3}$)aminocarbonyle, isoxazolidin-1-ylcarbonyle ou (cycloalkylènimino à 4 à 7 membres)-carbonyle, où dans les radicaux mentionnés précédemment, la partie cycloalkylènimino peut être substituée par un ou deux radicaux alkyle en $C_{1-3}$ et simultanément, chaque fois, une partie alkyle ou un substituant alkyle des radicaux (alkyle en $C_{1-3}$) aminocarbonyle, di(alkyle en $C_{1-3}$)aminocarbonyle ou cycloalkylèniminocarbonyle mentionnés précédemment, peut être substitué par un radical carboxy, et les atomes d'hydrogène restants des radicaux alkyle en $C_{1-4}$ peuvent être complètement ou partiellement remplacés par des atomes de fluor, dans lesquels
$R_3$ représente un atome d'hydrogène ou un radical alkyle en $C_{1-3}$ le cas échéant substitué par un radical carboxy, et
$R_4$ représente un atome d'hydrogène, un radical (alkyl en $C_{1-3}$)-$Y_2$- ou carboxy-(alkyl en $C_{1-3}$)-$Y_2$-, ou $R_3$ et $R_4$ représentent ensemble, avec l'atome d'azote situé entre eux, un radical cycloalkylènimino ayant 4 à 7 membres, le cas échéant substitué en position 1 par un radical carboxy, alkyle en $C_{1-3}$ ou carboxy(alkyle en $C_{1-3}$), dans lesquels
$Y_2$ représente une liaison carbone-azote, ou un radical carbonyle, imino ou -NH-CO-, où le radical carbonyle du radical -NH-CO- est relié par l'atome d'azote du radical $R_3NR_4$, et les radicaux imino présents dans la définition du reste $Y_2$ peuvent être chaque fois, substitués de manière supplémentaire, par un radical alkyle en $C_{1-3}$ ou carboxy(alkyle en $C_{1-3}$),
un radical alkyle en $C_{1-3}$ ou cycloalkyle en $C_{3-5}$, substitué en position 1 par un radical $R_5NR_6$, dans lesquels
$R_5$ représente un atome d'hydrogène, un radical alkyle en $C_{1-3}$, cycloalkyle en $C_{5-7}$, phénylcarbonyle, phénylsulfonyle ou pyridinyle, et
$R_6$ représente un radical alkyle en $C_{1-3}$, carboxy(alkyle en $C_{1-3}$) ou carboxy(alkyl en $C_{1-3}$)carbonyle ;
un radical alkyle en $C_{1-3}$, qui est substitué par un radical alcanoyle en $C_{2-4}$ ou cycloalkanoyle en $C_{5-7}$ et par un radical alkyle en $C_{1-3}$ substitué par un atome de chlore, de brome ou d'iode ;
$R_b$ représente un radical alkyle en $C_{1-3}$, et
$R_c$ représente un radical amidino le cas échéant substitué par un radical 2,2,2-trichloroéthoxycarbonyle, (alcoxy en $C_{1-8}$)carbonyle, acétoxyméthyloxycarbonyle, benzyloxycarbonyle ou benzoyle, où la partie benzoyle peut être mono- ou disubstituée par un atome de fluor, de chlore, de brome ou d'iode, par un radical alkyle en $C_{1-3}$ ou alcoxy en $C_{1-3}$ et les substituants peuvent être identiques ou différents,

leurs esters d'alcanol en $C_{1-3}$, leurs tautomères, leurs stéréoisomères et leurs sels.

**4.** Benzimidazoles de la formule générale (Ia) selon la revendication 2, dans laquelle :

A représente un radical méthylène ;
B représente un atome d'oxygène ou un radical imino ;
$R_a$ représente un radical cyclopropyle substitué en position 1 par le reste $R_1$-CO-, dans lequel :

$R_1$ représente un radical pyrrolidino ou pipéridino, le cas échéant substitué par un radical méthyle ou éthyle, où la partie méthyle ou éthyle peut être chaque fois substituée par un radical carboxy, carboxy (alcoxy en $C_{1-3}$), carboxy(alkyl en $C_{1-3}$)amino ou N-(alkyl en $C_{1-3}$)-carboxy(alkyl en $C_{1-3}$)amino,
un radical cyclopropyle substitué en position 1 par le reste $R_2$-CX-, dans lequel :
$R_2$ représente un radical phényle, pyridyle, pyrazolyle, le cas échéant substitué par un radical alkyle en $C_{1-3}$, et
X représente un atome d'oxygène, un radical (alcoxy en $C_{1-3}$)imino ou alkylidène en $C_{1-3}$, qui peuvent être chaque fois substitués, dans la partie alkyle ou alkoxy par un radical carboxy ;
un radical alkyle en $C_{1-3}$ substitué en position 1 par un radical imidazole, dans lequel le cycle imidazole peut être substitué par un radical phényle ou carboxy et par un ou deux radicaux alkyle en $C_{1-3}$ ou par un, deux ou trois radicaux alkyle en $C_{1-3}$, où les substituants peuvent être identiques ou différents et l'un des substituants alkyle mentionnés précédemment peut être simultanément substitué par un radical carboxy ou en la position 2 ou 3, par un radical amino, (alcanoyl en $C_{2-4}$)amino, (alkyl en $C_{1-3}$)amino, N-(alcanoyl en $C_{2-4}$) (alkyl en $C_{1-3}$)amino, ou di(alkyl en $C_{1-3}$)amino, où en outre, sur le cycle imidazole indiqué précédemment, un cycle phényle ou pyridine peut être condensé sur deux atomes de carbone voisins ;
un radical alkyle en $C_{1-2}$ substitué en position 1 par un radical benzimidazolon-1-yle, où le cycle imidazolone peut être substitué par un radical méthyle ou éthyle le cas échéant substitué par un radical carboxy ;
un radical méthyle ou éthyle, qui est substitué en position 1
par un radical $R_3NR_4$- ou $R_3NR_4$-(alkyle en $C_{1-3}$), et
par un radical di(alkyl en $C_{1-3}$)aminocarbonyle, par un radical isoxazolidin-1-ylcarbonyle, par un radical pyrrolidinocarbonyle ou pipéridinocarbonyle le cas échéant substitué par un radical alkyle en $C_{1-3}$, où dans les radicaux mentionnés précédemment, chaque fois, une partie alkyle ou un substituant alkyle peut être substitué par un radical carboxy, dans lesquels
$R_3$ représente un atome d'hydrogène ou un radical alkyle en $C_{1-3}$ le cas échéant substitué par un radical carboxy, et
$R_4$ représente un atome d'hydrogène, un radical (alkyl en $C_{1-3}$)-$Y_2$- ou carboxy-(alkyl en $C_{1-3}$)-$Y_2$-, ou
$R_3$ et $R_4$ représentent ensemble, avec l'atome d'azote situé entre eux, un radical cycloalkylènimino ayant 4 à 7 membres, le cas échéant substitué par un radical carboxy, dans lesquels
$Y_2$ représente une liaison carbone-azote, un radical carbonyle ou un radical imino le cas échéant substitué par un radical alkyle en $C_{1-3}$,
un radical alkyle en $C_{1-2}$ substitué en position 1 par un radical $R_5NR_6$, dans lequel
$R_5$ représente un radical pyridinyle, phénylcarbonyle ou phénylsulfonyle, et
$R_6$ représente un radical alkyle en $C_{1-3}$, carboxy(alkyle en $C_1$-$C_3$);
un radical n-propyle substitué en position 3 par un atome de chlore, qui est substitué en position 1 par un radical cyclopentylcarbonyle,
un radical cyclopropyle substitué en position 1 par un radical cyclopentylamino, qui est substitué sur l'atome d'azote par un radical carboxy(alkyl en $C_{1-3}$)carbonyle ;
$R_b$ représente un radical méthyle, et
$R_c$ représente un radical amidino le cas échéant substitué par un radical (alcoxy en $C_{1-8}$)carbonyle, acétoxyméthyloxycarbonyle, 2,2,2-trichloroéthoxycarbonyle, benzyloxycarbonyle ou benzoyle,

leurs esters d'alcanol en $C_{1-3}$, leurs tautomères, leurs stéréoisomères et leurs sels.

**5.** Benzimidazoles de la formule générale (Ia) selon la revendication 2, dans laquelle :

A représente un radical méthylène ;
B représente un radical imino ;
$R_a$ représente un radical cyclopropyle substitué en position 1 par le reste $R_1$-CO-, dans lequel :

$R_1$ représente un radical pyrrolidino ou pipéridino, le cas échéant substitué par un radical méthyle ou éthyle, où la partie méthyle ou éthyle peut être chaque fois substituée par un radical carboxy, carboxy (alcoxy en $C_{1-3}$), carboxy(alkyl en $C_{1-3}$)amino ou N-(alkyl en $C_{1-3}$)-carboxy(alkyl en $C_{1-3}$)amino,
un radical cyclopropyle substitué en position 1 par le reste $R_2$-CX-, dans lequel :

$R_2$ représente un radical phényle, pyridyle, pyrazolyle, le cas échéant substitué par un radical alkyle en $C_{1-3}$, et
X représente un atome d'oxygène, un radical (alcoxy en $C_{1-3}$)imino ou alkylidène en $C_{1-3}$, qui sont chaque fois substitués, dans la partie alkyle ou alkoxy par un radical carboxy ;
un radical alkyle en $C_{1-2}$ substitué en position 1 par un radical imidazole, dans lequel le cycle imidazole peut être substitué par 1 à 3 radicaux méthyle ou par deux radicaux méthyle et un radical éthyle, où l'un des substituants méthyle ou éthyle mentionnés précédemment peut être simultanément substitué par un radical carboxy ;
un radical méthyle ou éthyle, qui est substitué en position 1
par un radical $R_3NR_4$- ou $R_3NR_4$-($CH_2$)-, et
par un radical di(alkyl en $C_{1-3}$)aminocarbonyle, un radical pyrrolidinocarbonyle ou pipéridinocarbonyle le cas échéant substitué par un radical alkyle en $C_{1-3}$, où dans les radicaux mentionnés précédemment, chaque fois, une partie alkyle ou un substituant alkyle peut être substitué par un radical carboxy, dans lesquels
$R_3$ représente un atome d'hydrogène ou un radical alkyle en $C_{1-3}$ le cas échéant substitué par un radical carboxy, et
$R_4$ représente un radical (alkyl en $C_{1-3}$)-$Y_2$- ou carboxy-(alkyl en $C_{1-3}$)-$Y_2$-, dans lesquels
$Y_2$ représente une liaison carbone-azote, un radical carbonyle, ou un radical imino le cas échéant substitué par un radical alkyle en $C_{1-3}$ ;
$R_b$ représente un radical méthyle, et
$R_c$ représente un radical amidino le cas échéant substitué par un radical (alcoxy en $C_{1-8}$)carbonyle, acétoxyméthyloxycarbonyle, 2,2,2-trichloroéthoxycarbonyle, benzyloxycarbonyle ou benzoyle,

leurs esters d'alcanol en $C_{1-3}$, leurs tautomères, leurs stéréoisomères et leurs sels.

**6.** Benzimidazoles de la formule générale I selon au moins l'une des revendications 1 à 5, dans laquelle le reste $R_a$ est en position 5,
leurs tautomères, leurs stéréoisomères et leurs sels.

**7.** Composés suivants de la formule générale (Ia) :

(a) le 2-(4-amidinophénylaminométhyl)-1-méthyl-5-[1-(pyrrolidin-1-ylcarbonyl)cyclopropyl]benzimidazole ;
(b) le (E/Z)-2-(4-amidinophénylaminométhyl)-1-méthyl-5-[1-[(pyridin-2-yl)(carboxyméthyloxyimino)-méthylène]cyclopropyl]benzimidazole ;
(c) le 2-(4-amidinophénylaminométhyl)-1-méthyl-5-[1-(2-carboxyéthylamino)-1-(pyrrolidin-1-ylcarbonyl) éthyl]-benzimidazole ;
(d) le 2-(4-amidinophénylaminométhyl)-1-méthyl-5-[1-[2-(2-carboxyéthyl)pyrrolidin-1-ylcarbonyl)cyclopropyl]-benzimidazole ;
(e) le 2-(4-amidinophénylaminométhyl)-1-méthyl-5-[2-(2-carboxyéthyl)-4,5-diméthylimidazol-1-ylméthyl]-benzimidazole ;

(f) le 2-(4-amidinophénylaminométhyl)-1-méthyl-5-[1-(carboxyméthylamino)-1-(pyrrolidin-1-ylcarbonyl)éthyl]-benzimidazole, et

(g) le 2-(4-amidinophénylaminométhyl)-1-méthyl-5-[1-(N-méthylcarboxyméthylcarbonylaminométhyl)-1-méthyl-1-(pyrrolidin-1-ylcarbonyl)éthyl]benzimidazole,

ainsi que leurs esters d'alcanol en $C_{1-3}$, leurs N-(alcoxy en $C_{1-8}$)carbonyl-, N-benzyloxycarbonyl- et N-benzoylamidines, leurs tautomères, leurs stéréoisomères et leurs sels.

**8.** 2-(4-Amidinophénylaminométhyl)-1-méthyl-5-[1-(carboxyméthylamino)-1-(pyrrolidin-1-ylcarbonyl)éthyl]-benzimidazole, ses esters d'alcanol en $C_{1-3}$ et ses N-(alcoxy en $C_{1-8}$)carbonyl-, N-benzyloxycarbonyl- et N-benzoylamidines, ses tautomères, stéréoisomères et sels.

**9.** (R)-2-(4-Amidinophénylaminométhyl)-1-méthyl-5-[1-(carboxyméthylamino)-1-(pyrrolidinocarbonyl)éthyl]-benzimidazole, ses esters d'alcanol en $C_{1-3}$ et ses N-(alcoxy en $C_{1-8}$)carbonyl-, N-benzyloxycarbonyl- et N-benzoylamidines, et ses sels.

**10.** Sels physiologiquement compatibles des composés selon les revendications 1 à 9, dans lesquels $R_c$ représente l'un des radicaux amidine mentionnés dans les revendications 1 à 9.

**11.** Composé pharmaceutique, contenant un composé selon au moins l'une des revendications 1 à 9, dans lequel $R_c$ représente l'un des radicaux amidine mentionnés dans les revendications 1 à 9, ou un sel selon la revendication 10, en plus du cas échéant, un ou plusieurs supports et/ou diluants inertes.

**12.** Utilisation d'un composé selon au moins l'une des revendications 1 à 9, dans lequel $R_c$ représente l'un des radicaux amidine mentionnés dans les revendications 1 à 9, ou un sel selon la revendication 10, pour préparer un composé pharmaceutique avec une activité allongeant la durée de la thrombine, une activité inhibant la thrombine et une activité inhibitrice sur des protéases à sérine apparentées.

**13.** Procédé de préparation d'un composé pharmaceutique selon la revendication 11, **caractérisé en ce que** l'on incorpore de manière non chimique, un composé selon au moins l'une des revendications 1 à 9, dans lequel $R_c$ représente l'un des radicaux amidine mentionnés dans les revendications 1 à 9, ou un sel selon la revendication 10, dans un ou plusieurs supports et/ou diluants inertes.

**14.** Procédé de préparation des composés selon les revendications 1 à 10, **caractérisé en ce que** :

a) pour la préparation d'un composé de la formule générale I, dans laquelle $R_c$ représente un radical cyano, on cyclise un composé le cas échéant formé dans le mélange réactionnel, de la formule générale :

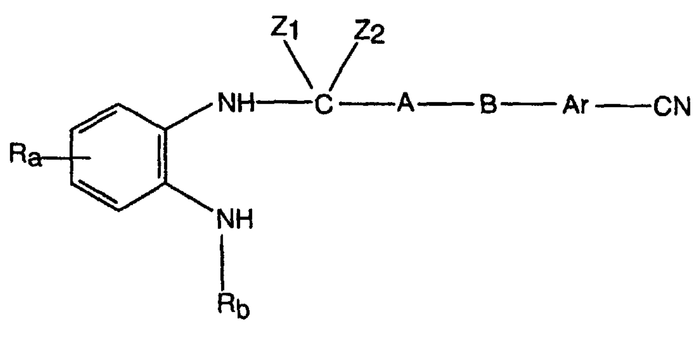

(II)

dans laquelle :

$R_a$, $R_b$, Ar, A et B sont définis comme mentionné dans les revendications 1 à 9 ;
$Z_1$ et $Z_2$, qui peuvent être identiques ou différents, représentent un radical amino, hydroxy ou mercapto, le cas échéant substitué par un radical alkyle ayant 1 à 6 atomes de carbone, ou
$Z_1$ et $Z_2$ représentent ensemble un atome d'oxygène ou de soufre, un radical imino le cas échéant substitué

par un radical alkyle ayant 1 à 3 atomes de carbone, un radical alkylènedioxy ou alkylènedithio, ayant chaque fois 2 ou 3 atomes de carbone, ou

b) pour la préparation d'un composé de la formule générale I, dans laquelle $R_a$ représente un radical $R_2$-CX'-(cycloalkyle en $C_{3-5}$), dans lequel $R_2$ est défini comme mentionné dans les revendications 1 à 9, et X' représente l'un des radicaux imino mentionnés pour X dans les revendications 1 à 9, on fait réagir un composé de formule générale :

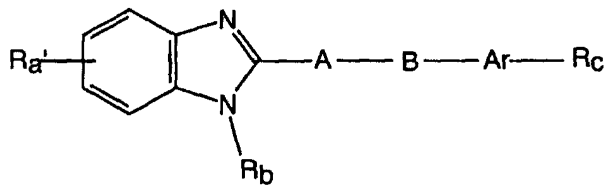

(IV)

dans laquelle :

$R_b$, $R_c$, Ar, A et B sont définis comme mentionné dans les revendications 1 à 9, et
$R_a$' représente un radical $R_2$-CO-(cycloalkyle en $C_{3-5}$), où $R_2$ est défini comme mentionné dans les revendications 1 à 9,
avec une amine de formule générale :

$$H_2X' \qquad (V)$$

dans laquelle :
X' représente un reste imino mentionné pour X dans les revendications 1 à 9, ou

c) pour la préparation d'un composé de la formule générale I, dans laquelle $R_a$ représente un radical $R_2$-CX''-(cycloalkylène en $C_{3-5}$), dans lequel $R_2$ est défini comme mentionné dans les revendications 1 à 9, et X' ' représente l'un des radicaux alkylidène mentionnés pour X dans les revendications 1 à 9, on fait réagir un composé de formule générale :

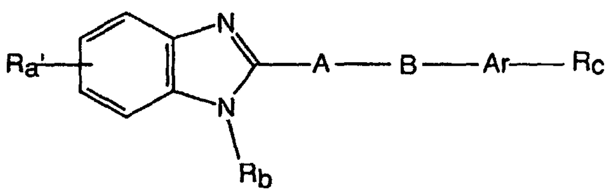

(IV)

dans laquelle :

$R_b$, $R_c$, Ar, A et B sont définis comme mentionné dans les revendications 1 à 9, et
$R_a$' représente un radical $R_2$-CO-(cycloalkylène en $C_{3-5}$), où $R_2$ est défini comme mentionné dans les revendications 1 à 9,
avec une phosphone de formule générale :

$$Z_3\text{-}HX'' \qquad (VI)$$

dans laquelle :
X'' représente l'un des radicaux alkylidène mentionnés pour X dans les revendications 1 à 9, et
$Z_3$ représente un radical triphénylphosphono ou di(alcoxy en $C_{1-3}$)phosphono, ou

d) pour la préparation d'un composé de la formule générale I, dans laquelle $R_c$ représente un radical amidino, qui peut être substitué par un ou deux radicaux alkyle en $C_{1-3}$, on fait réagir un composé le cas échéant formé dans le mélange réactionnel, de formule générale :

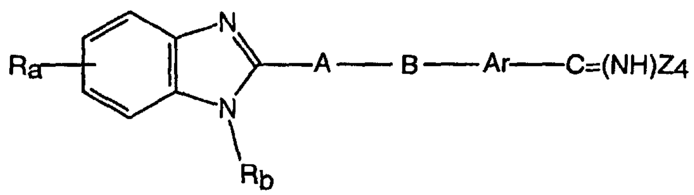

(VII)

dans laquelle :

$R_a$, $R_b$, Ar, A et B sont définis comme mentionné dans les revendications 1 à 9, et
$Z_4$ représente un radical alcoxy, aralcoxy, alkylthio ou aralkylthio,
avec une amine de formule générale :

$$H(R_7)NR_8 \quad \text{(VIII)}$$

dans laquelle :
$R_7$ et $R_8$, qui peuvent être identiques ou différents, représentent chaque fois, un atome d'hydrogène ou un radical alkyle en $C_{1-3}$, ou avec ses sels, ou

e) pour la préparation d'un composé de la formule générale I, dans laquelle $R_a$ représente un radical imidazolidin-2,4-dion-5-yle, qui peut être substitué par un ou deux radicaux alkyle en $C_{1-3}$, où simultanément, un radical alkyle peut être substitué par un radical carboxy ou (alcoxy en $C_{1-3}$)carbonyle, on cyclise un composé le cas échéant formé dans le mélange réactionnel, de formule générale :

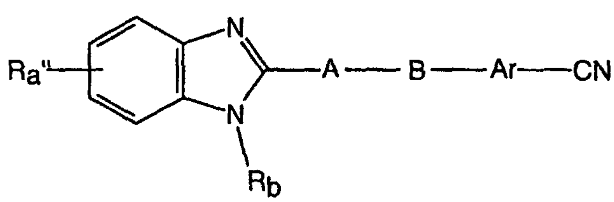

(IX)

dans laquelle :

$R_b$, Ar, A et B sont définis comme mentionné dans les revendications 1 à 9, et
$R_a$'' représente un radical aminocarbonylamino, qui est substitué en position 3 par un radical (alcoxy en $C_{1-3}$)carbonyl(alkyle en $C_{1-3}$), ou

f) pour la préparation d'un composé de la formule générale I, dans laquelle $R_c$ représente un radical hydroxyamidino, on fait réagir un nitrile de formule générale :

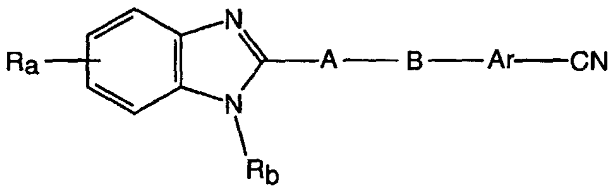

(X)

dans laquelle :

$R_a$, $R_b$, Ar, A et B sont définis comme mentionné dans les revendications 1 à 9, avec l'hydroxylamine ou ses sels, ou

g) pour la préparation d'un composé de la formule générale I, dans laquelle $R_a$ contient un radical carboxy et $R_c$ est défini comme mentionné dans les revendications 1 à 9 ou $R_a$ est défini comme mentionné dans les revendications 1 à 9 et $R_c$ représente un radical amidino le cas échéant substitué par un radical hydroxy ou par un ou deux radicaux alkyle en $C_{1-3}$, on convertit un composé de formule générale :

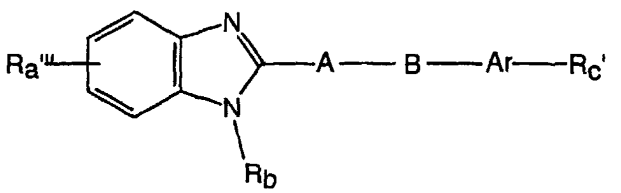

(XI)

dans laquelle :

$R_b$, Ar, A et B sont définis comme mentionné dans les revendications 1 à 9, et
$R_a$' ' ' et $R_c$' possèdent les significations mentionnées pour $R_a$ et $R_c$ dans les revendications 1 à 9, avec la condition que $R_a$ contient un radical convertissable par hydrolyse, traitement avec un acide ou une base, thermolyse ou hydrogénolyse en un radical carboxy et $R_c$ est défini comme mentionné dans les revendications 1 à 9, ou $R_c$ contient un radical convertissable par hydrolyse, traitement avec un acide ou une base, thermolyse ou hydrogénolyse en un radical amidino le cas échéant substitué par un radical hydroxy ou par un ou deux radicaux alkyle en $C_{1-3}$ et $R_a$ est défini comme mentionné dans les revendications 1 à 9,
par hydrolyse, traitement avec un acide ou une base, thermolyse ou hydrogénolyse, en un composé de la formule générale I, dans laquelle $R_a$ contient un radical carboxy et $R_c$ est défini comme mentionné dans les revendications 1 à 9 ou $R_a$ est défini comme mentionné dans les revendications 1 à 9 et $R_c$ représente un radical amidino le cas échéant substitué par un radical hydroxy ou par un ou deux radicaux alkyle en $C_{1-3}$, ou

h) pour la préparation d'un composé de la formule générale I, dans laquelle $R_c$ représente un radical amidino qui est substitué par un radical (alcoxy en $C_{1-8}$)carbonyle ou par un reste clivable in vivo, on fait réagir un composé de formule générale :

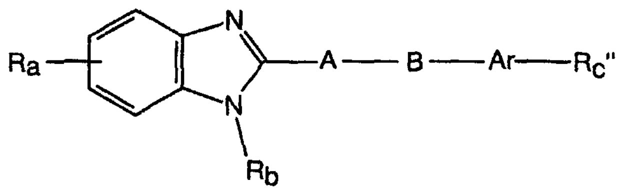

(XII)

dans laquelle :

$R_a$, $R_b$, Ar, A et B sont définis comme mentionné dans les revendications 1 à 9, et
$R_c$" représente un radical amidino,
avec un composé de formule générale :

$$Z_5 - R_9 \qquad (XIII)$$

dans laquelle:

$R_9$ représente un radical (alcoxy en $C_{1-8}$)carbonyle ou le reste acyle d'un des restes clivables in vivo mentionnés au début, et
$Z_5$ représente un groupe partant nucléofuge, et
si souhaité, ensuite un composé ainsi obtenu de la formule générale I, qui contient un radical ($R_3NR_4$)(alkyle en $C_{1-3}$), dans lequel au moins un des restes $R_3$ ou $R_4$ représente un atome d'hydrogène, est converti avec un isocyanate ou halogénure de carbamoyle approprié, en un composé urée correspondant, de la formule générale I, et/ou
un composé ainsi obtenu de la formule générale I, qui contient un radical $H_2N$(alkyle en $C_{1-3}$), est converti avec un ester d'acide acrylique approprié, en un composé 2-(alcoxy en $C_{1-3}$)carbonyléthyle de la formule générale I, et/ou
un composé ainsi obtenu de la formule générale I, qui contient un radical ($R_3NR_4$) (alkyle en $C_{1-3}$), dans lequel $R_3$ et $R_4$ représentent chaque fois un atome d'hydrogène, est converti avec un dihalogénoalcane approprié, en un composé correspondant de la formule générale I, dans laquelle $R_3$ et $R_4$ représentent ensemble, avec l'atome d'azote situé entre eux, un radical cycloalkylènimino à 4 à 7 membres correspondant, et/ou
un composé ainsi obtenu de la formule générale I, dans lequel $R_c$ représente un radical amidino, est converti avec un dérivé d'acide halogénoacétique, ainsi que ensuite, hydrolyse et décarboxylation, en un composé amidino correspondant, substitué par un ou deux radicaux méthyle, et/ou
un composé ainsi obtenu de la formule générale I, dans lequel $R_c$ représente un radical hydroxyamidino, est converti à l'aide d'une hydrogénation catalytique, en un composé amidino correspondant, et/ou
un composé ainsi obtenu de la formule générale I, dans lequel $R_a$ contient un radical carboxy, est converti par transestérification, en un ester correspondant, et/ou
un reste protecteur utilisé pendant les réactions pour protéger un radical réactif, est clivé, et/ou
un composé ainsi obtenu de la formule générale I, est séparé en ses stéréoisomères, et/ou
un composé ainsi obtenu de la formule générale I, est converti en son sel, en particulier pour une utilisation pharmaceutique, en son sel physiologiquement compatible, avec un acide ou une base inorganique ou organique.